# EUROPEAN PATENT APPLICATION

(11) **EP 3 214 171 A1**
(43) Date of publication of application: **06.09.2017**
(21) Application number: 15855075.6
(22) Date of filing: 29.10.2015
(51) Int. Cl.: C12N 5/0775, A61K 35/12, A61P 25/00, A61P 27/02

(54) **PLACENTA-DERIVED CELLS EXCRETING C3 OR C1R COMPLEMENT AND COMPOSITION CONTAINING SAME**

(30) Priority: 29.10.2014 KR 20140148457
(71) Applicant: Sungkwang Medical Foundation, Seoul 06135 (KR)
(72) Inventor: MOON, Ji Sook, Seoul 05649 (KR); KIM, Chul, Anyang-si Gyeonggi-do 13953 (KR); PARK, Ji Min, Busan 47541 (KR); BAE, Sang Hun, Incheon 21553 (KR); KIM, Joopyung, Seongnam-si Gyeonggi-do 13600 (KR); KIM, Hyun Sook, Seoul 06362 (KR); KIM, Ok Joon, Seoul 06216 (KR)
(74) Representative: Weickmann & Weickmann PartmbB
(86) International application number: PCT/KR2015/011493
(87) International publication number: WO 2016/068616

(57) **Abstract**

The present invention relates to placenta-derived cells excreting C3 or C1R complement proteins, a pharmaceutical composition containing same, and a method for treating diseases using the placenta-derived cells. The placenta-derived cells, according to the present invention, can be effectively applied to Alzheimer's disease, Parkinson's disease, HIV, neurodegenerative diseases such as dementia and epilepsy, or angiogenesis-related diseases such as cancer and retinopathy, thereby having high industrial utility.

<210>    1
<211>    20
<212>    DNA
<213>    Artificial Sequence

<220>
<223>    forward primer sequence of mIL-1


<400>    1
cccaagcaat acccaaagaa                                                   20


<210>    2
<211>    20
<212>    DNA
<213>    Artificial Sequence

<220>
<223>    Reverse primer sequence of mIL-1


<400>    2
gcttgtgctc tgcttgtgag                                                   20


<210>    3
<211>    20
<212>    DNA
<213>    Artificial Sequence

<220>
<223>    forward primer sequence of mTNF-a


<400>    3
gctccagtga attcggaaag                                                   20


<210>    4
<211>    20
<212>    DNA
<213>    Artificial Sequence

<220>
<223>    Reverse primer sequence of mTNF-a

<400>    4
gattatggct cagggtccaa                                                    20


<210>    5
<211>    22
<212>    DNA
<213>    Artificial Sequence

<220>
<223>    forward primer sequence of mIL-10


<400>    5
cagggatctt agctaacgga aa                                                 22


<210>    6
<211>    26
<212>    DNA
<213>    Artificial Sequence

<220>
<223>    Reverse primer sequence of mIL-10


<400>    6
gctcagtgaa taaatagaat gggaac                                             26


<210>    7
<211>    19
<212>    DNA
<213>    Artificial Sequence

<220>
<223>    forward primer sequence of mTGF-b


<400>    7
caagggctac catgccaac                                                     19


<210>    8
<211>    18
<212>    DNA
<213>    Artificial Sequence

<220>
<223>    Reverse primer sequence of mTGF-b


<400>    8
agggccagga ccttgctg                                                      18


<210>    9
<211>    18
<212>    DNA
<213>    Artificial Sequence

<220>
<223>    forward primer sequence of mMMP-9

<400>    9
tgccgtcgaa gggatacc                                                    18


<210>    10
<211>    19
<212>    DNA
<213>    Artificial Sequence

<220>
<223>    Reverse primer sequence of mMMP-9


<400>    10
gacccgaagc ggacattgt                                                   19


<210>    11
<211>    22
<212>    DNA
<213>    Artificial Sequence

<220>
<223>    forward primer sequence of mIDE


<400>    11
gaagacaaac gggaataccg tg                                               22


<210>    12
<211>    19
<212>    DNA
<213>    Artificial Sequence

<220>
<223>    Reverse primer sequence of mIDE


<400>    12
cgctgaggac ttgtctgtg                                                   19


<210>    13
<211>    21
<212>    DNA
<213>    Artificial Sequence

<220>
<223>    forward primer sequence of mGAPDH


<400>    13
gtgagggaga tgctcagtgt t                                                21


<210>    14
<211>    15
<212>    DNA
<213>    Artificial Sequence

<220>
<223>    Reverse primer sequence of mGAPDH

<400>    14
ggctggcatt gctct                                                        15

<210>    15
<211>    24
<212>    DNA
<213>    Artificial Sequence

<220>
<223>    forward primer sequence of hTGF-b

<400>    15
aaattgaggg ctttcgcctt agcg                                              24

<210>    16
<211>    24
<212>    DNA
<213>    Artificial Sequence

<220>
<223>    everse primer sequence of hTGF-b

<400>    16
tcttctccgt ggagctgaag caat                                              24

<210>    17
<211>    20
<212>    DNA
<213>    Artificial Sequence

<220>
<223>    forward primer sequence of hMMP-9

<400>    17
ttgacagcga caagaagtgg                                                   20

<210>    18
<211>    20
<212>    DNA
<213>    Artificial Sequence

<220>
<223>    Reverse primer sequence of hMMP-9

<400>    18
gccattcacg tcgtccttat                                                   20

<210>    19
<211>    21
<212>    DNA

<213>    Artificial Sequence

<220>
<223>    forward primer sequence of hIDE


<400>    19
ctcggaacct tgcttcaaca c                                                21


<210>    20
<211>    18
<212>    DNA
<213>    Artificial Sequence

<220>
<223>    Reverse primer sequence of hIDE


<400>    20
ggcccgctga agacgata                                                    18


<210>    21
<211>    21
<212>    DNA
<213>    Artificial Sequence

<220>
<223>    forward primer sequence of hGAPDH


<400>    21
agggctgctt ttaactctgg t                                                21


<210>    22
<211>    21
<212>    DNA
<213>    Artificial Sequence

<220>
<223>    Reverse primer sequence of hGAPDH


<400>    22
ccccacttga ttttggaggg a                                                21


<210>    23
<211>    21
<212>    DNA
<213>    Artificial Sequence

<220>
<223>    forward primer sequence of mPEDF


<400>    23
gcccagatga aagggaagat t                                                21


<210>    24

<211>    16
<212>    DNA
<213>    Artificial Sequence

<220>
<223>    Reverse primer sequence of mPEDF


<400>    24
tgagggcact gggcat                                                        16


<210>    25
<211>    24
<212>    DNA
<213>    Artificial Sequence

<220>
<223>    forward primer sequence of mAng-1


<400>    25
cctctggtga atattggctt ggga                                               24


<210>    26
<211>    24
<212>    DNA
<213>    Artificial Sequence

<220>
<223>    Reverse primer sequence of mAng-1


<400>    26
agcatgtact gcctctgact ggtt                                               24


<210>    27
<211>    24
<212>    DNA
<213>    Artificial Sequence

<220>
<223>    forward primer sequence of hPEDF


<400>    27
tatcacctta accagccttt catc                                               24


<210>    28
<211>    21
<212>    DNA
<213>    Artificial Sequence

<220>
<223>    Reverse primer sequence of hPEDF


<400>    28
gggtccagaa tcttgccaat g                                                  21

<210>    29
<211>    21
<212>    DNA
<213>    Artificial Sequence

<220>
<223>    forward primer sequence of hAng-1


<400>    29
ccgtgaatct ggagccgttt g                                                21


<210>    30
<211>    20
<212>    DNA
<213>    Artificial Sequence

<220>
<223>    Reverse primer sequence of hAng-1


<400>    30
aagcccgaca gtcagtggag                                                  20


<210>    31
<211>    19
<212>    DNA
<213>    Artificial Sequence

<220>
<223>    siRNA for TGF-b


<400>    31
cagaguacac acagcauau                                                   19


<210>    32
<211>    19
<212>    DNA
<213>    Artificial Sequence

<220>
<223>    siRNA for TGF-b1


<400>    32
auaugcugug uguacucug                                                   19

## Description

### TECHNICAL FIELD

The present invention relates to a placenta-derived cell secreting C3 or C1r complement, a pharmaceutical composition including the same, and a method of treating a disease by using the same.

### BACKGROUND ART

Recently, as average life expectancy increases and society ages, there is growing interest in aging and related diseases, especially, neurodegenerative diseases related to neuronal cell injury such as neurological disorders including stroke, dementia, Alzheimer's disease, Parkinson's disease, etc. The neurological diseases are characterized in that death or degeneration of specific brain cells progresses temporarily or for a long period of time. Once brain cells die, they do not regenerate, and eventually, lead to fatal loss of brain function. In particular, Alzheimer's disease, which is a representative neurological disorder, afflicts about 50% of the elderly aged over 80, and is the fourth leading cause of death, following cancer, heart disease, and stroke. However, the only way to treat Alzheimer's disease is an indirect method which mainly focuses on alleviating symptoms of patients and delaying the progression of the disease.

Meanwhile, angiogenesis is a biological process that provides new blood vessels to tissues or organs. Normal angiogenesis is tightly regulated by angiogenesis regulators. However, when angiogenesis is not autonomously regulated and pathologically grows, diseases develop. Angiogenesis-related diseases that appear in pathological conditions may be largely classified into inflammatory diseases such as arthritis, ophthalmologic diseases such as diabetic retinopathy, dermatological diseases such as psoriasis, and cancer. Although a fundamental treatment for angiogenesis-related diseases is to inhibit angiogenesis, many angiogenesis inhibitors currently developed have a problem of toxicity in the body.

Accordingly, use of stem cells has been actively studied as a new method of treating neurological diseases or angiogenesis-related diseases. Among them, mesenchymal stem cells have an ability to self-renew and differentiate into several tissues according to conditions, and have various therapeutic efficacies. Thus, mesenchymal stem cells are an excellent candidate as a therapeutic agent for the diseases. However, the number of mesenchymal stem cells is not only very small in adult human bone marrow, but also rapidly decreases as they age. Therefore, since mesenchymal stem cells are not enough for future cell therapy, alternative methods are needed.

Until a recent date, placenta has been recognized as a temporary organ to be discarded after birth. However, in recent years, it was revealed that various cells such as mesenchymal cells, decidua cells, amnion cells, and endothelial cells are found in portions of the placenta. Placenta-derived cells are advantageous in that ethical issues may be avoided and a large amount of cells may be obtained. In addition, if placenta-derived cells having excellent therapeutic characteristics for diseases may be obtained, they may be used as a cell therapeutic agent to address the existing disadvantages.

Under this background, the present inventors isolated/cultured novel placenta-derived cells, and they found that these novel cells have a property of expressing complements, a therapeutic effect on neurological diseases or angiogenesis-related diseases, and an anti-aging effect, thereby completing the present invention.

### DETAILED DESCRIPTION OF THE INVENTION

### TECHNICAL PROBLEM

An object of the present invention is to provide a placenta-derived cell.

Another object of the present invention is to provide a pharmaceutical composition for treating or preventing neurological diseases or angiogenesis-related diseases, including the placenta-derived cell.

Still another object of the present invention is to provide a composition for reducing senescence, including the placenta-derived cell.

Still another object of the present invention is to provide a method of treating neurological diseases, angiogenesis-related diseases, or aging-associated symptoms of a subject by administering into the subject the composition including the placenta-derived cell.

### TECHNICAL SOLUTION

An aspect provides a placenta-derived cell secreting a C3 or C1 complement protein.

As used herein, the term "placenta" is an organ formed in the uterus of a pregnant mammal, and composed of a decidual membrane derived from uterine mucous membrane of a mother, a chorionic membrane and an amniotic membrane derived from a fertilized egg, and placenta is part where exchange of materials between the mother and fetus occurs.

The term "placenta-derived cell" refers to a cell that is isolated from the placenta and have an ability to differentiate into a variety of tissue cells including mesodermal cells such as bone, cartilage, fat, and muscle cells, or ectodermal cells such as nerve cells.

The placenta from which the cells of the present invention are derived may include amniotic membrane, chorionic membrane, decidual membrane, and umbilical cord, or a combination thereof. For example, the placenta-derived cells may be derived from a placental amniotic membrane. Further, specifically, the placenta-derived cells may be derived from a placental chorionic plate membrane. In this case, since the placenta-derived cells are derived from a single extra-embryonic mesoderm, it may be further valued as a raw material for cell therapy.

The placenta-derived cells of the present invention may be characterized by secreting a C3 or C1r complement protein.

The term "complement" refers to a protein component of a body fluid (mainly serum) that nonspecifically binds with an antigen-antibody complex and is involved in infection, inflammation, immune response, etc. to exhibit various biological activities. The complement includes 9 components of C1 to C9 (complement components), and C1 may be divided into a complex of C1q, C1r, and C1r according to a cleavage product of the complement component. The complement system to induce immune responses is activated by three pathways: classical pathway of complement system, alternative pathway of complement system, and lectin pathway of complement system. All the three pathways lead to activation of C3 which is one of nine complement components, which causes accumulation of C3b on the pathogen surface to cause phagocytosis and to destroy the target pathogen, although the three pathways are initiated by different causes. It is reported that deficiency of C3 is vulnerable to bacterial infection, and the abnormal decrease or increase of C3 is involved in various immunodeficiency diseases as well as neurodegenerative diseases such as Alzheimer's disease and Parkinson's disease.

The placenta-derived cells may further secrete one or more proteins selected from the group consisting of EPPK1, PTX3, EEF1A1, LGALS3BP (galectin-3-binding protein), FLJ53478, PXDN, and TGFβ. The placenta-derived cells may secrete, for example, EPPK1, PTX3, EEF1A1, LGALS3BP, FLJ53478, PXDN, and TGF.

In an Example of the present invention, it was confirmed that C3, C1r, PTx3, EEF1A1, LGALS3BP, and FLJ53478 were secreted from the placenta-derived cells according to the present invention, whereas all of C3, C1r, PTx3, EEF1A1, LGALS3BP, and FLJ53478 were not detected in neural progenitor cells. C3, C1r, PTx3, EEF1A1, LGALS3BP, and FLJ53478 are immune-related proteins. Meanwhile, an aging-related protein, EPPK1 was found to be secreted from neural progenitor cells, but its amount measured in placenta-derived cells was about 7 times higher than that in neural progenitor cells.

The placenta-derived cells of the present invention may express the C3 or C1r complement protein in an amount of 0.1 pg/ml/1×10⁶ cells to 1000 pg/ml/1×10⁶ cells. For example, the placenta-derived cells may express the C3 or C1r complement protein in an amount of 1 pg/ml/1×10⁶ cells to 1000 pg/ml/1×10⁶ cells, or 10 pg/ml/1×10⁶ cells to 1000 pg/ml/1×10⁶ cells.

Further, the placenta-derived cells may express EPPK1 in an amount about 7 times higher than neural progenitor cells (based on an expression level).

Further, the placenta-derived cells may have a phenotype of CD9⁺, CD13⁺, CD90⁺ and CD200⁺. Further, placenta-derived cells may be SSEA4⁻, TRA-1-60⁻, TRA-1-81⁻ and CD34⁻. As used herein, the term "phenotype of +" means that a marker exists in a larger amount or higher concentration, as compared to that of other reference cells. That is, a marker of cells exists inside the cells or on the surface of the cells, and therefore, if the cells may be distinguished from one or more other cell types by using the marker, the cells may be described as having a phenotype of the marker +, and it also means that the cells have the marker in an amount large enough to display a higher value of signals than a background value, for example, in an amount large enough to allow signaling of a cell measuring device. The term "phenotype of -" means that a marker may not be detected, as compared to a background value, even though an antibody specific to a surface marker of particular cells is used.

Another aspect provides a pharmaceutical composition including the placenta-derived cells.

The placenta-derived cells included in the pharmaceutical composition are the same as described above.

In one specific embodiment, provided is a pharmaceutical composition for treating or preventing neurological diseases, including the placenta-derived cells.

The neurological disease may be, for example, Alzheimer's disease, Parkinson's disease, HIV dementia, epilepsy, schizophrenia, depression, manic depression, neurogenic disorders, autism, stroke, Lou Gehrig's disease, Huntington's disease, multiple sclerosis, brain damage, cerebral palsy, or spinal cord injury. Preferably, the neurological disease may be Alzheimer's disease.

In an Example of the present invention, the placenta-derived cells according to the present invention were administered into an Alzheimer's disease-induced mouse model, and as a result, the cells showed an effective therapeutic effect on Alzheimer's disease.

In another specific embodiment, provided is a pharmaceutical composition for treating or preventing angiogenesis-related diseases, including the placenta-derived cells.

The angiogenesis-related disease may be, for example, cancer, diabetic retinopathy, retinopathy of prematurity, corneal graft rejection, neovascular glaucoma, erythrosis, proliferative retinopathy, psoriasis, hemophilic joints, capillary proliferation in atherosclerotic plaques, keloids, wound granulation, vascular adhesion, rheumatoid arthritis, osteoarthritis, autoimmune disease, Crohn's disease, recurrent stenosis, atherosclerosis, intestinal adhesion, cat scratch disease, ulcer, hepatopathy, glomerulonephritis, diabetic nephropathy, malignant neuropathy, thrombotic microangiopathy, organ graft rejection, nephropathy, or diabetes. Preferably, the angiogenesis-related disease may be retinopathy-related diseases such as diabetic retinopathy, retinopathy of prematurity, or proliferative retinopathy.

In an Example of the present invention, the placenta-derived cells according to the present invention were administered into the retina of an OIR mouse model, and as a result, neovascular tuft formation was suppressed and normal retinal vasculogenesis was increased in proliferative retinopathy, as compared with a control group.

In still another specific embodiment, provided is a composition for reducing senescence, including the placenta-derived cells.

With regard to the composition, the reducing of senescence may include retarding or preventing of senescence of a cell or a subject, or converting of senescent cells into young cells.

As used herein, the term "senescence of a cell" includes one or more of a decrease of a proliferation ability of the cell, an accumulation of lipofuscin, an increase of an activity of β-galactosidase, an increase of mitochondrial-derived reactive oxygen species, and a decrease of a mitochondrial membrane potential, and a decrease of G0 and/or G1 phase of the cell, as compared with a reference cell, or a process that causes those described above. The term "young cell" includes one or more of an increase of a proliferation ability of the cell, a decrease of an accumulation of lipofuscin, a decrease of an activity of β-galactosidase, a decrease of mitochondrial-derived reactive oxygen species, and an increase of a mitochondrial membrane potential, and an increase of G0 and/or G1 phase of the cell, as compared with a reference cell.

With regard to the composition, the reducing of senescence of a cell may include one or more of an increase of a proliferation ability of the cell, a decrease of an accumulation of lipofuscin, a decrease of an activity of β-galactosidase, a decrease of mitochondrial-derived reactive oxygen species, and an increase of a mitochondrial membrane potential, and a decrease of G0 and/or G1 phase of the cell. The cell may be myocytes including myoblasts, fibroblasts, cells from a subject suffering Hutchinson-Gilford progeria syndrome, or nerve cells.

In still another aspect, provided is a composition for treating symptoms related to senescence of a subject or a cell, including the placenta-derived cells. The symptoms related to senescence of a cell may include skin wrinkles, reduced ability to repair wounds, sarcopenia, Hutchinson-Gilford progeria syndrome, or any combination thereof. The symptoms related to senescence of a cell may include symptoms related to lipofuscin accumulation. The symptoms related to lipofuscin accumulation may include neuronal ceroid lipofuscinoses (NCL), age-related macular degeneration, neurofibrillary tangles, brown atrophy of the heart, Alzheimer's disease, Parkinson's disease, amyotrophic lateral sclerosis (ALS), acromegaly, denervation atrophy, lipid myopathy, or chronic obstructive pulmonary disease (COPD).

The pharmaceutical composition including the placenta-derived cells may include a medium obtained from a culture of the placenta-derived cells, in addition to the placenta-derived cells. The medium may be obtained by culturing the placenta-derived cells in Alpha-MEM containing FGF4 and heparin, and then culturing the cells in a serum-free medium. Since the placenta-derived cells of the present invention secrete C3, C3 is included in the medium. Therefore, the medium may have more excellent effects of treating neurological diseases, of treating angiogenesis-related diseases, of reducing senescence or treating senescence-related symptoms. Further, the medium may include C1r, EEF1A1, LGALS3BP, and FLJ53478 which are immune-related proteins and EPPK1 which is a senescence-related protein, in addition to C3.

The pharmaceutical composition including the placenta-derived cells may further include a pharmaceutically acceptable additive, in addition to the placenta-derived cells as an active ingredient, and may be formulated in a unit dosage form suitable for administration into the body of a patient according to a common method in the pharmaceutical field. As the formulation suitable for the purpose, a parenteral formulation may include an injectable formulation or a topical formulation. For example, if necessary, the pharmaceutical composition may be parenterally used in the form of an injectable formulation of a sterile solution or a suspension prepared by using water or other pharmaceutically acceptable solvent. For example, the pharmaceutical composition may be formulated as a generally-admitted unit dosage form by suitably combining with a pharmaceutically-acceptable carrier or medium, such as a sterile solution, a physiological saline, a vegetable oil, an emulsifier, a suspending agent, a surfactant, a stabilizer, an excipient, a vehicle, a preservative, a binder, etc.

Further, the composition of the present invention may include one or more stabilizers such as a compound promoting engraftment (e.g., anti-T cell receptor antibody, immunosuppressant, etc.), albumin, dextran 40, gelatin, hydroxyethyl starch, etc.

The formulation may be orally or parenterally administered according to an ordinary method, and specifically, parenterally administered. The parenteral administration may include topical or systemic administration. The topical administration may include direct administration to a lesion or a surrounding area thereof, for example, direct administration to the brain or spinal cord, the surrounding area thereof, or the opposite site. The systemic administration may include administration into the spinal fluid, vein, or artery. The spinal fluid may include the cerebrospinal fluid. The artery may be a site supplying blood to a lesion.

A daily dosage of the composition may be 1x10⁴ cells/kg to 1x10⁷ cells/kg, for example 5x10⁵ cells/kg to 5x10⁶ cells/kg depending on the body weight. An administration frequency may be once or several times. A practical administration dose of the composition may be increased or decreased, considering various relevant factors such as a disease to be treated, severity of the disease, an administration route, body weight, age, and sex of a patient.

The composition of the present invention may be administered singly or in combination with other pharmaceutically active compound as well as in a proper collection. Further, the composition of the present invention may be formulated as a pharmaceutical preparation by a method well known in the art so as to provide a rapid, sustained, or delayed release of the active ingredient after being administered to mammals. In the preparation of the formulation, the active ingredient may be mixed or diluted with a carrier, or encapsulated in a container-shaped carrier.

Still another aspect provides a kit for treating neurological diseases, angiogenesis-related diseases, or senescence-related symptoms, including the pharmaceutical composition. The kit may further include components required for culturing the placenta-derived cells. The placenta-derived cells, the pharmaceutical composition, and the neurological diseases, angiogenesis-related diseases, and senescence-related symptoms are the same as described above.

Still another aspect provides a method of treating neurological diseases, angiogenesis-related diseases, or senescence-related symptoms, including the step of administering into a subject the pharmaceutical composition including the placenta-derived cells.

With regard to the treatment method, the placenta-derived cells and pharmaceutical composition administered to a subject are the same as described above. With regard to the method, the placenta-derived cells may be cells collected from the patient himself or cells derived from other medical animal. The cells may be cryopreserved cells.

The method may further include the step of culturing the placenta-derived cells in a dish coated with polyornithine and fibronectin; and incubating the cultured cells in a medium containing FGF4 and heparin under CO₂ condition of 1% to 7%. Further, the CO₂ condition may be 2% to 5%, or 3%.

The administration may be topical or systemic administration. For example, the administration may be oral, rectal, intravenous, nasal, intraperitoneal, subcutaneous, or topical administration, and the topical administration may be, for example, direction administration into a lesion or a surrounding area thereof. The administration may be administration of an effective amount for preventing or treating the diseases. The effective amount may be readily selected by those skilled in the art depending on conditions of a disease selected. Further, the pharmaceutical composition of the present invention may be administered to a target cell by using any device capable of delivering the active ingredient.

The treatment method of the present invention is not limited to human. The term "subject" may include mammals including monkeys, cows, horses, sheep, pigs, chickens, turkeys, quails, cats, dogs, mice, rats, rabbits, guinea pigs, or humans, but is not limited thereto. In an Example, the subject may be a mammal. Commonly, placenta-derived cells may be also used in a mammal excluding a human to perform the method of the present invention.

Still another aspect provides neuronal progenitor cells derived from placenta-derived cells.

The placenta-derived cells may be derived from a fetus or a mother (that is, the cells may have a genotype of the fetus or the mother). A placental cell population or a cell population including placental cells may include placenta-derived cells derived from only the fetus or mother, and may also include a mixed cell population of the placental cells derived from the fetus and the mother. The placenta-derived cells and the cell population including placenta-derived cells may be identified and selected according to morphological markers and culture characteristics described below.

The placenta-derived cells of the present invention may be derived from an amniotic membrane, a chorionic membrane, a decidual membrane, and an umbilical cord of the placenta or a combination thereof. For example, the cells may be derived from the amniotic membrane of the placenta.

The term "neuronal progenitor cells (neuronal precursor cells)" refer to cells that are able to differentiate into various types of neuronal cells. The neuronal progenitor cells may be used interchangeably with neural stem cells (neural progenitor cells or neural precursor cells). Further, the neuronal progenitor cells may be cells derived from neural stem cells. The neuronal progenitor cells may be CD9⁺, CD13⁺, and CD90⁺. Further, the neuronal progenitor cells may be SSEA-4⁻, TRA-1-60⁻, TRA-1-81⁻, SSEA-1⁻, CD34- and CD44⁺. The neuronal progenitor cells may be derived by culturing the placenta-derived cells at a high density in the presence of FGF4.

The neural progenitor cells may be differentiated from the placenta-derived cells. In this case, the placenta-derived cells are the same as described above.

The neural progenitor cells derived from the placenta-derived cells may be derived from the placenta-derived cells by a high-density cell culture method. The high-density cell culture method may be a method of spreading cells at a density of 10,000 cells/cm² in the center of a dish coated with 15 ug/mL polyornithine (Sigma) and 4 µg/mL fibronectin (Sigma) by spotting cells at about 1/3 of the total plate space, and subsequently, culturing the cells in a complete medium containing 25 ng/mL FGF4 and 1 µg/mL heparin. The placenta-derived cells may be obtained by grinding the placenta, digesting the ground placenta by using a solution containing 2 mg/ml trypsin (Sigma), 20 ug/ml DNase I(Sigma), 1.2 U/ml Dispase(Gibco), and 1 mg/ml collagenase IV (Sigma), and culturing the collected cells in an Alpha-MEM medium (Gibco, New York, USA) containing 10% fetal bovine serum (FBS, Gibco) and penicillin/streptomycin (Gibco), 25 ng/mL FGF4 (R&D Systems), and 1 µg/mL heparin (Sigma).

Still another aspect provides a pharmaceutical composition for treating or preventing neurological diseases, including the neural progenitor cells.

The neurological diseases may be Alzheimer's disease, Parkinson's disease, HIV dementia, epilepsy, schizophrenia, depression, manic depression, neurogenic disorders, autism, stroke, Lou Gehrig's disease, Huntington's disease, multiple sclerosis, brain damage, cerebral palsy or spinal cord injury. Preferably, the neurological disease may be Alzheimer's disease. In an Example, the neural progenitor cells derived from the placenta-derived cells according to the present invention were administered into Parkinson's disease model mice, resulting in improvement of the disease.

### ADVANTAGEOUS EFFECTS OF THE INVENTION

Placenta-derived cells according to an aspect have effects of treating neurological diseases and angiogenesis-related diseases and of reducing senescence, and therefore, may be used as a material in the development of new drugs for treating neurological diseases, angiogenesis-related diseases, or senescence-related symptoms, and also widely used in basic research related to the treatment of neurological diseases, angiogenesis-related diseases, or senescence-related symptoms.

### DESCRIPTION OF THE DRAWINGS

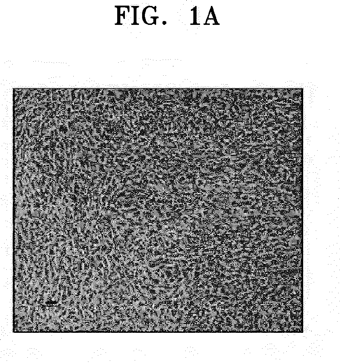

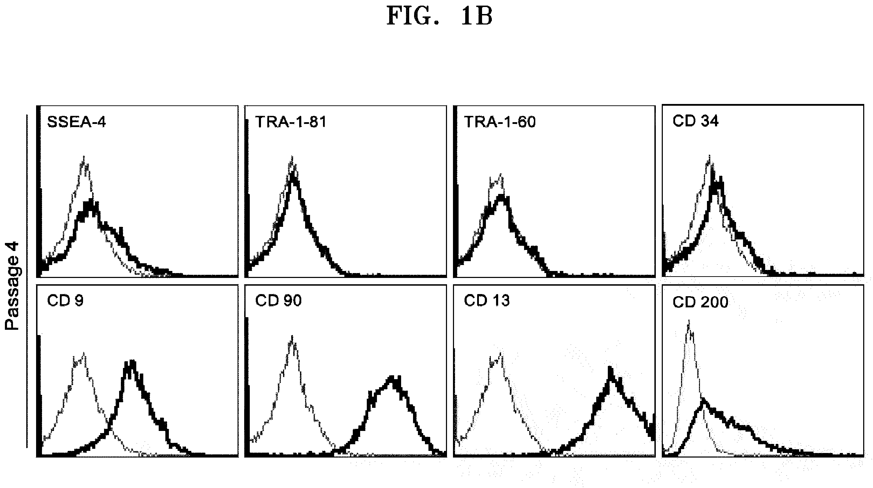

FIG. 1 shows placenta-derived cells. (A) morphology of placenta-derived cells of the present invention which were recovered at passage 4, (B) result of FACS analysis of immunophenotypic profile of placenta-derived cells.

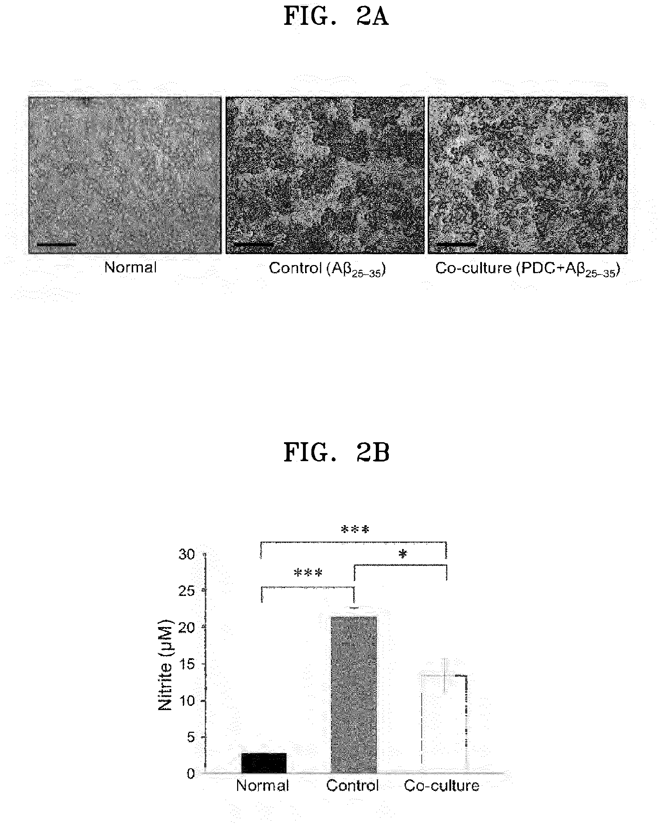

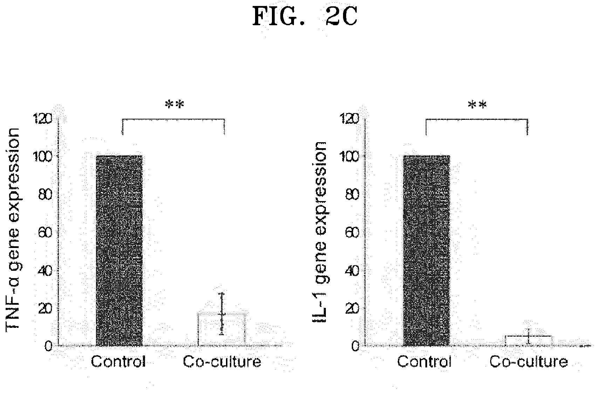

FIG. 2 shows effects of placenta-derived cells (PDCs) in microglial cells stimulated with Aβ₂₅₋₃₅. (A) The Aβ₂₅₋₃₅-activated microglial cells showed densely packed cell clumps, whereas microglial cells co-cultured with PDCs did not cause clumping. (B) Tumor necrosis factor (TNF-α) and interleukin 1 (IL-1) which are proinflammatory cytokines significantly decreased in microglial cells co-cultured with PDCs. Scale bar, 100 µm. **p < 0.01 for each comparison. Data were expressed as means ± S.E.M.

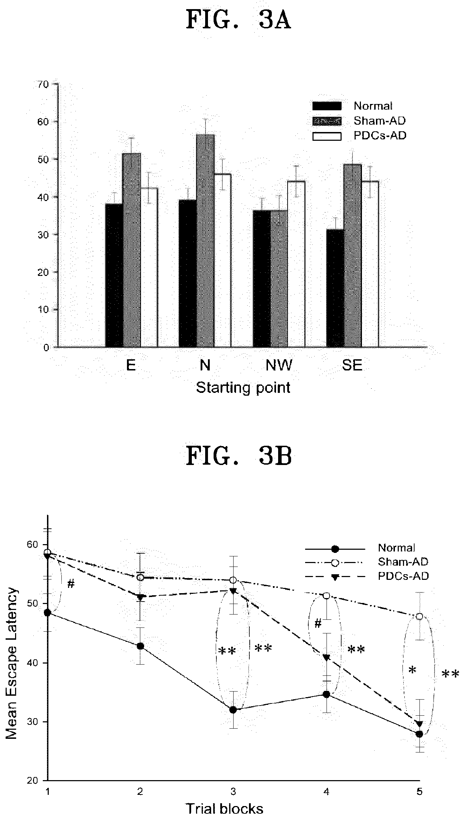

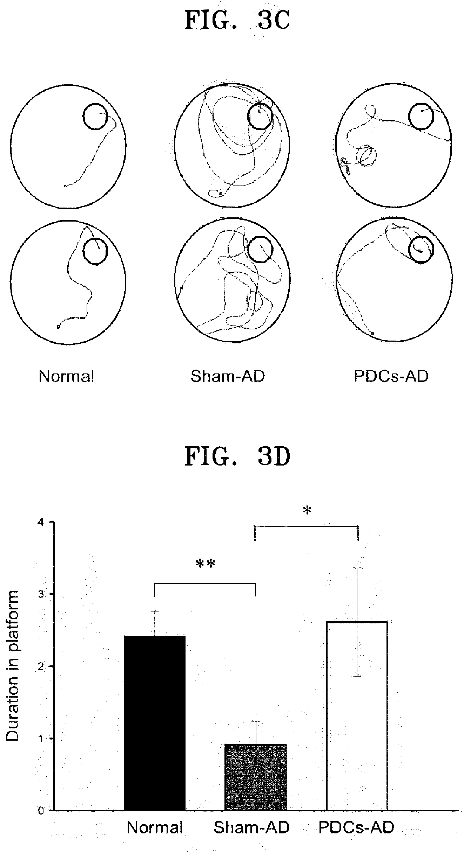

FIG. 3 shows effects of PDCs on spatial memory in a water maze test of mice. (A) No significant difference in escape latency was found on each starting point among the groups. (B) A PDC-treated group exhibited significantly decreased mean escape latencies, as compared with a PBS-treated group after the third trial block. (C) Representative swimming paths at the fifth trial block. (D) In the PDC-injected group, a time spent in the target zone during a probe test was significantly increased, as compared with the PBS-injected group. **, P < 0.01; *, P < 0.05; #, P < 0.06 for each comparison. Data are expressed as mean ± S.E.M.

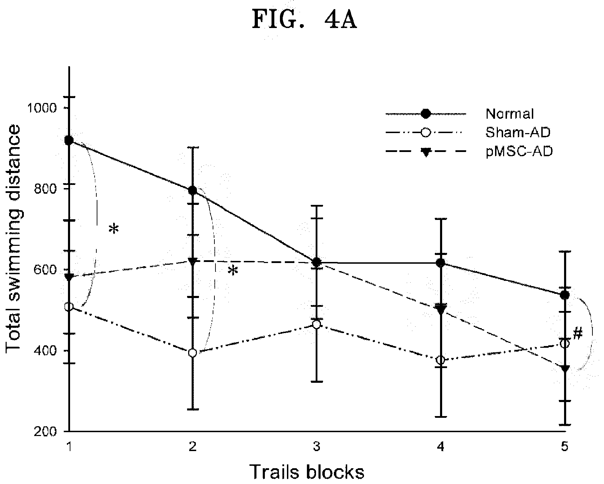

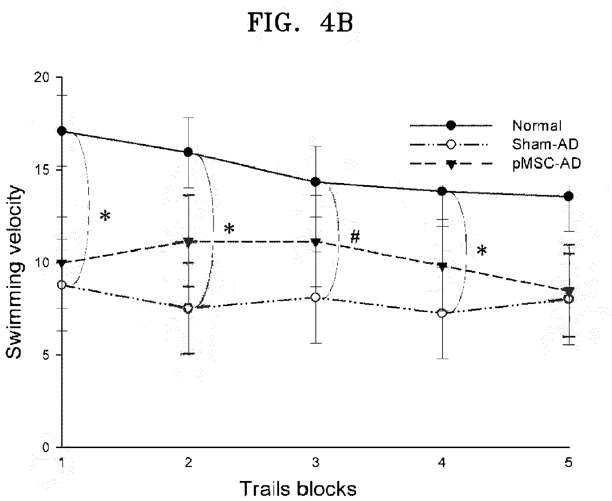

FIG. 4 shows effects of PDCs on swimming activity in WMT. (A) A total swimming distance, and (B) A swimming velocity. *P < 0.05. #p < 0.08. Data are expressed as mean ± S.E.M.

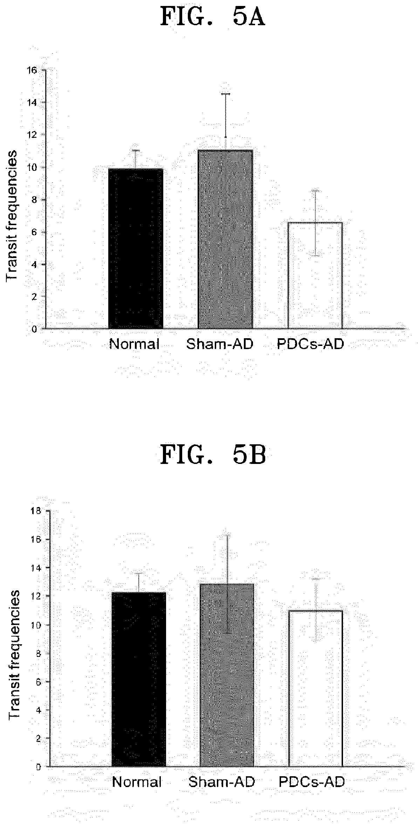

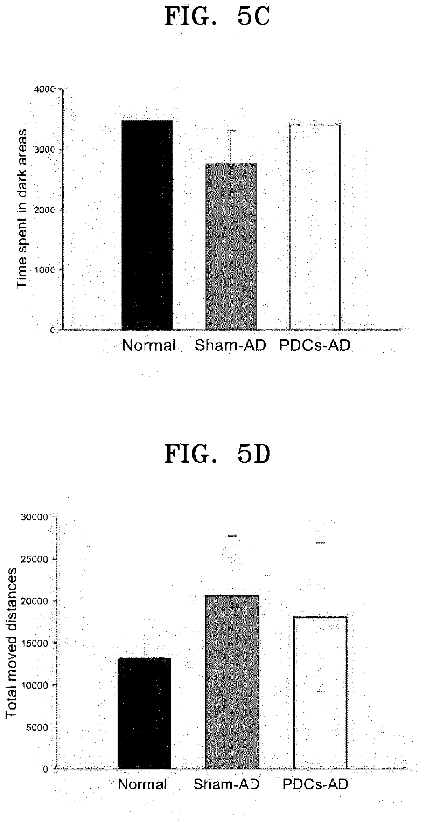

FIG. 5 shows anxiety-like behavior and locomotor activity. (A) Elevated plus-maze test (B) Light/dark transition test (C) Open field test (D) Locomotion test.

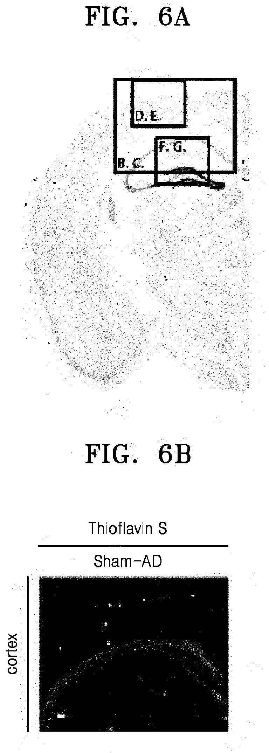

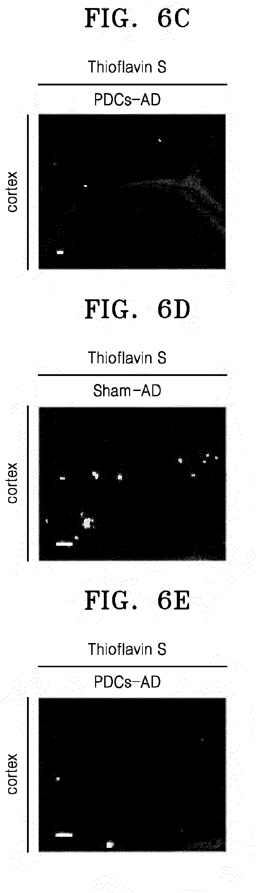

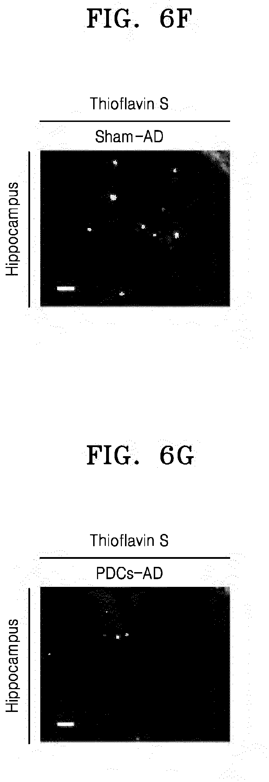

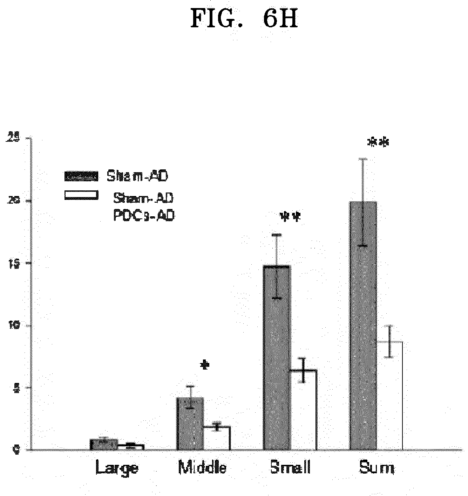

FIG. 6 shows effect of PDC administration on cerebral Aβ plaque deposition. (A) Brain histology of the hippocampal and cortical areas. Mouse frozen brain section from the cortex and hippocampus (low magnification x40, B and C), high magnification (x100, D, E, F and G)) were stained with thiofalvin S (green). (H) Results of quantitative analysis of Aβ in APPswe (Tg2576) mice injected with PDC or PBS. Scale bar, 100 mm. **, P < 0.01; *, P < 0.05 for each comparison. Data are expressed as mean ± S.E.M.

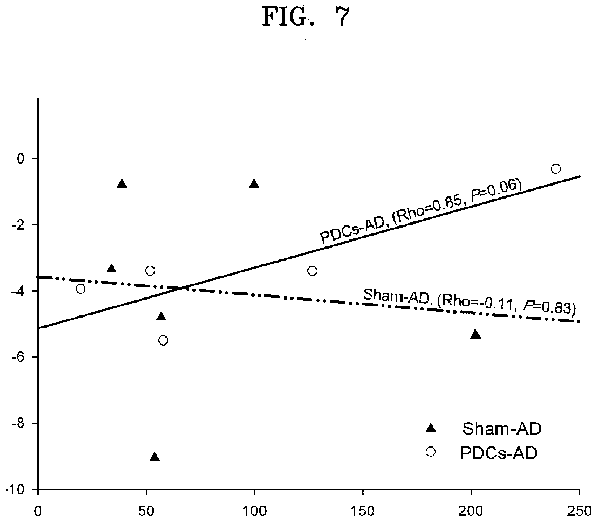

FIG. 7 shows a correlation between escape latency in the WMT and the number of amyloid plaques.

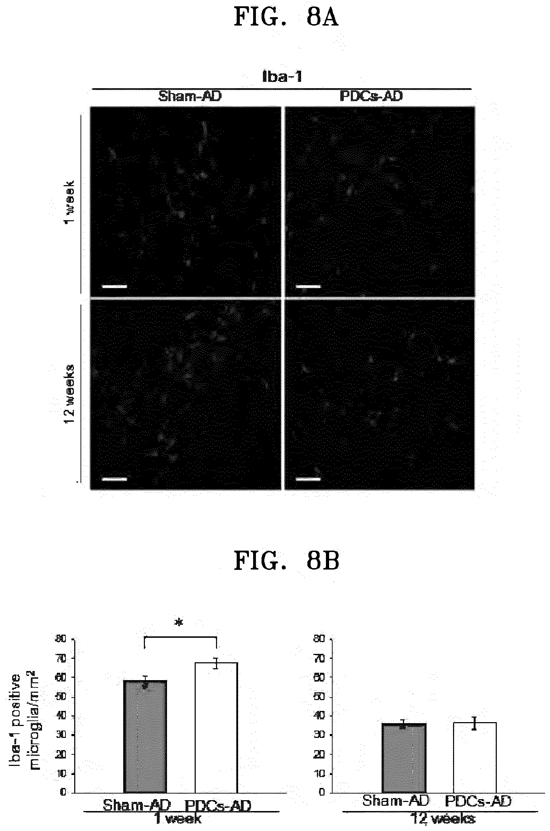

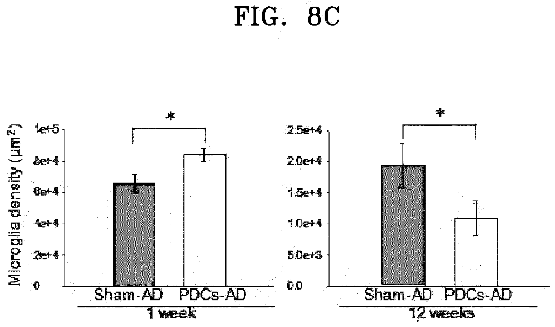

FIG. 8 shows effect of PDC administration on microglia regulation. (A) Immunofluorescence using a primary antibody (lba-1) was used to label microglia. The pattern of microglia in the brain cortex area in APPswe mice injected with PDC or PBS. (B) Quantitative image analysis of mean number of microglia per mm² at 1 week and 12 weeks post-transplantation. (C) Percentage of lba-1 positive cells at 1 week and 12 weeks post-transplantation. Scale bar, 20 µm. *, P < 0.05 for each comparison. Data are expressed as mean ± S.E.M.

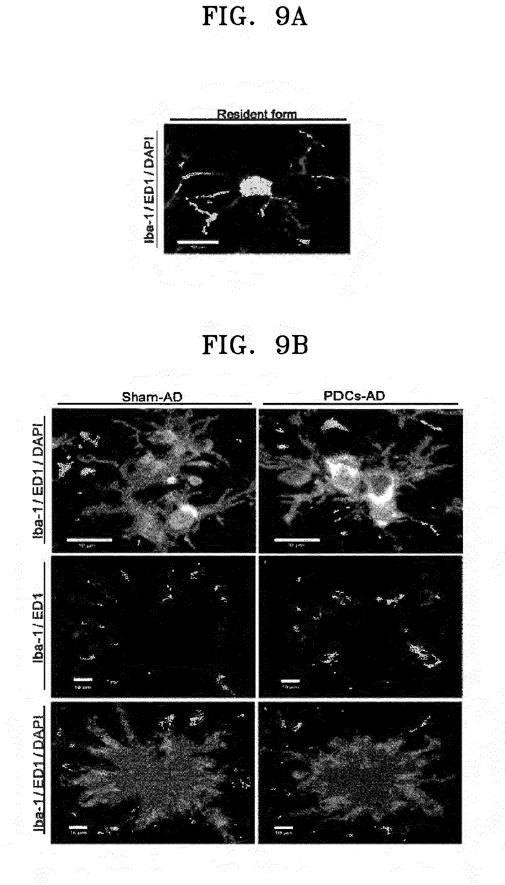

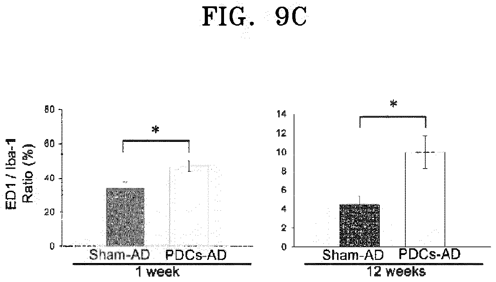

FIG. 9 shows effect of PDC administration on phagocytic activity of microglia. Expression of ED-1(CD68) immunoreactivity (red) in lba-1 positive microglial cells (green). ED1 was expressed in the cytosol of activated microglia around the Aβ plaques (B), but, not in resident microglia (A). (C) Quantitative image analysis of a ratio of ED1 expression out of lba-1 positive microglia at 1 week and 12 weeks post-transplantation. Scale bar, 10 µm. *, P < 0.05 for each comparison. Data are expressed as mean ± S.E.M.

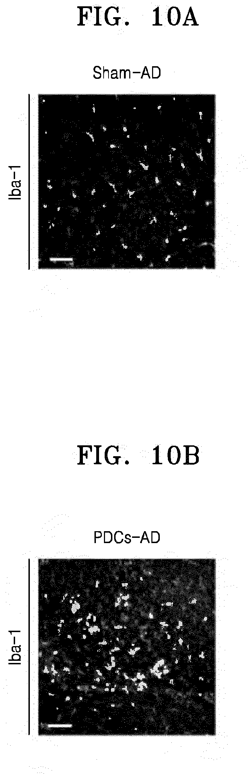

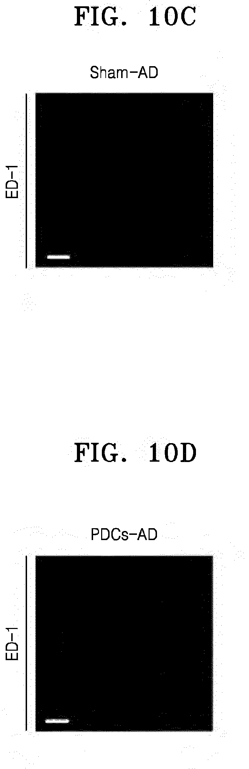

FIG. 10 shows ED-1 immunoreactivity of microglia. No ED-1 activity was detected in resident microglia (A and C). ED-1 immunoreactivity was observed in activated microglia (lba-1) in close proximity to Aβ plaque (B and D). Scale bar, 20 µm.

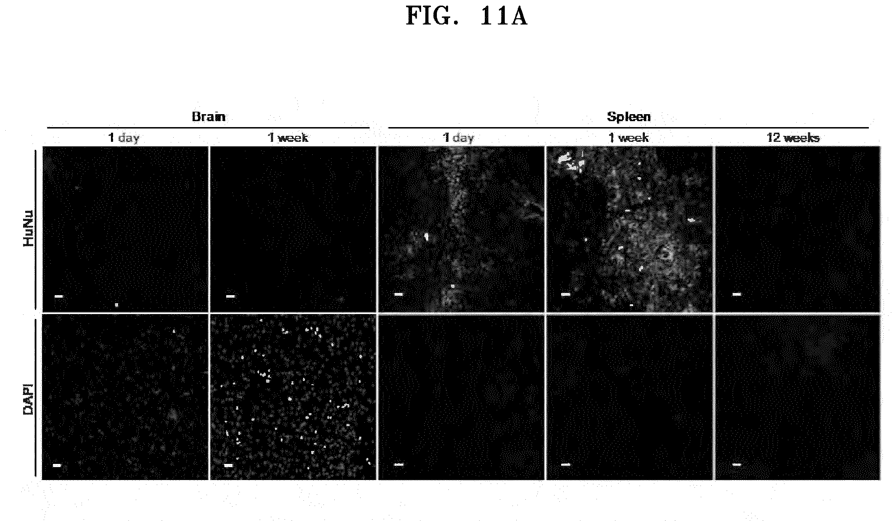

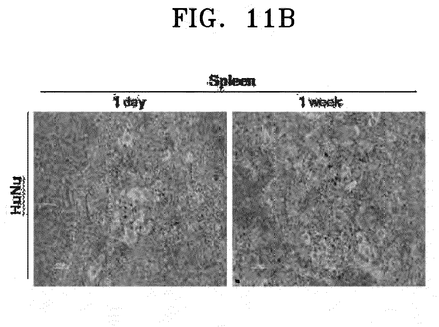

FIG. 11 shows a time course of PDC detection in the brain and spleen. (A) No HuNu were detected in brain tissues at 1 day and 1 week post-injection whereas HuNu were detected in the spleen at 1 day and 1 week post-injection, but not detected at 12 weeks. (B) HuNu positive cells in the spleen were identified by DAB staining at 1 day and 1 week post-injection. Scale bar 20 µm(A); 100 µm(B).

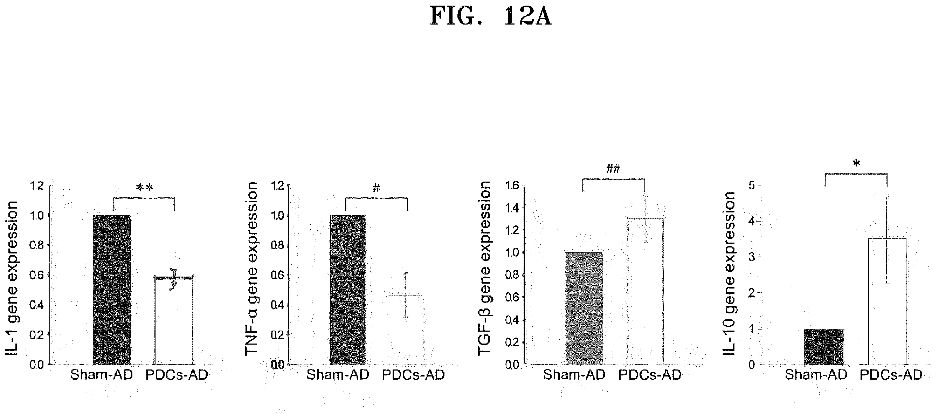

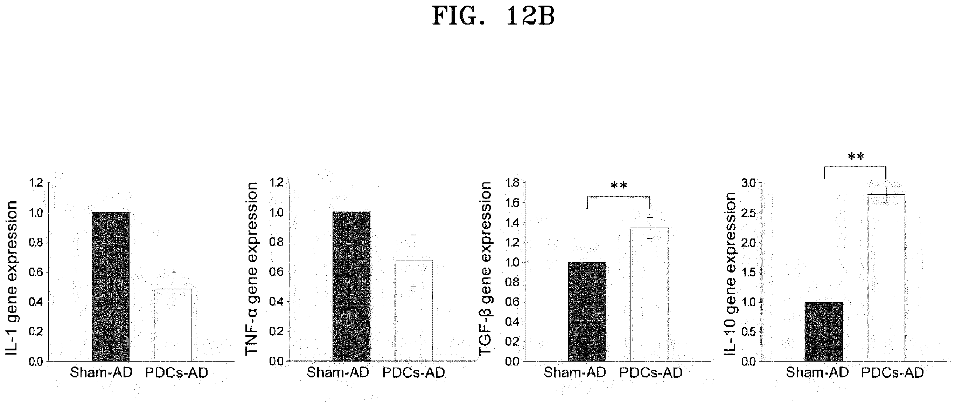

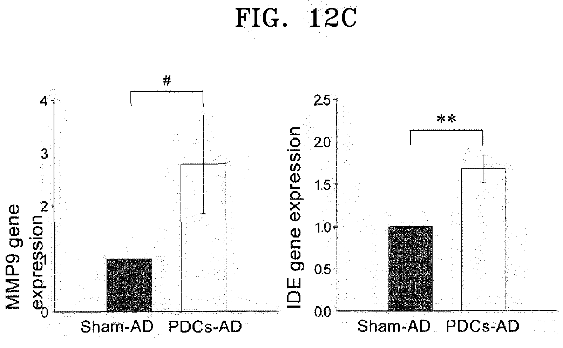

FIG. 12 shows effect of PDC administration on inflammatory cytokines and proteolytic enzyme release, and is result of quantifying mRNA expression levels of the inflammatory cytokines and proteolytic enzymes in PBS or PBS-injected APPswe mice. (A) mRNA expression levels of IL-1, TNF-α, transforming growth factor (TGF-β), and interleukin 10 (IL-10) at 1 week post-injection, (B) mRNA expression levels of IL-1, TNF, TNF-α, TGF-β and IL-10 at 12 weeks post-injection, (C) mRNA expression levels of matrix-metalloproteinase 9 (MMP9) and insulin-degrading enzyme (IDE) at 1 week post-injection. **, P < 0.01; *, P < 0.05; #, P < 0.06; ##, P < 0.08 for each comparison. Data are expressed as mean ± S.E.M.

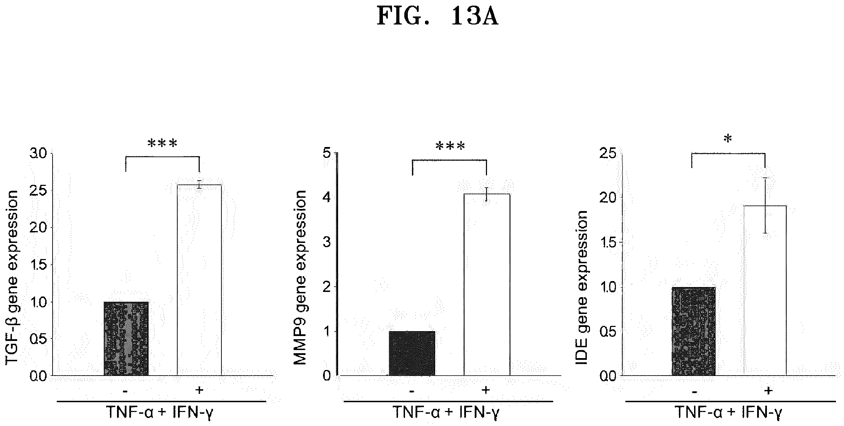

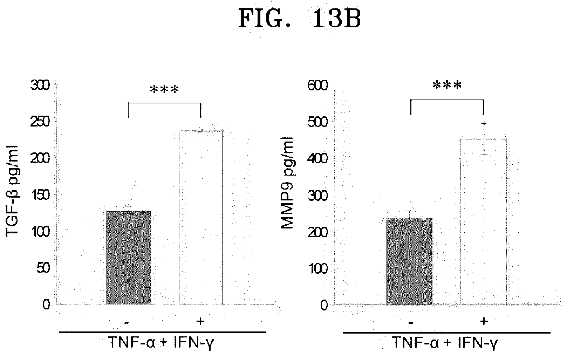

FIG. 13 shows in-vitro response of PDC to inflammatory stimulation. (A) Comparison of mRNA expression of TGF-β, MMP9, and IDE in PDCs stimulated with TNF-α and IFN-γ and in unstimulated PDCs. (B) Comparison of protein expression of TGF-β and MMP9 in PDCs stimulated with TNF-α and IFN-γ and in unstimulated PDCs by ELISA. ***, P<0.001; **, P<0.01; *, P<0.05 for each comparison. Data are expressed as mean ± S.E.M.

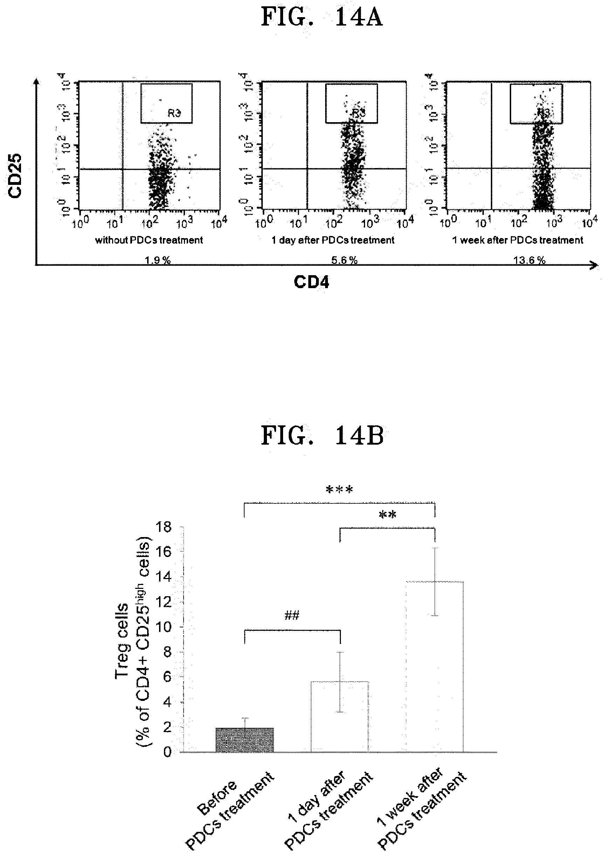

FIG. 14 shows effect of PDC administration on regulation of regulatory T cells (Treg) in Alzheimer mice, and shows distribution of regulatory T cells (Treg) in spleen mononuclear cell (SMNC). (A) Treg CD4⁺CD25^{high} cells in salt- or PDC-injected Alzheimer mice at 1 day and 1 week post-injection. (B) A percentage of Treg(%) at each time point, compared with salt-injected mice. ***, P < 0.001; **, P < 0.01; ##, P < 0.08 for each comparison. Data are expressed as mean ± S.E.M.

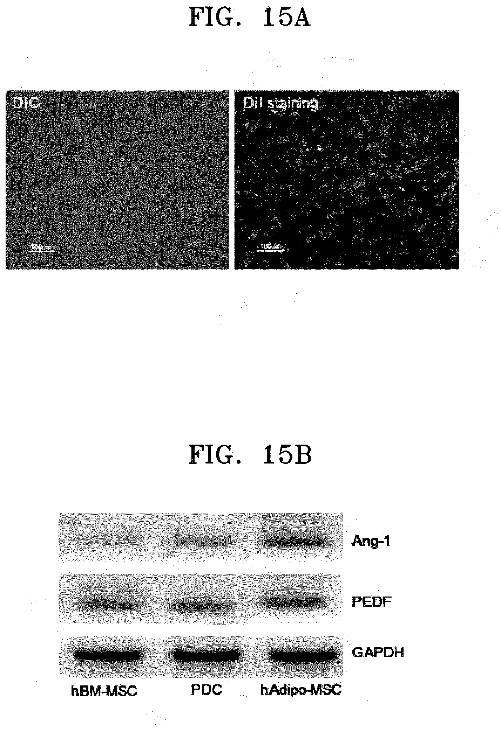

In FIG. 15, (A) morphology of PDCs obtained at passage 6 (left panel) and Dil-stained PDCs (right panel). (B) Comparison of Angiopoietin-1 (Ang-1) and Pigment epithelium-derived factor (PEDF) in human Bone marrow-MSC (hBM-MSC), hPDC, and human Adipocyte-MSC (hAdipo-MSC).

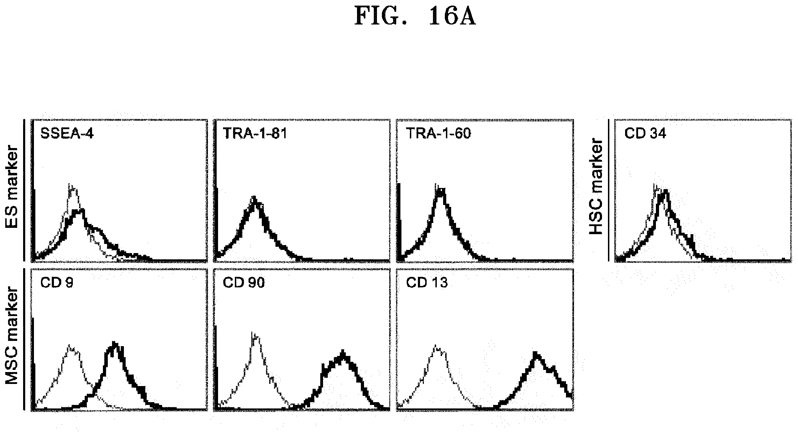

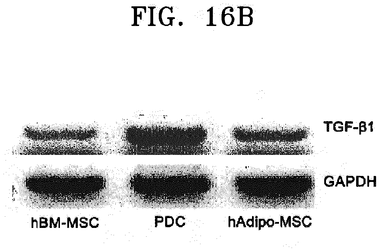

FIG. 16 shows characterization of PDCs. (A) Result of FACS analysis of the immunophenotypic profiles of PDCs. PDCs were negative for SSEA4, TRA-1-81, and TRA-1-60 (ES markers) and CD34 (hematopoietic and endothelial cell markers), and positive for CD9 (nontrophoblast marker) and CD13, CD90 (MSC markers). (B) Comparison of TGF-β expression in PDC, hBM-MSC and hAdipo-MSC.

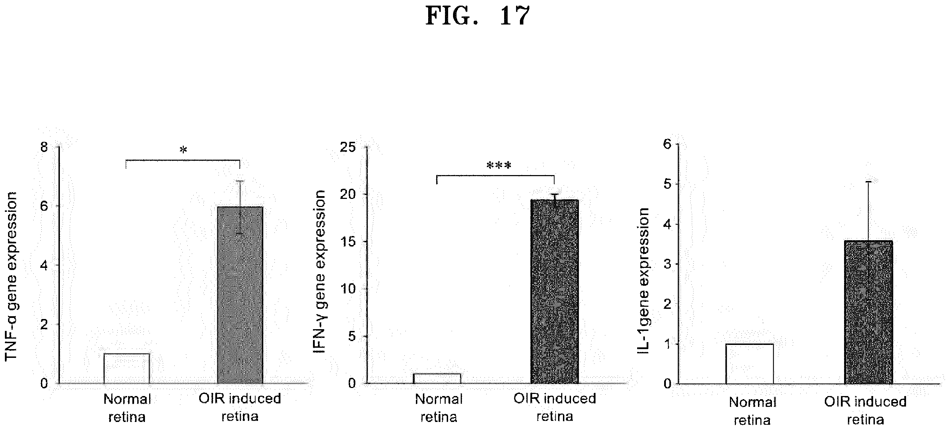

FIG. 17 shows profile of proinflammatory cytokines in the retinas of OIR-induced mice. Proinflammatory cytokines were increased in retinas of the OIR-induced group, as compared with a control group. IFN-γ and TNF-α levels were significantly increased. ***, p<0.001; *, p<0.05 for each comparison. Data are expressed as mean ± S.E.M.

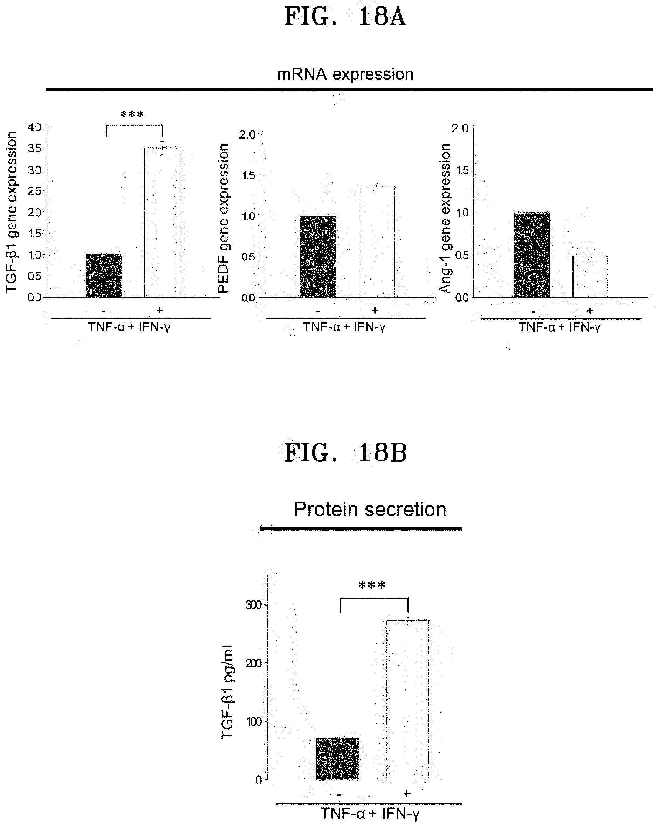

FIG. 18 shows response of PDCs to pathological conditions in vitro. (A) In vitro mRNA expression of antineovascular factors was determined by real-time PCR after stimulation with IFN-γ and TNF-α. (B) TGF-β levels were significantly increased after stimulation, while other factors were not changed. Protein levels of TGF-β in the culture supernatant of PDCs were determined by ELISA. TGF-β levels in the culture supernatant of stimulated PDCs were significantly increased, compared with normal cells. ***, p<0.001 for each comparison. Data were normalized to the level (=1.0) of mRNA in control cells (-) and expressed as the means ± S.E.M.

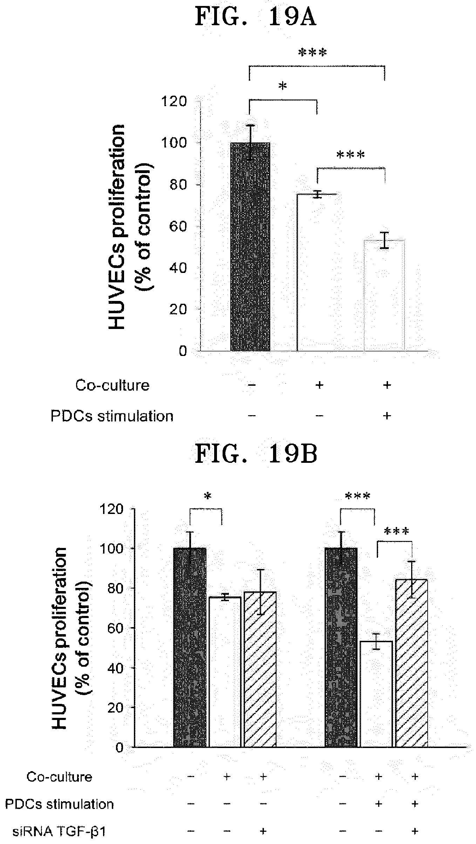

FIG. 19 shows inhibitory effect of PDCs on human umbililcal vein endothelial cell (HUVEC) proliferation. (A) HUVECs co-cultured with PDCs exhibited decreased proliferation, compared with HUVECs cultured alone. PDCs pretreated with IFN-γ and TNF-α showed greater suppressive effects on HUVEC proliferation than normal PDCs. (B) Transfection of TGF-β siRNA blocked the suppressive effects of IFN-γ- and TNF-α-treated PDCs, but the suppressive effects of untreated normal PDCs were not affected by transfection of TGF-β siRNA. ***, p<0.001; *, p<0.05 for each comparison. Data are expressed as the means ± S.E.M.

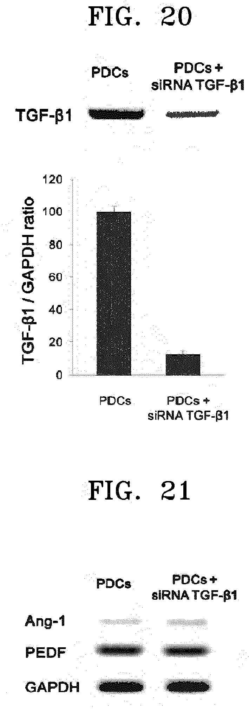

FIG. 20 shows effect of TGF-β-specific siRNA in PDCs. RNAs were extracted from TGF-β siRNA-transfected PDCs or normal PDCs, and mRNA expression of TGF-β was analyzed. TGF-β expression levels significantly decreased in TGF-β siRNA-transfected PDCs. ***, p<0.001 for each comparison. Data are expressed as the means ± S.E.M.
FIG. 21 shows PEDF and Ang-1 expression after TGF-β siRNA transfection. PEDF and Ang-1 expression levels were not changed after TGF-β siRNA transfection.

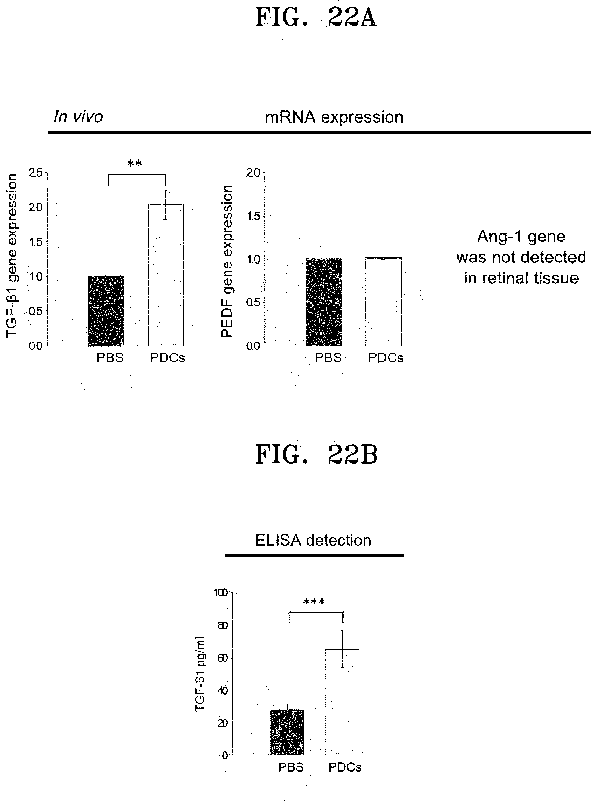

FIG. 22 shows effect of PDCs on expression of antineovascular factors in vivo. (A) The mRNA expression levels of antineovascular factors in the retina were determined by quantitative real-time PCR. Expression of TGF-β was significantly increased in the retinas of PDC-treated mice. (B) The protein levels of TGF-β in the mouse retina were determined by ELISA. The TGF-β levels in the retinas of PDC-treated mice were significantly increased, compared with PBS-treated mice. ***, p<0.001; **, p<0.01 for each comparison. Data were normalized to the level (=1.0) of mRNA in control mice (PBS-treated) and expressed as the means ± S.E.M.

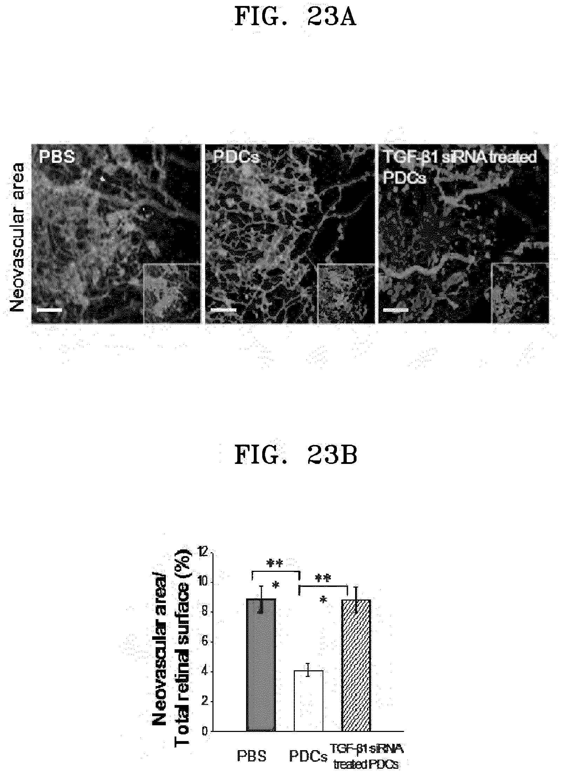

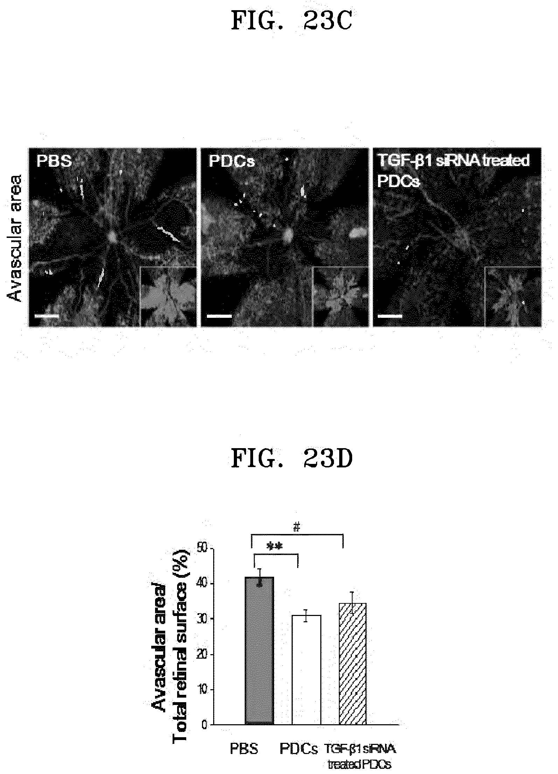

FIG. 23 shows effect of PDCs on retinal neovascularization. Representative retina flat mounts were prepared at P17. Areas of retinal neovascular tuft formation (magnification×200, A) and vaso-obliteration (magnification×40, C) were determined by computer-assisted image analyses. Quantified areas are shown in gray (inset). Analyzed data show the percentage of areas of vaso-obliteration (B) and neovascular tuft formation (D) in the retina. Vaso-obliteration and neovascular tuft formation were reduced in the PDC-treated group, compared with a control group. TGF-β siRNA-treated PDCs failed to reduce neovascular tuft formation, compared with nontransfected PDCs, but vaso-obliteration was slightly reduced in the retinas of mice treated with TGF-β siRNA-treated PDCs, compared with the control group. ***, p < 0.001; **, p < 0.01; *, p < 0.05; #, p < 0.06 for each comparison. Data were expressed as the means ± S.E.M.

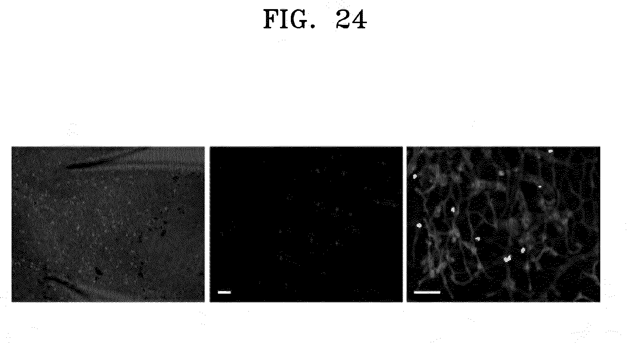

FIG. 24 shows tracking of PDCs in the retina in-vivo. The migrated PDCs were detected in the retina at 5 days after injection. The left panel shows a merged image of DIC (gray) and Dil-labeled PDCs (red). The middle panel shows a merged image of DAPI (blue) and Dil-labeled PDCs (red). The right panel shows a merged image of a GS-lectin-stained vessel (green) and Dil-labeled PDCs (red). Scale bar = 20 µm.

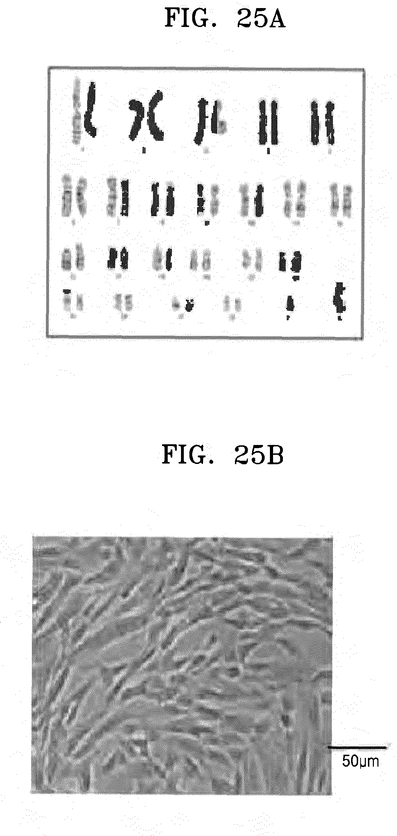

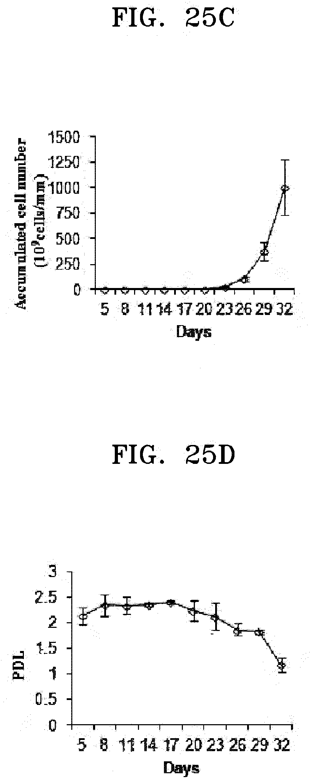

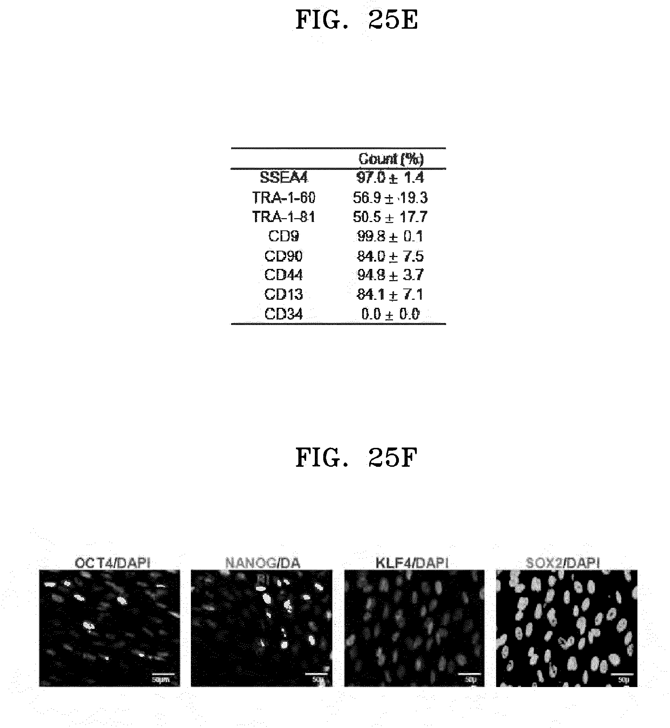

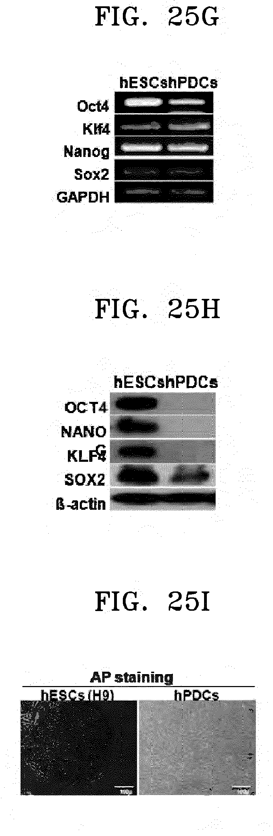

FIG. 25 shows characteristics of human placenta-derived cells (hPDCs) at passage 1. (A) Karyotyping of hPDCs, (B) Morphology of hPDCs, (C) Growth curve of hPDCs, (D) Population of doubling level (PDL) of hPDCs, and (E) FACS analysis of the immunophenotypic profiles of hPDCs. hPDCs were negative for CD34 (hematopoietic cell and endothelial cell marker), and positive for SSEA4, TRA-1-60, and TRA-1-81 (ES markers), and CD9 and CD44, CD13, CD90 (MSC markers). (F-H) mRNA and protein levels of stem cell markers such as OCT4, NANOG, KLF4 and SOX2 analyzed by RT-PCR, immunofluorescence staining, and immunoblotting. (I) Result of alkaline phosphatase (AP) assay. Scale bar = 100um.

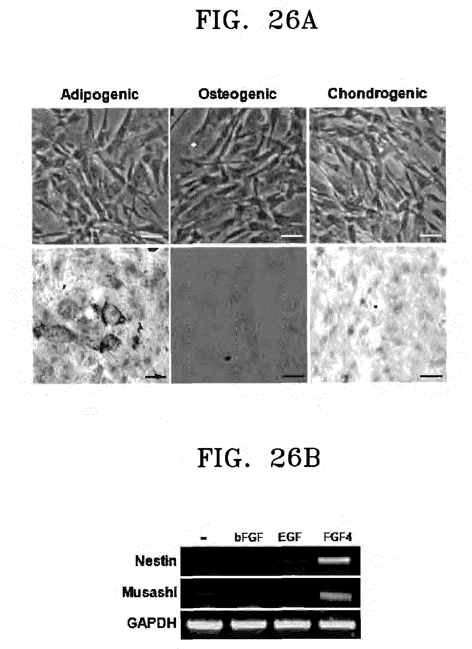

FIG. 26 shows a differentiation potential of hPDCs. (A) Differentiation potential of hPDCs. Oli Red O staining was used for adipogenic differentiation, Von Kossa staining was used for osteogenic differentiation, and Alcian blue staining was used for chondrogenic differentiation. scale bar = 5 mm. (B) hPDCs were treated with the indicated growth factors (10 ng/ml bFGF, 10 ng/ml EGF, 25 ng/ml FGF4)) for 3 days. mRNA expression of Nestin and Musashi under 25 ng/mL FGF4 condition was detected by RT-PCR.

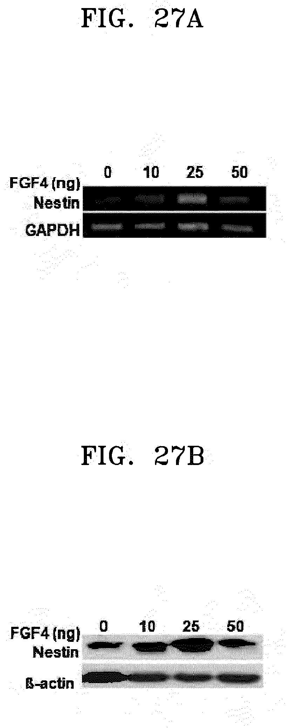

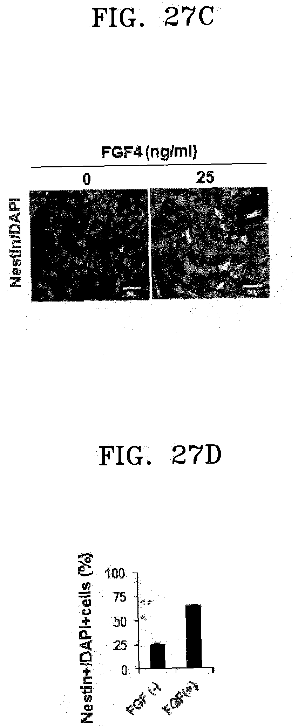

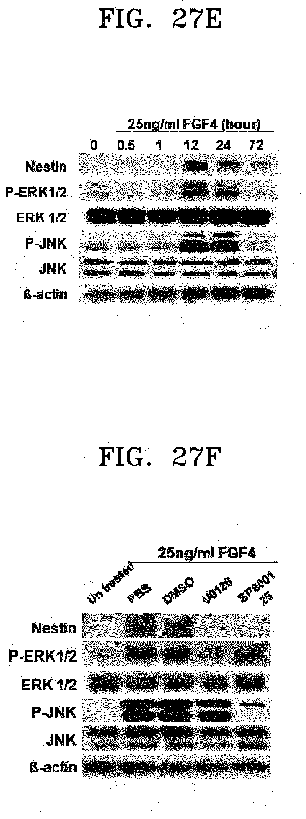

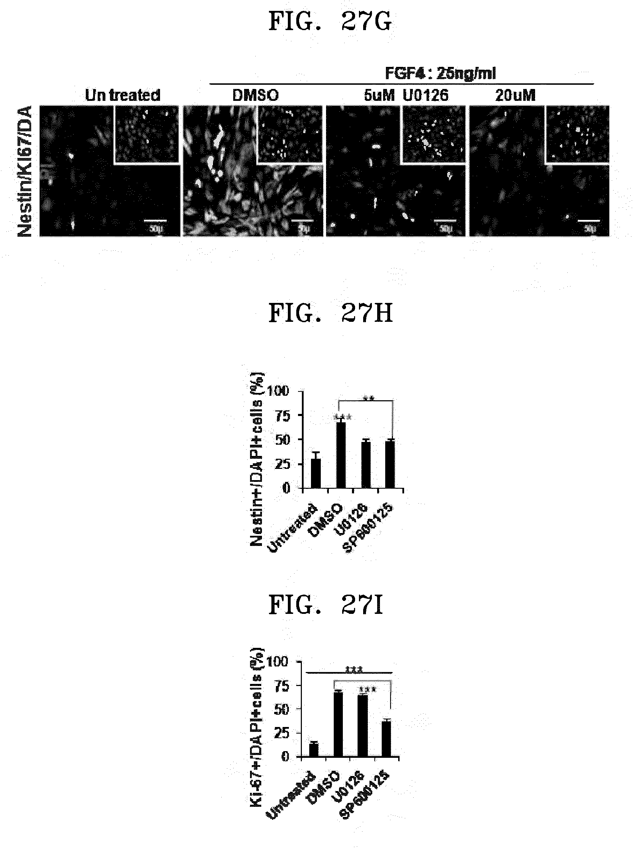

FIG.27 shows direction induction of hPDCs into neural progenitor cells by FGF4. hPDCs were treated with FGF4 of indicated concentrations for 3 days. mRNA of Nestin (A) was analyzed by RT-PCR, and a protein level of Nestin was analyzed by (B) immunoblot analysis and (C) immunofluorescence staining. mRNA and protein levels of Nestin were significantly increased at 25 ng/mL of FGF4. Scale bar = 100 um. ***, P < 0.0001 for each comparison. Data were expressed as the means ± S.E.M. (E) FGF4-mediated ERK1/2 and JNK expression and activity were analyzed by immunoblotting using an appropriate antibody for a short period of time after treatment of hPDCs with FGF4. (F to I) hPDCs were cultured for 3 days in the presence of FGF4, together with U0126 and SP600125. Next, the cells were analyzed by immunoblotting and immunofluorescence staining with antibody. Nestin protein was reduced in U0126 and SP600125. A proportion of Ki-67 was decreased by JNK inhibition. Scale bar = 100 µm. ***, P < 0.0001 for each comparison. Data were expressed as the means ± S.E.M.

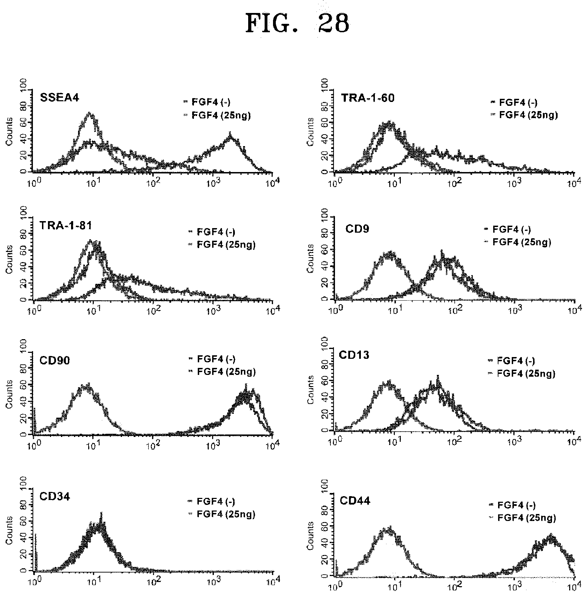

FIG. 28 shows result of FACS analysis of the immunophenotypic profiles of hPDCs in FGF4(-) or FGF4(25 ng). hPDCs were negative for CD34 (hematopoietic cell and endothelial cell marker), and positive for CD9 and CD44, CD13, CD90 (MSC markers). Expression of SSEA4, TRA-1-60, and TRA-1-81 (ES markers) was decreased in the presence of FGF4.

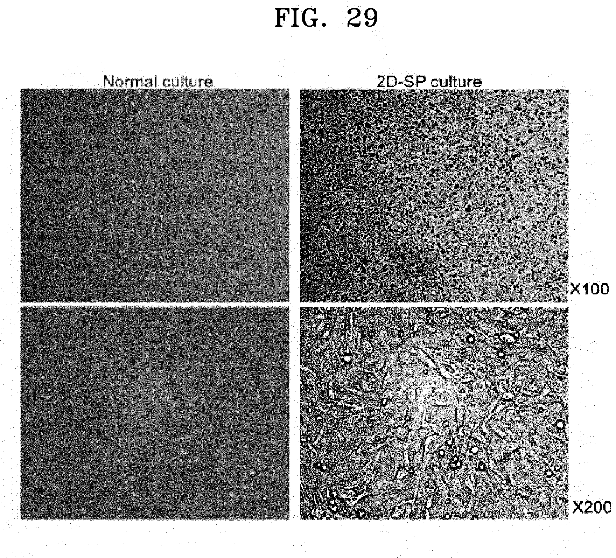

FIG. 29 shows hPDCs cultured in an induction medium containing FGF4 in a high-density (HD) culture system. hPDCs under normal culture conditions and in the HD culture system were visualized by time-lapse digital imaging using a microscope.

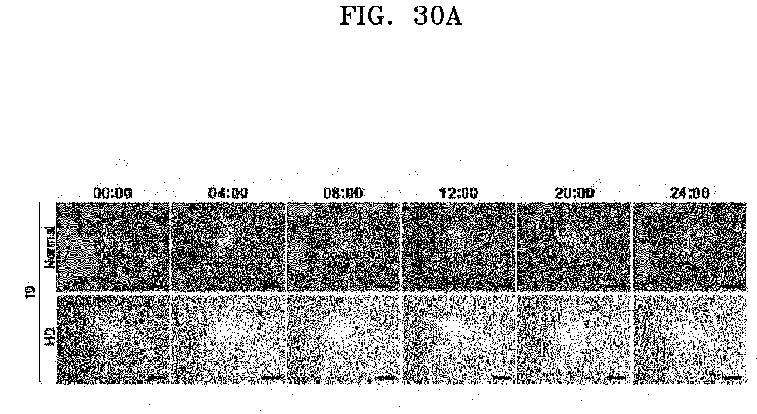

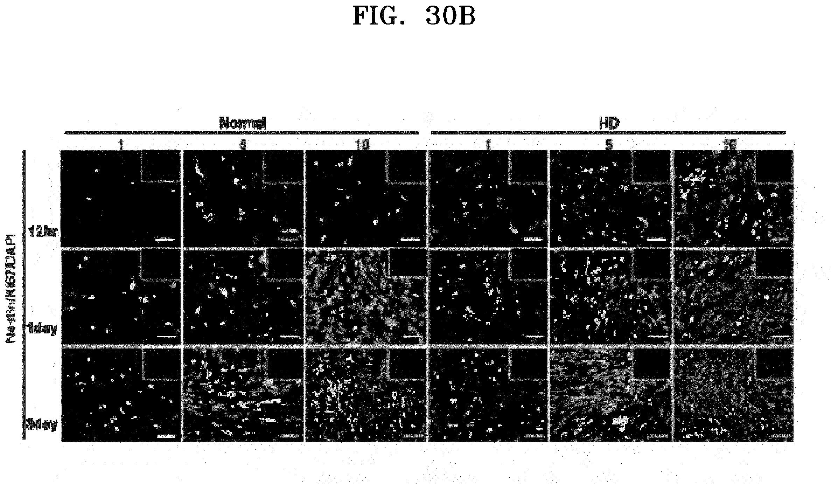

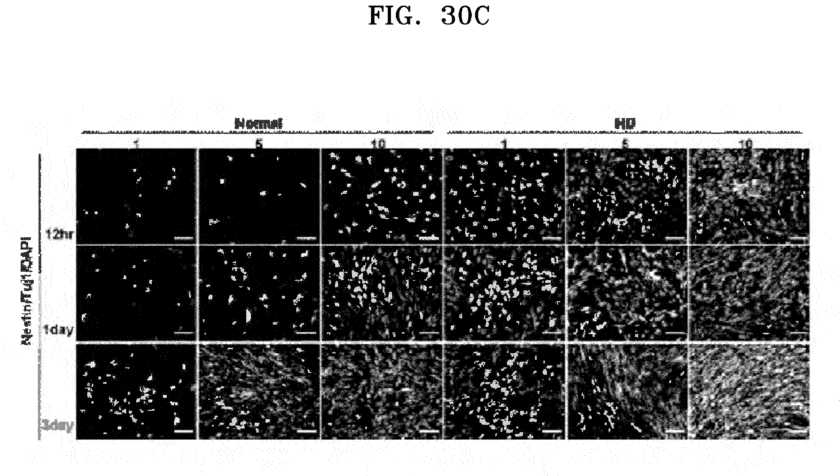

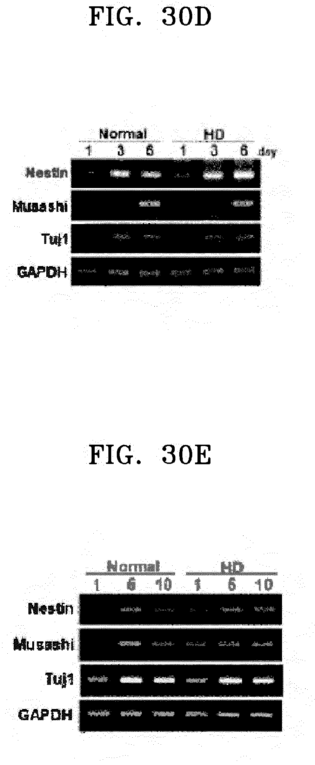

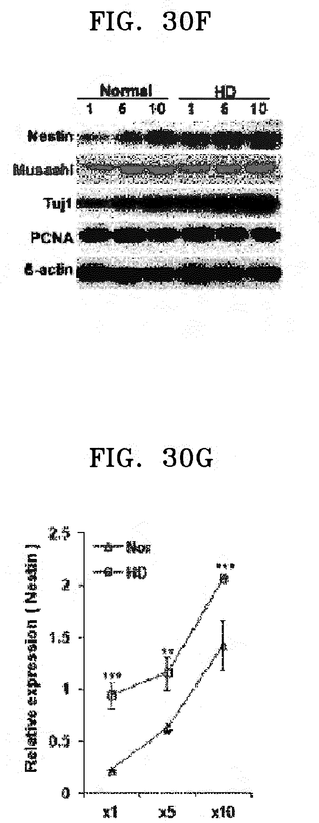

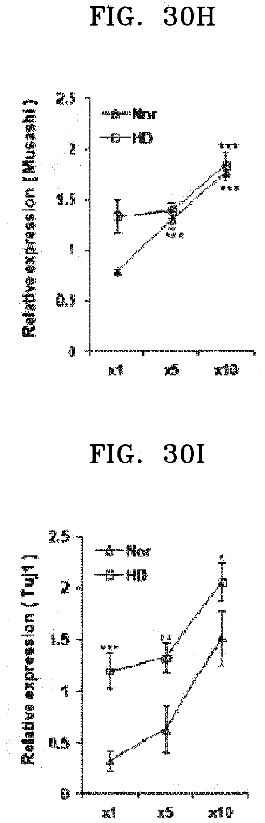

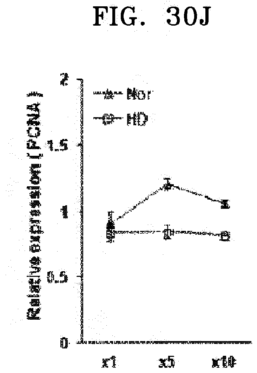

FIG. 30 shows that cell-cell contact induces a high level of neuronal cells in a high-density culture system. (A) Morphology of hPDCs in the HD culture system for the indicated time. (B, C) Result of immunofluorescence staining of hPDCs in an induction medium containing FGF4 in the HD culture system or under normal conditions for 12 hours, 1 day, and 3 days by using antibodies against Nestin, Ki-67, and Tuj1. Indicated cell density: 1,000 cells/cm2(1x) and 10,000 cells/ cm2(10x). Scale bar = 100um. (D to E) Result of analyzing mRNA levels of Nestin, Musashi, and Tuj1. Expression of (F, G) Nestin, (F, H) Musashi, and (F, I) Tuj1 was increased under HD culture and at a high density of normal conditions. PCNA expression was slightly decreased under HD culture (F, J). ***, P < 0.0001 for each comparison. Data were expressed as the means ± S.E.M.

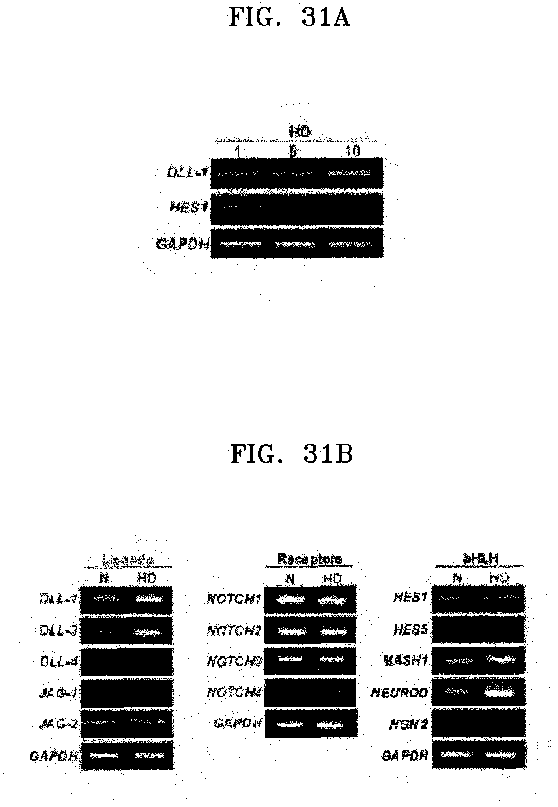

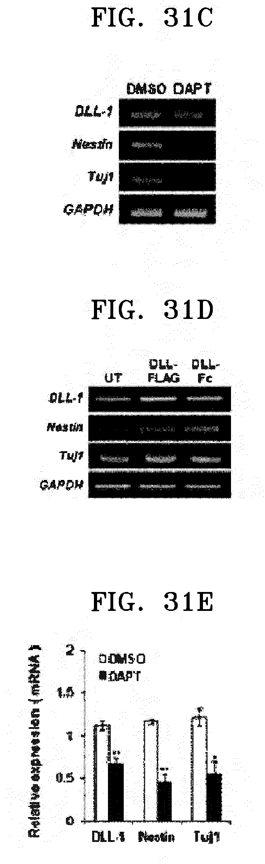

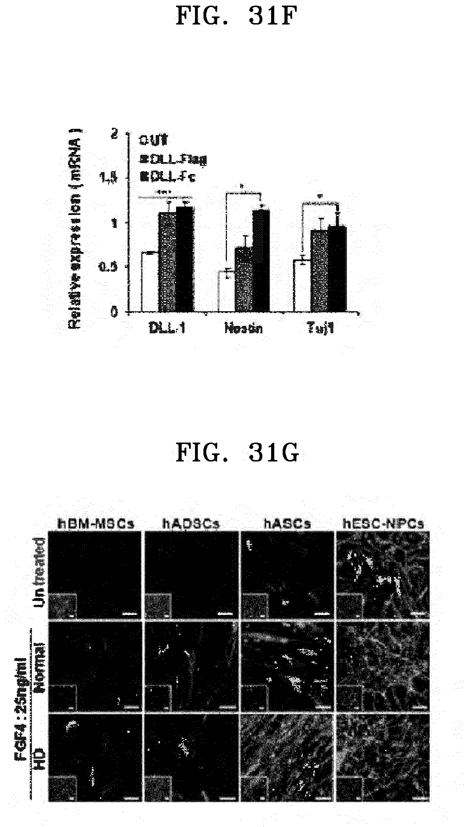

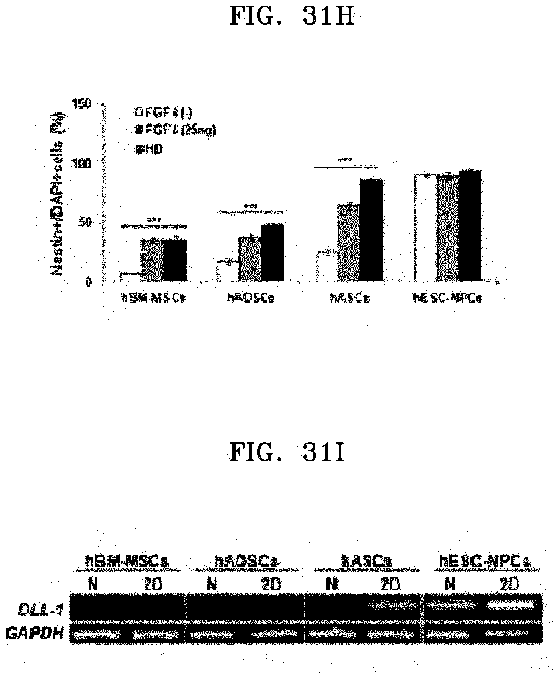

FIG. 31 shows that cell-cell contact induces a high level of neuronal cells by increasing DLL1 expression in NPCs. (A) expression levels of genes related to Delta-like 1 and notch receptor in hPDCs cultured under HD culture or normal conditions. (B) DLL-1 and HES1 gene expression levels in the HD culture system. (C, E) mRNA levels of DLL-1, Nestin, and Tuj1 in the presence of DAPT. (D, F) mRNA levels of Nestin and Tuj1 in DLL-1-overexpressed hPDCs by DLL-FLAG and DLL-Fc. (G-H) effect of FGF4 in hPDC-, human bone marrow-, and adipose-derived MSCs (positive control: human fetal NPC). A high proportion of Nestin-positive cells is present in hPDCs, compared with other adult stem cells. (I) mRNA of DLL-1 was expressed only in hPDCs under HD culture system. Scale bar = 100um. ***, P < 0.0001 for each comparison. Data were expressed as the means ± S.E.M.

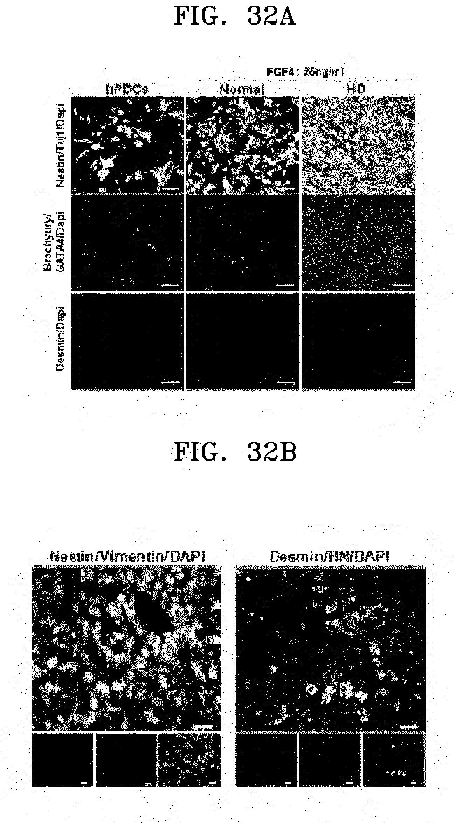

FIG. 32 shows result of culturing hPDCs for 3 days under FGF4(-) or FGF4(+) condition in 2D sphere culture system. Nestin, Tuj1 and other lineage differentiation proteins, Brachyury, Desmin, and GATA4 were detected in cells by immunofluorescence staining. scale bar = 100 um.

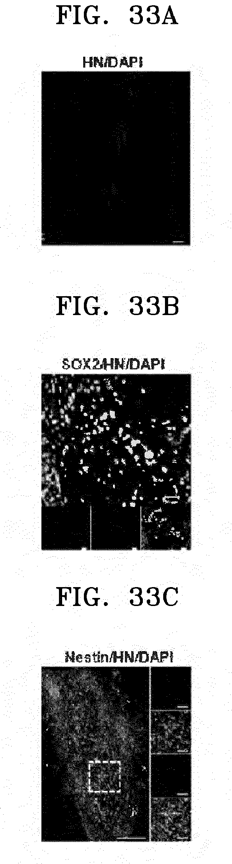

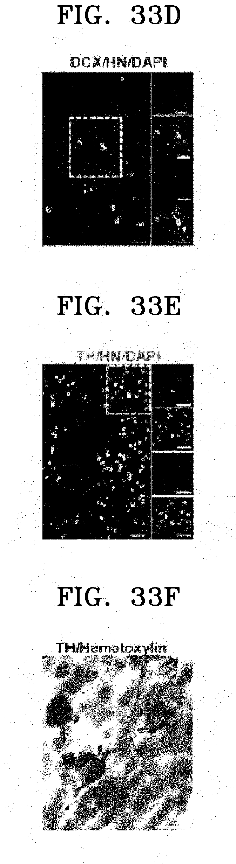

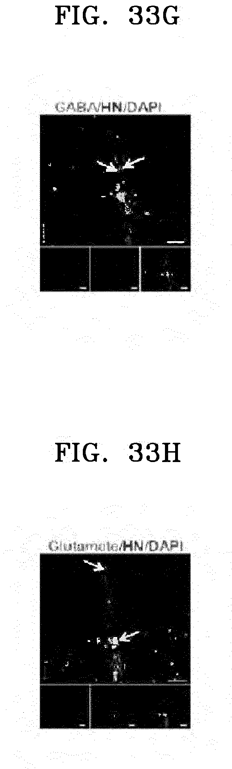

FIG. 33 shows characteristics of cells transplanted into Parkinson rat models. (A) a pluripotent stem cell marker, Sox-2, (B) a neuronal progenitor cell marker, Nestin, (C) a migration marker, DCX, (E) a neuron marker, TH in a transplanted site and a migrated site.

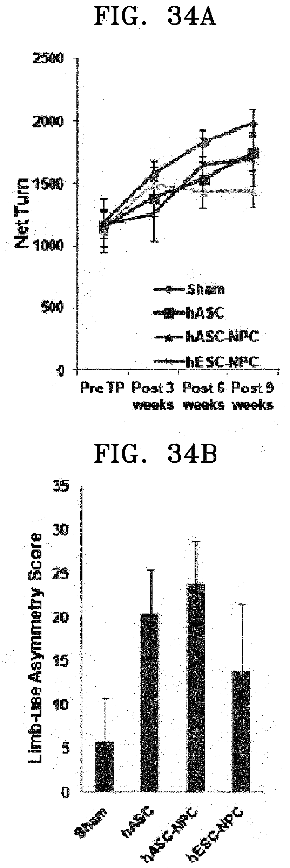

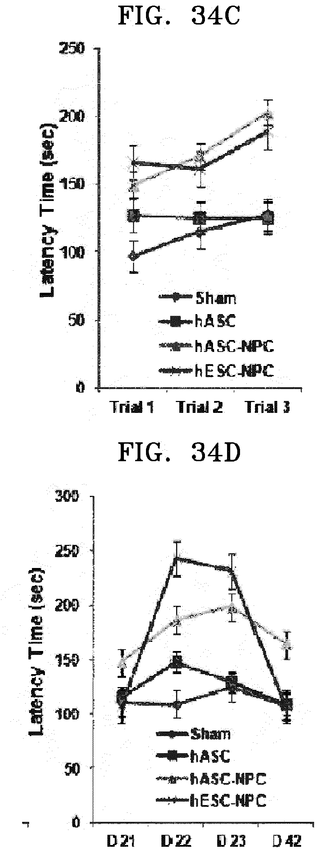

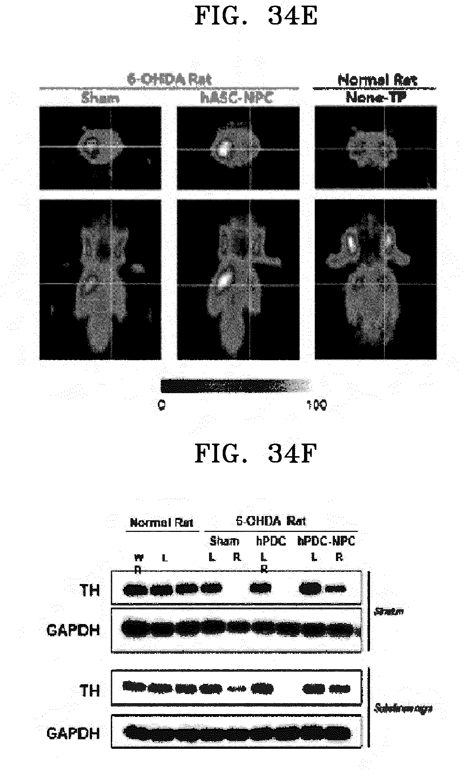

FIG. 34 shows functional recovery measured by a motor behavior task of hPDC-transplanted 6-OHDA rats and PET image analysis. (A) Behavioral assays by a rotation test for 90 minutes: Amphetamine(5mg/kg, i.p.)-induced rotational scores. (B) Cycling asymmetric scores in a cylinder Task. (C-D) Motor learning test by a rotarod experiment at 3 weeks and 6 weeks post-transplantation. For the short-term motor memory experiment, three trials performed in a first session were analyzed. For the long-term motor memory experiment, the time spent on the rod for three consecutive days after 21-24 days and 42 days post-transplantation was analyzed. (E) PET images showing that BP was increased after infusion of placenta-derived cells. Coronal and transaxial 18F FP-CIT PET image slices of dopaminergic impairment model (sham control), placenta-derived cells-induced neural progenitor cells (hPDC-NPC), and normal control group (not transplanted).

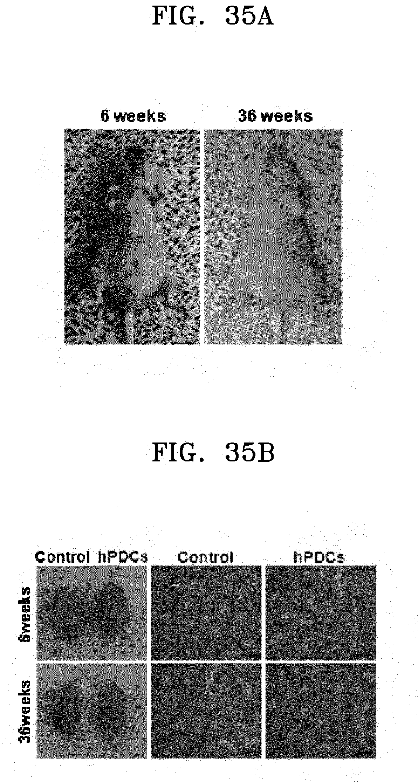

FIG. 35 shows result of analyzing teratoma formation. (A-B) A safety test; 5×105 cells of hPDC-NPCs in 10 µl were injected into BALB/c-nu Sic male mice (Central Lab. Animal Inc, Korea). Results were measured at 6 weeks and 36 weeks after cell injection, and no teratoma formation was observed.

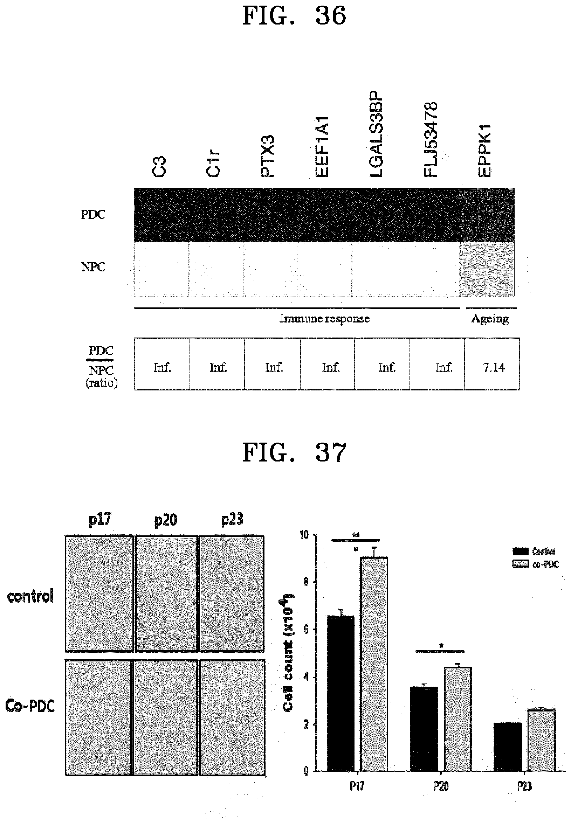

FIG. 36 shows comparison of protein expression levels in CM (conditioned medium) of placenta-derived cells and placenta-derived neural progenitor cells. In figures, Inf. (infinite) represents protein expression only in MSCs.
FIG. 37 shows a decrease in a senescence marker beta-galctosidase (20 and 23 generations) and an increase in the number of cells (17 and 20 generations) in LL-24 after co-culture with hPDCs.

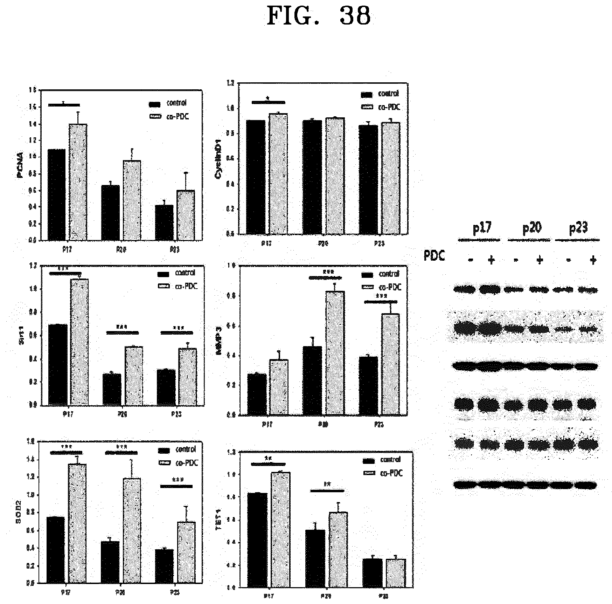

FIG. 38 shows changes of senescence marker expression and changes of proteins in LL-24 after co-culture with hPDCs.

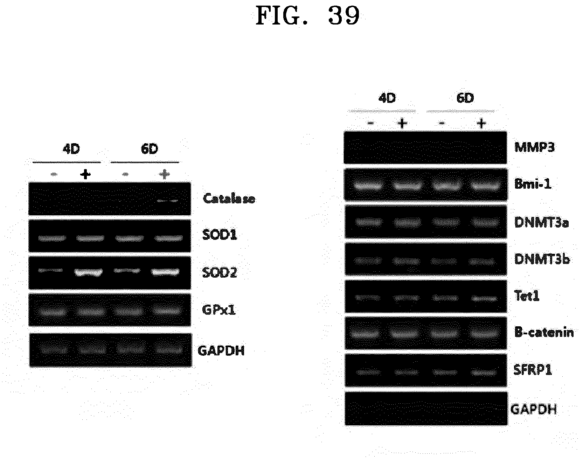

FIG. 39 shows increased expression of antioxidant markers and epigenetic markers in hNPCs after co-culture with hPDCs.

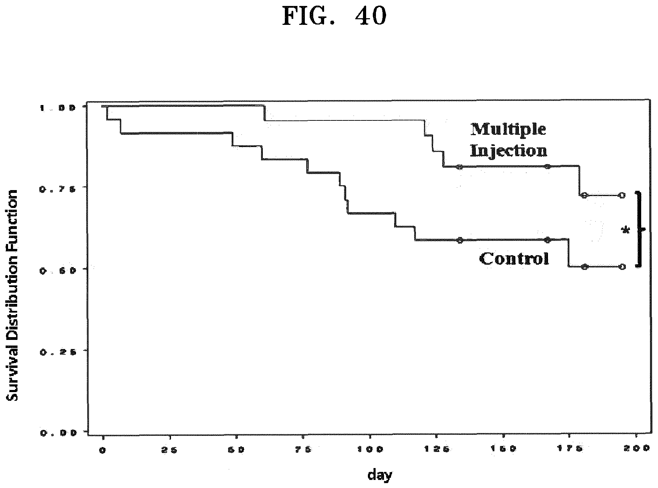

FIG. 40 shows increased survival rates of senescent mice administered with hPDCs.

### MODE OF THE INVENTION

Hereinafter, the present invention will be described in more detail with reference to Examples. However, these Examples are for illustrative purposes only, and the scope of the present invention is not intended to be limited by these Examples.

### Example 1. Culture and Characterization of Placenta-Derived Cells

### 1.1. Isolation and Culture of Placenta-Derived Cells

Normal human placenta (= 37 weeks of gestation) without symptoms of internal, obstetric, or surgical complications was obtained after cesarean section. The placenta was carefully dissected, washed several times with PBS, mechanically ground, and digested with 0.5% collagenase IV (Sigma, St. Louis MO, USA) for 30 min at 37°C. The recovered cells were cultured in alpha-MEM supplemented with 10% fetal bovine serum (Gibco, Grand Island, NY, USA), each 100 µg/ml penicillin and streptomycin, 25 ng/ml FGF4 (R&D system, Minneapolis, MN, USA), and 1 µg/ml heparin (Sigma) in a T25 flask (Nunc, Rochester, NY, USA). Cells were cultured at a density of 1 × 104 cells/cm2 in a dish coated with polyornithine (Sigma) and 4 µg/ ml fibronectin (Sigma). A complete medium containing 25 ng/ml FGF4 and 1 µg/ml heparin was added and the cells were incubated for 6 days at 37°C under the condition of 3% CO2.

In each experiment, the cells were at about 80% density. To track the injected cells, PDC was incubated with 2 mM CM-Dil(1,1'-dioctadecyl 3,3,3',3'-tetramethy lindocarbocyanine pechlorate)(Molecular Probes, Eugene, OR) which is a lipophilic membrane-bound fluorescent dye at 37°C for 30 minutes (FIG. 15).

### 1.2. Analysis of Proteins Expressed In Placenta-Derived Cells

4 ×105 cells/mm3 of placenta-derived cells were counted, and cultured for 3 days in a 100 mm dish containing Alpha-MEM supplemented with 10% fetal bovine serum (FBS), 1% penicillin/streptomycin, 25 ng/mL FGF4, and 1 µg/mL heparin. Next, cells were washed with a serum-free (ITS or Alpha-MEM) medium supplemented with 1% penicillin/streptomycin, and then 10 ml of serum-free (ITS or Alpha-MEM) medium was added to the placental cells. 2 days later, a supernatant was filtered by using a 0.22um filter and collected (conditioned medium). The obtained conditioned medium (CM) was stored at 4°C, and then used.

To measure complement proteins, protein fragmentation was performed by trypsin treatment to prepare peptides, and a protein pellet was dissolved in 1 ml of 50 mM ammonium bicarbonate to reduce a final concentration up to 5 mM. For alkylation, proteins were treated with 14 mM iodoacetamide, and then excessive iodoacetamide was removed by DTT treatment such that a final concentration of the protein was 7 mM. The reduced and alkylated proteins were removed by treatment with trypsin at 37°C for one day. Lastly, 2% formic acid was added and trypsin-treated peptides were dried. The trypsin-treated peptides were desalted by a Sep-Pak C18 cartridge, followed by MS (mass spectrometer) analysis. The cartridge was washed with 100% acetonitrile, and calibrated with 0.1% formic acid. The trypsin-treated peptides were dissolved in 0.5% formic acid, and put in the cartridge, followed by washing with 0.1% formic acid and 5% MEOH. Then, two chemical materials were extracted by treatment of 70% acetonitrile, and peptides were dried and stored at -80°C.

All measurements were performed in a V-mode, and analysis was performed using a positive mode Nano ESI. Calibration was performed by using a fibrinogen solution at a concentration of 400 mol/µl, and LCMS data were collected in a DDA mode. LC-MS/MS data were processed with a Protein Lynx Global Server (PLGS). The ion detection, clustering, and normalization were processed using PLGS. The processed data were mapped against data of the IPI human database by using PLGS and MASCOT DAEMON programs. Search settings for mapping were determined by a program and the peptides used in analysis were limited to at least one sequence discrepancy and fragments of cysteine carbamidomethylated and digested with trypsin.

As shown in FIG. 36, the results of examining expressed proteins showed that when protein expressions of CM from the placenta-derived cells according to the present invention and placenta-derived NPC (neural progenitor cell) were compared, C3,C1r, EEF1A1, LGALS3BP (Galectin-3-binding protein), and FLJ53478, which are immune-related proteins, were detected only in the placenta-derived cells of the present invention, whereas a senescence-related protein, EPPK1 detected in the placenta-derived cells was about 7 times higher than that detected in NPC.

### 1.3. Immunophenotyping of Placenta-Derived Cells

For phenotyping, cells were stained with the following antibodies of appropriate concentrations at 4°C for 20 minutes in a FACS buffer: FITC-labeled anti-SSEA4, TRA-1-81, CD34 and PE-labeled anti-TRA-1-60, CD90, CD9, CD13(BD Pharmingen, San Diego, CA, USA); PE-labeled CD200(R&D system). After washing the cells, analysis was performed by using Cell Quest software and a FACSCaliber (BD, San Jose, CA, USA).

FIG. 1 shows morphology of placenta-derived cells (PDC) recovered at passage 4. Flow coefficient analysis of immunophenotypic profile of PDCs showed that PDCs were negative for CD34 (hematopoietic cell and endothelial cell marker), SSEA4, TRA-1-60, and TRA-1-81 (embryonic stem cell markers), and positive for CD9 (a marker of non-trophoblast), and CD13 and CD90. These expression profiles confirmed that the placenta-derived cells of the present invention have multipotency. Moreover, PDCs were positive for a cell surface antigen CD200 (FIG. 1 B). CD200 which is recently recognized as a transmembrane glycoprotein is known to play an important role in immune regulation and tolerance via its receptor CD200R.

Meanwhile, expression levels of anti-neovascular factors (including neovessel stabilization-promoting factors) in PDCs were compared with those of hBM-MSC (bone marrow-derived mesenchymal stem cells) and hAdipo-MSC (adipose-derived mesenchymal stem cells). There was no difference in PEDF and ANG-1 expression (FIG. 15B), but much higher TGF-β expression was observed in PDC than in hBM-MSC and hAdipo-MSC (FIG. 16B).

### Example 2. Effect of Placenta-Derived Cells of The Present Invention on Alzheimer's Disease

### 2.1. In-vitro analysis of PDC Effect

### <Experimental method>

### (1) Treatment of microglial cells with amyloid peptide

In an incubator, BV2 mouse microglial cell line was maintained at 37°C and 5% CO2 in DMEM supplemented with 10% fetal bovine serum (Gibco), 2 mM glutamine, and penicillin-streptomycin (Gibco). Synthetic Aβ25-35 (Sigma) was dissolved in sterile distilled water at a concentration of 1 mM, and incubated at 37°C for 72 hours for aggregation. Treatment of BV2 cells with Aβ25-35 was performed in triplicate. For this purpose, BV2 cells were cultured in a transwell system. hpMSCs (2X105 cells/well) were seeded in a transwell, and BV2 cells (2X105 cells/well) were spread on a 6-well dish, and allowed to adhere overnight. The plates were incubated for 24 hours prior to Aβ25-35 treatment, and BV2 cells were treated with 50 µm amyloid peptide for 24 hours. After treatment of Aβ25-35, NO2- was measured in a culture supernatant to evaluate NO production in BV2 cells. 50 µl of a sample was mixed with 50 µl of a Griess reagent (Promega, Madison, WI, USA) in a 96-well plate, followed by incubation for 10 minutes at 25°C. Absorbance at 550 nm was measured by using a microplate reader.

### (2) Analysis of mRNA and cytokine levels in placenta-derived cells after IFN-γ and TNF-α stimulation

In-vitro stimulation of PDC: PDCs were cultured in 1 ml of DMEM medium (2 X 105 cells/ml) in a 24-well plate, and stimulated with TNF-α (10ng/ml; R&D systems) and IFN-γ (100ng/ml; Peprotech) for 6 hours. 24 hours later, the cells were recovered, and a supernatant was analyzed by an enzyme-linked immunosorbent assay (ELISA) for TGF-β (R&D systems).

Measurement of cytokines: sandwich ELISA for TGF-β and MMP-9 (R&D systems) was performed according to the manufacturer's instructions to measure concentrations of cytokines in PDCs or a culture supernatant 24 hours after stimulation. The concentrations of cytokines were calculated by comparison with known standards. All measurements were performed in triplicate, and all results were expressed as mean ± S.E.M.

### <Results>

### (1) Inhibitory activity of PDCs on Aβ25-35-induced microglial cells

In order to examine whether CD200 expressed in PDCs has an immune regulatory effect on amyloid-activated microglial cells, an immortalized murine microglial cell line, BV2 cells were stimulated with 50 µM of Aβ25-35 peptide which is a neurotoxic domain of a full length Aβ, and then co-cultured with PDCs. After stimulation with Aβ25-35 for 24 hours, the activated microglial cells showed densely packed cell clumps, whereas microglial cells co-cultured with PDCs did not cause clumping (FIG. 2A). In addition, PDCs influenced Aβ25-35 mediated NO production in microglial cells. Normal microglial cells produced a very little amount of nitrite (2.7±0.1 µM). After stimulation of Aβ25-35 for 24 hours, NO production was dramatically increased (21.4±1.2 µM; p<0.001). In contrast, co-culture with AMSCs exhibited a significant decrease in nitrite accumulation (13.4±2.2 µM) (FIG. 2B). Next, influence of PDCs on mRNA expression of TNF-α and IL-1 in activated microglial cells was investigated. As shown in FIG. 2C, LPS (100 ng/ml) caused a marked up-regulation of TNF-α and IL-1 in microglial cells. Compared with vehicle-treated microglial cells, co-cultures with PDCs significantly inhibited LPS-induced overproduction of TNF-α and IL-1β.

### (2) Expression levels of TGF-β and MMP-9 in PDCs after stimulation with proinflammatory cytokines, IFN-γ and TNF-α

Co-administration of exogenous IFN-γ and TNF-α stimulated PDCs in vitro. Quantitative real-time PCR confirmed a significant increase in TGF-β expression after co-administration of IFN-γ and TNF-α (p < 0.001; FIG. 13A). MMP-9 and IDE mRNA levels were significantly higher in PDCs cultured with proinflammatory cytokines than in PDCs cultured without cytokines (p < 0.0009 and p < 0.05, respectively; FIG. 13A). The level of TGF-β was significantly higher in cell lysates (236.70 pg/mL) and supernatants (155.14 pg/mL) from PDCs cultured with proinflammatory cytokines than in cell lysates (126.73 pg/mL) and supernatants (117.63 pg/mL) from PDCs cultured without cytokines (p < 0.03 and p < 0.000006, respectively; FIG. 13B), and the level of MMP-9 was significantly higher in cells lysates (452.09 pg/mL) and supernatants (261.06 pg/mL) from cells cultured with cytokines than in cell lysates (235.59 pg/mL) and supernatants (95.51 pg/mL) cultured without cytokines (p < 0.002 and p < 0.04, respectively). These findings are consistent with an in vivo model, in which the placenta-derived cells of the present invention play a potent immunomodulatory role in Alzheimer's disease (AD) brains.

### 2.2. In-vivo analysis of PDC Effect

### <Experimental method>

### (1) Characterization and treatment of experimental animal

A transgenic mouse model of Alzheimer's disease (AD) was used to evaluate the effects of PDC (aminotic membrane-derived MSC) transplantation. APPswe (Tg2576) mice were obtained from Taconic Laboratory (Germantown, NY, USA). Adult female APPswe mice (15-16 months old; 20 g; 8 mice were injected with PDC, and 6 mice were injected with PBS) were used for the behavioral experiments and for pathological analysis at 12 weeks after transplantation. Also, adult male APPswe mice (12-13 months old; 20 g; 8 mice were injected with PDC, and 6 mice were injected with PBS) were used for additional pathological analysis at 1 week after transplantation. Wild-type littermate mice (normal group, n = 10) were compared with the APPswe groups. To evaluate the immunomodulatory effects of PDCs on AD pathology, 3xTg-AD mice (6-7 months old; 3 female mice at each time point) purchased from Jackson Laboratory (Bar Harbor, ME, USA) were used. All experimental animals were housed in specific pathogen-free conditions, and handled in accordance with an animal protocol approved by the CHA University Institutional Animal Care and Use Committee (IACUC).

### (2) Preparation and injection of human placental cells

The placenta-derived cells isolated in Example 1.1 were cultured at a density of 1×104 cells/cm2 in a dish coated with 15 g/ml polyornithine (Sigma) and 4 µg/ml fibronectin (Sigma). A complete medium supplemented with 25 ng/ml FGF4 and 1 µg/ml heparin was added thereto, and cells were incubated under condition of 3% CO2 at 37°C for 6 days. For intravenous injection, 200 µl of cell suspension (about 2×106 cells) were injected into the tail vein (PDC-injected group). 200 µl of PBS was injected into the tail vein as a control group (PBS-injected group).

### (3) Behavioral evaluation of experimental animal model and recording

### Behavioral evaluation

Exploration-based tasks such as the elevated plus-maze, the light/dark transition test, and the locomotor and open field tests were used to evaluate basal locomotor and anxiety-like behaviors, with some modifications (see later). Finally, cognitive changes were measured using the water maze test (WMT). All behavioral tests were performed 6 weeks after PDC transplantation.

WMT: A tank (1.1 m in diameter) containing a platform (10 cm in diameter) was used for this test. Mice were subjected to 36 trials over the initial 5 consecutive days. Trial blocks, 1, 2, 3, and 4 included 8 trials and the 5th block constituted 4 trials. On Day 6, a single probe trial was included in the trial blocks. On Day 7, all mice were subjected to 6 cued learning tests, which served as a sensorimotor index for each mouse.

Locomotion test: Test was performed in a 40-cm square. Two areas were differentiated within the open field: the periphery and the central area. Mice were placed in the center of the open field and allowed to freely move for 60 minutes while being tracked by an automated tracking system.

Open field test: Test was performed in a 40-cm square with a black background and an inner circular platform 10 cm in diameter, which was illuminated by a lamp. Mice were placed in the center of the open field arena and allowed to freely move for 60 minutes while being tracked by a video-recording system.

Light/dark transition test: A cage (21 X 42 X 25 cm) was divided into sections of equal size by a partition containing a door. One chamber was brightly illuminated by white diodes (390 lux), and the other chamber was dark (2 lux). Mice were allowed to move freely between the 2 chambers for 5 minutes. The total numbers of transitions, the time spent in each chamber, and the latency to enter the light chamber were recorded.

Elevated plus maze: The maze included 4 arms (45 cm in length x 10 cm in width) in the form of a cross: 2 arms were open arms and 2 arms (of the same size) were enclosed by roof and walls. The maze was set up in the test room 50 cm above the floor. For each trial, mice were placed on the central platform facing an open arm and allowed to move freely for 5 minutes. The total number of transitions, the time spent in the each chamber, and the latency to enter the light chamber were recorded.

Video monitoring: The WMT, locomotion test, open field test, and elevated plus maze were assessed and recorded using a charge-coupled device camera (CCD) connected to a video monitor and a computer. The tests were conducted using a video-tracking system (Ethovision; Noldus, Wageningen, Netherlands).

### (4) Separation and treatment of tissue from cell-transplanted mouse and immunohistological analysis thereof

Tissue preparation and thioflavin S staining: Mice were sacrificed at 1 week (PBS-injected group n = 7; PDC-injected group, n = 7) and 12 weeks (PBS-injected group n = 7; PDC-injected group, n = 8) after injection. Mice were anesthetized and immediately cardiac-perfused with PBS. After perfusion, the brains were removed and split into left and right hemispheres for analysis. Left hemispheres were fixed in 4% formalin, and incubated in 30% sucrose before freezing and cutting a freezing cryostat (Leica, Germany). Serial coronal sections at 20 µm intervals were collected from the rostral anterior commissure to the caudal hippocampus as landmarks. Six brain sections per mouse, each separated by 300 µm, were used for quantification. Brain sections were incubated in 0.5% thioflavin S (Sigma) dissolved in 50% ethanol, and then washed twice with 50% ethanol each, once with water, and mounted with a mounting medium. Right hemispheres were stored at -80°C for RNA analysis.

Immunofluorescence analysis: Aβ plaque-free brain areas were selected for morphological analysis of activated microglial cells because the massive infiltration of microglial cells around Aβ plaques made it impossible to accurately assess the morphology of a single microglia. Immunofluorescence staining for microglial cell (lba-1) and CD68 (ED1) was performed by incubating sections with antibodies against (1:500; Wako, Osaka, Japan) and ED1 (1:500; Serotec, Washington, DC, USA) overnight, followed by incubation with Alexa Fluor 488-, or 594-conjugated secondary antibodies for 1 hour at room temperature. Double immunofluoresence staining of microglial cells, CD68, and astrocytes was performed using an antibody against human nuclei (HuNu) (1:100; Millipore, Billerica, MA, USA). Normal goat serum (isotype control), or PBS (0.1 M, pH 7.4) were used instead of primary antibody for the negative controls. Human nuclear (HuNu) staining was performed using the horseradish peroxidase (HRP)-diaminobenzidine (DAB) method. Images were acquired using a microscope equipped with a confocal microscope (Zeiss, Oberkochen, Germany), and for quantitative analyses, Adobe Photoshop CS5 software was used. Aβ plaques were classified according to their size, and counted. Large (>100 mm in diameter), middle-sized (50-100 µm in diameter), and small (<50 mm in diameter) plaques were detected and counted in the cortex and hippocampus by confocal microscopy.

### (5) RNA isolation from right hemisphere of animal model and quantitative real-time polymerase chain reaction (PCR)

Total RNA was extracted from the cells (PDC and BV2 cells) and right hemispheres by using a TRIzol reagent (Invitrogen, Carlsbad, CA, USA). 1 µg of purified total RNA was converted into cDNA by using SuperScript™ III (Invitrogen) and all cDNA samples were stored at -80 °C. Real-time PCR was performed in a LightCycler (Roche), and the relative expression of a housekeeping gene (GAPDH) and the gene expressions of TGF-β IL-10, TNF-α, IFN-γ, IL-1, IDE, and MMP9 were determined. Primers used for real-time PCR are listed in Table 1. The values for target gene expression were normalized against GAPDH. An expression value of control was assigned in the data set as standard and relative expression levels for all other samples were calculated.

**[Table 1]**

| Sequences of primers used in real-time PCR | | | |
|---|---|---|---|
| Gene name | Accession No. | 5'-Forward primer sequence-3' | 3'-Reverse primer sequence-5' |
| mIL-1 | NM_008361.3 | CCCAAGCAATACCCAAAGAA (SEQ ID NO: 1) | GCTTGTGCTCTGCTTGTGAG (SEQ ID NO: 2) |
| mTNF-α | NM_013693.2 | GCTCCAGTGAATTCGGAAAG (SEQ ID NO: 3) | GATTATGGCTCAGGGTCCAA (SEQ ID NO: 4) |
| mIL-10 | NM_010548.2 | CAGGGATCTTAGCTAACGGAAA (SEQ ID NO: 5) | GCTCAGTGAATAAATAGAATGGGAAC (SEQ ID NO: 6) |
| mTGF-β | NM_011577.1 | CAAGGGCTACCATGCCAAC (SEQ ID NO: 7) | AGGGCCAGGACCTTGCTG (SEQ ID NO: 8) |
| mMMP-9 | NM_013599.2 | TGCCGTCGAAGGGATACC (SEQ ID NO: 9) | GACCCGAAGCGGACATTGT (SEQ ID NO: 10) |
| mIDE | NM_031156.2 | GAAGACAAACGGGAATACCGTG (SEQ ID NO: 11) | CCGCTGAGGACTTGTCTGTG (SEQ ID NO: 12) |
| mGAPDH | NM_008084.2 | GTGAGGGAGATGCTCAGTGTT (SEQ ID NO: 13) | GGCTGGCATTGCTCT (SEQ ID NO: 14) |
| hTGF-β | NM_000660.4 | AAATTGAGGGCTTTCGCCTTAGCG (SEQ ID NO: 15) | TCTTCTCCGTGGAGCTGAAGCAAT (SEQ ID NO: 16) |
| hMMP-9 | NM_004994.2 | TTGACAGCGACAAGAAGTGG (SEQ ID NO: 17) | GCCATTCACGTCGTCCTTAT (SEQ ID NO: 18) |
| hIDE | NM_004969.3 | CTCGGAACCTTGCTTCAACAC (SEQ ID NO: 19) | GGCCCGCTGAAGACGATA (SEQ ID NO: 20) |
| hGAPDH | NM_002046.3 | AGGGCTGCTTTTAACTCTGGT (SEQ ID NO: 21) | CCCCACTTGATTTTGGAGGGA (SEQ ID NO: 22) |

### (6) Immunophenotyping of mouse model lymphocytes

Mouse lymphocytes were isolated from the spleen. Single-cell suspensions of the spleen were obtained, and subjected to Ficoll-Conray density gradient centrifugation to remove dead cells and red blood cells. Mouse lymphocytes were stained in the FACS buffer for 30 minutes at 4°C with FITC-labeled anti-CD4 and PE-labeled anti-CD25. Relative immunofluorescence of cells was analyzed using a FACSCalibur™ (flow cytometer) (BD).

### (7) Statistical analysis

Statistical analyses were conducted in a CHA University mainframe computer using a Statistical Analysis System (SAS; SAS Korea, Inc., Seoul, Korea), version Enterprise 4.0. Basic motor function analysis, data of image intensity, and real-time PCR data were analyzed using either a T-test or two-way variance analysis, followed by LSD (Fisher's least significant difference) post hoc tests. All other measures were analyzed using a mixed model analysis (SAS, PROC MIXED) to account for the random effect of mice. The correlation between the WMT and plaque counting was analyzed using the Pearson correlation coefficient. Data are presented as mean ± SEM and a p value of < 0.05 was considered significant.

### <Results>

### (1) Improvement of memory function by transplantation of PDC

In a water maze test (WMT), the mean escape latencies required to reach a hidden platform from each starting point (east E, north N, northwest NW, and southeast SE; FIG. 3A) were measured. There was no statistically significant interaction between the groups at any of the starting points (F_{6,66} = 0.50: F₆: degrees of freedom for the between-groups, starting point (n:7)-1=value(6), F₆₆: the total degrees of freedom, the total number of samples, p = 0.80). However, there was a significant interaction between the trial block and test groups (F_{8,88} = 2.73, F₈: degrees of freedom for the between-groups, starting point (n:7)-1=value(6), F88: the total degrees of freedom, the total number of samples. p = 0.009; FIG. 3B). In trial block 1, the normal control tended to find the hidden platform more quickly than the PBS- and PDC-injected groups (p = 0.06; FIG. 3B), whereas in trial block 2, mean escape latencies of 3 groups were not significantly different (FIG. 3B). The latency in the normal control group was significantly faster in trial block 3 than the PBS-injected and PDC-injected groups (p < 0.0001 and p < 0.0002, respectively; FIG. 3B).

Interestingly, from trial block 3 to trial block 5, the PDC-injected group showed a dramatic change in mean escape latency, indicating that impaired memory function was reversed by injection of placenta-derived cells. Moreover, though there was no statistically significant difference between the PDC-injected group and the normal control mice in trial blocks 4 and 5, there was a significant difference between the PDC-injected group and the PBS-injected group (p < 0.06 for trial block 4 and p < 0.002 for trial block 5; FIG. 3B). The results for the normal control mice were also significantly different from those of the PBS-injected group in trial blocks 4 and 5 (p < 0.002 and p <0.0003, respectively; FIG. 3B). Representative swimming path at trial block 5 from each group exhibited increased spatial learning acquisition in the PDC-injected group relative to the PBS-injected group (FIG. 3C).

Next, a probe test was performed on Day 5, 24 hours after the last training trial. The probe trial involved removing the platform and recording the length of time each mouse spent within the zone previously occupied by the platform over a period of 60 seconds. The results were 2.41 ± 0.35 seconds for the normal control group, 0.92 ± 0.31 seconds for the PBS-injected group, and 2.61±0.75 seconds for the PDC-injected group (F_{2,21} = 4.32, p = 0.03; FIG. 3D). The time spent in the zone was significantly less for the PBS-injected group than for the normal control (p < 0.008) and PDC-injected groups (p < 0.02). In terms of total swimming distance, there was a significant interaction between the trial block and test groups (F_{8,88} = 2.68, p < 0.01; FIG. 1A). There was no difference between the normal control group and the PDC-injected group in terms of swimming velocity (interaction between trial block and test group; F_{8,88} = 2.40, p < 0.02) throughout the first 4 trial blocks, but there was a significant difference between the normal control group and the PBS-injected group (p < 0.05; FIG. 4B).

There was no difference between wild-type mice (normal control group), APPswe mice injected with PBS (PBS-injected group), or APPswe mice injected with PDCs (PDC-injected group) in terms of locomotion tests, the elevated plus-maze test, light/dark transition tests, or open field tests (FIG. 5).

### (2) Amelioration of Aβ plaques in brains of APPswe mice by PDC treatment

Every sixth section from brain tissue excised from mice treated with PDCs for 12 weeks was stained with cresyl violet (FIG. 6A) and thioflavin S (FIGS. 6B to 6G). The number of Aβ plaques in 2 brain areas, the cortex and the hippocampus, was much lower in the PDS-injected group (8.72 ± 1.22) than in the PBS-injected group (19.84 ± 3.45) (FIG. 6H; p < 0.02). Plaques were analyzed according to subcategories based on the size of the individual Aβ plaques. The results showed that the number of small (<50 mm in diameter) and intermediate (50-100 mm in diameter) plaques were significantly lower in the PDC-injected group than in the PBS-injected group (FIG. 6H; p < 0.01 and p < 0.03, respectively), whereas the number of large plaques (>100 mm in diameter) was not different between the groups (FIG. 6H; p < 0.1). This was not the case 1 week after PDC injection (data not shown).

Further, the PDC-injected group tended to show a positive correlation between mean escape latency in the WMT and the number of amyloid plaques (rho = 0.85, p < 0.06), whereas no such correlation was found in the PBS-injected group (p = 0.83; FIG. 7).

### (3) Regulation of recruitment of microglial cells by PDC transplantation and increase of proportion of alternatively-activated, phagocytic microglial cells by PDC transplantation

To investigate the mechanism(s) by which the number of Aβ plaques decreased in the PDC-injected group, the number of resident microglial cells was counted. At 1 week post-injection, the number of lba-1-positive microglial cells significantly increased in the PDC-injected group (67.58 cells/mm²) compared with the PBS-injected group (58.36 cells/mm², p < 0.02; FIGS. 8A and B), whereas the number of lba-1-positive microglial cells was not different from each group at 12 weeks post-injection (p = 0.95; FIGS. 8A and B). Further quantitative image analysis revealed that the density of lba-1-positive microglial cells was higher in the PDC-injected group than in the PBS-injected group at Week 1 post-injection (p < 0.04; FIG. 8C), but lower at Week 12 post-injection (p < 0.05; FIG. 8C). These findings suggest that injected PDCs were able to recruit microglial cells during the initial acute stage after transplantation in the AD mouse model, and PDCs maintained a lower number of resident microglial cells, despite the proinflammatory environment. This supports an immunosuppressive role for MSC.

The density of activated microglia within the lba-1-positive population that was also positive for ED1 was analyzed. FIG. 9A shows that ED1 negative resting microglia and FIG. 9B shows ED1-positive microglia in the PDC-injected group were mainly scattered around the Aβ plaques, and not found in brain areas where there were no Aβ plaques (FIG. 10). Next, Aβ plaques and a surrounding area of microglial cells of a similar size were selected for more precise analysis. Most lba-1-positive microglial cells in the PDC-injected group were ED-1-positive, whereas the few lba-1-positive microglial cells in the PBS-injected group appeared to be co-stained with ED1 (FIG. 9B). Consistent with this result, a ratio of ED1 expression to expression was significantly higher in the PDC-injected group than in the PBS-injected group at 1 week and 12 weeks post-injection (p < 0.02 and p < 0.05, respectively; FIG. 9C). These findings also suggested the transplanted PDCs modulated the phagocytic activity of microglial cells.

Finally, HuNu immunostaining was performed to further define the location of the transplanted PDCs. No human cells were found in PDC-injected mouse brains at 1 week post-injection, but HuNu positive cells were identified in the spleen at least until 1 week post-injection (FIG. 11).

### (4) Modulation of IL-1, TNF-α, IL-10, and TGF-β expression and secretion of Aβ-degrading enzymes by PDCs

The levels of mRNA encoding the proinflammatory cytokines, IL-1 and TNF-α, were lower in the PDC-injected group than in the normal control group at 1 week post-injection (p < 0.01 and p < 0.06, respectively). However, the level of mRNA encoding the anti-inflammatory cytokine, IL-10 was higher in the PDC-injected group than in the normal control group (p < 0.04). The level of TGF-β mRNA was not significantly different between the two groups (p < 0.08; FIG. 12A).

By 12 weeks post-injection, mRNA levels for IL-10 and TGF-β were significantly higher in the PDC-injected group than in the normal control group (p < 0.0003 and p < 0.01, respectively), but there was no significant difference in the expression of IL-1 and TNF-α mRNA (FIG. 12B).

The levels of Aβ-degrading enzymes, including insulin-degrading enzyme (IDE) and MMP-9, were higher in the PDC-injected group than in the normal control group at 1 week post-injection (p < 0.005 and p < 0.06, respectively; FIG. 12C).

### (5) Immune regulatory function by PDCs

It was examined whether PDCs promotes induction of regulatory T cells which are CD4⁺CD25^{high} T cells having an ability to regulate tolerance in immune responses. At 1 day and 7 days post-injection of PDCs into AD mice, flow cytometry of CD4⁺CD25 ^{high} T cells from splenocytes was performed at each time point (FIG. 14A). A percentage of CD4⁺CD25 ^{high} T cells in lymphocytes isolated from PDC-injected AD mice was remarkably increased (3 times or higher), compared with PBS-injected AD mice (FIG. 14B).

### Example 3. Effect of PDC on Retinopathy

### 3.1. Response of PDC to Pathological Conditions In-vitro

### <Experimental method>

### (1) Culture and treatment of placenta-derived cells

Immediately after receiving the placenta, each tissue (decidua basalis) was carefully dissected and rinsed with PBS. The harvested pieces of tissue were cut into small pieces and digested with 0.5% collagenase IV (Sigma, St. Louis MO, USA) in a shaker incubator at 37°C for 30 minutes. The harvested cells were cultured in a T 25 flask (2 × 10⁵ cells/mm³) in alpha-MEM (Gibco, Grand Island, NY, USA) supplemented with 10% fetal bovine serum (Gibco, Grand Island. NY, USA), 100 µg/ml each of penicillin and streptomycin (Gibco), 25 ng/ml FGF4 (R&D Systems, Minneapolis, MN, USA), and 1 µg/ml heparin (Sigma). All cultures were maintained at 37°C in an incubator with 5% CO₂, and the culture medium was changed every day. In each experiment, cells were grown at a density of about 80%, and cells of no more than passage 5 (P5) were used for experiments. To track the injected cells, PDCs were incubated with 2 mM CM-Dil(1,1'-dioctadecyl 3,3,3',3'-tetramethy lindocarbocyanine pechlorate) which is a lipophilic membrane-bound fluorescent dye (Molecular Probes, Eugene, OR) for 30 min at 37°C (FIG. 15).

### (2) Immunophenotypic analysis of PDC

Flow cytometry analysis was performed on PDCs at P5. 2 × 10⁵ cells were incubated with either fluorescein isothiocyanate (FITC) or phycoerythrin (PE) conjugated with SSEA4, TRA-1-60, TRA-1-81, CD34, CD13, and CD90, CD9 (BD, Becton Dickinson, San Jose, CA) antibodies for 30 minutes. Ten thousand events were acquired per antibody in a FACS Calibur Flow Cytometer (BD, Hialeah, FL). The results were then analyzed using CellQuest Pro software.

### (3) In-vitro stimulation and analysis of PDCs

PDCs were seeded at 2 × 10⁵ cells/ml, cultured overnight at a volume of 1 ml, and then stimulated with the proinflammatory cytokines TNF-α (10 ng/ml; R&D Systems) and IFN-γ (100 ng/ml; Peprotech, Rocky Hill, NJ, USA) for 6 hours. Concentrations and times were chosen after titration (data not shown). Supernatants were collected and assayed by ELISA.

### (4) Treatment with siRNA targeting TGF-β1

Using the human nucleotide sequences for TGF-β1 mRNA obtained from GenBank (GI:260655621) and referring to the design strategy of siRNA, two pairs of 21-bp reverse repeat sequences targeting TGF-β1 mRNA were synthesized by Bioneer (Seoul, Korea). siRNA targeted forward: 5'-CAGAGUACACACAGCAUAU-3'(1243-1261) (SEQ ID NO: 31); reverse, 5'-AUAUGCUGUGUGUACUCUG-3'(1261-1243) (SEQ ID NO: 32) of the TGF-β coding sequence. siRNA (at a final concentration of 100 pmol) was transfected with Lipofectamine 2000 (Invitrogen, Gaithersburg, MD) according to the manufacturer's instructions. The efficacy of TGF-β1 gene silencing was assessed by quantitative real-time PCR and Western blotting.

### (5) Co-culture of human umbilical vein endothelial cells with PDCs

Human umbilical vein endothelial cells (HUVECs) were generously provided by Dr. Hyung-Min Chung at CHA University (Seoul, Korea). Cells were grown on plates in the EGM-MV Bullet Kit (5% FBS in EBM medium containing 12 µg/ml BBE, 1 µg/ml hydrocortisone, and 1 µl/ml GA-1000). HUVECs (passage 6) with 70% confluency were used for most experiments.

PDCs (2 X 10⁵ cells/well) were seeded in a Transwell (BD) 6-well plate with a membrane pore size of 0.4 µm and HUVECs (2 X 10⁵ cells/well) were spread on a 6-well dish, and allowed to attach overnight. For each treatment, PDCs were treated with/without proinflammatory cytokines or siRNA. siRNA treatment was conducted, before stimulation with the proinflammatory cytokines. The plates were left to incubate for 24 hours before the proliferation assay was carried out.

### (6) Inhibition of HUVEC proliferation

HUVECs were seeded in a 6-well plate (2 × 10⁵ cells/well) containing a growth medium. After a starvation phase of 16 hrs (1% FCS), cells were stimulated with VEGF165 (20 ng/ml; R&D) and incubated with a 5'-bromodeoxyuridine (BrdU) solution at a final concentration of 0.01 mM for 24 hours. Cell proliferation was assessed using a BrdU-based proliferation assay. The incorporated BrdU was stained with a specific anti-BrdU (Abcam, Cambridge, MA) fluorescent antibody. Absorbance was read with a microplate reader, VersaMax (Molecular Devices, Sunnyvale, CA, USA).

### <Results>

### (1) Characteristics of PDCs

Flow cytometric analysis of the immunophenotypic surface profiles of PDCs showed that they were negative for SSEA4, TRA-1-60, and TRA-1-81 (embryonic stem cell markers) and CD34 (a hematopoietic and endothelial cell marker), and positive for CD13 and CD90 (MSC markers) and CD9 (a nontrophoblast marker) (FIG. 16A).

The expression levels of anti-neovascular factors (including those that promote neovessel stabilization) in PDCs were compared with those of hBM-MSCs and hAdipo-MSCs. There was no difference in PEDF and ANG-1 expression (FIG. 15B), but TGF-β expression was much stronger in PDCs, compared with hBM-MSCs and hAdipo-MSCs (FIG. 16B).

### (2) Response of PDCs to pathological conditions in-vitro

To investigate whether PDCs maintain their antineovascular properties under pathological conditions in which proinflammatory cytokines are present, cytokine expression was analyzed in OIR-induced retinas. Multiple inflammatory cytokines were up-regulated, particularly, TNF-α and IFN-γ (FIG. 17). Based on this result, the effects of IFN-γ and TNF-α on PDCs were examined in-vitro. The mRNA and protein were extracted from PDCs at 0 hr and 24 hrs after treatment with IFN-γ and TNF-α. RT-PCR analysis showed that PDCs exposed to IFN-γ and TNF-α exhibited no significant differences in PEDF mRNA levels, and slightly decreased levels of ANG-1 (FIG. 18A). In contrast, expression of TGF-β was remarkably up-regulated in PDCs (p < 0.001; FIG. 18A). Consistent with this finding, increased secretion of TGF-β protein was confirmed by ELISA (271.82 ± 6.35 pg/ml vs. 70.45 ± 2.36 pg/ml, p < 0.001; FIG. 18B). These data suggest that PDCs respond to environmental changes by enhancing secretion of TGF-β.

### (3) Suppression Effect of PDCs on HUVEC Proliferation

To investigate whether TGF-β induction by PDCs plays an important role in their suppression of neovascularization, HUVECs were co-cultured with normal or IFN-γ-and TNF-α-treated PDCs. IFN-γ and TNF-α treatment of PDC (PDC stimulation) was performed 1 day before co-culture. Inhibitory effects on proliferation were measured by the BrdU-based proliferation assay, and the results demonstrated the efficiency of PDC-mediated suppression of HUVEC proliferation. As shown in FIG. 19, small but significant suppression of proliferation was observed in HUVECs co-cultured with normal PDCs, compared with HUVECs cultured alone. More importantly, IFN-γ- and TNF-α-treated PDCs suppressed the proliferation of HUVECs to a greater extent than normal PDCs (Fig. 19A). This suggests that the PDCs exposed to retinopathy-like conditions suppress the proliferation of HUVECs more efficiently than the PDCs under normal conditions.

Next, the mechanism of the suppressive effect of PDCs was examined. To evaluate the role of TGF-β, TGF-β expression was silenced by siRNA transfection. The transfection efficiency of TGF-β siRNA in the PDC culture was confirmed by quantitative real-time PCR, which showed that TGF-β siRNA decreased TGF-β expression by 80-90%, compared with control PDCs (FIG. 20) and there were no changes in PEDF and Ang-1 expressions (FIG. 21). As shown in FIG. 19B, TGF-β siRNA transfection interfered with suppressive effect of PDCs on HUVEC proliferation. Importantly, TGF-β siRNA-transfected normal PDCs generated no significant differences in their suppressive capacity (p = 0.77), as compared with non-transfected normal PDCs. In contrast, IFN-γ and TNF-α-treated PDCs that had reduced TGF-β expression lost the ability to suppress HUVEC proliferation (p = 0.007).

Taken together, these results indicate that PDCs play a suppressive role in HUVEC proliferation under retinopathic conditions, likely via the up-regulation of TGF-β expression.

### 3.2. Effect of PDCs Under Retinopathic Conditions In-Vivo

### <Experimental method>

### (1) Induction of oxygen-induced retinopathy (OIR)

OIR was induced in C57BL/6J mice. On P7, pups and their mothers were transferred from room air to an environment of 75% oxygen for 5 days and afterward returned to room air. Body weights were recorded on P12 and P17 (Table 2).

**[Table 2]**

| Day after birth | Body weights | |
|---|---|---|
| | PBS treated mice | PDCs treated mice |
| P12 | 4.23±0.15 | 4.54±0.19 |
| P17 | 4.85±0.17 | 4.56±0.08 |

### (2) Intraperitoneal transplantation of PDCs

Immediately after their return to room air on P12, pups were matched for their body weights and were randomly assigned into groups that received an intraperitoneal (IP) transplantation of either 1 × 10⁶ cells diluted in 50 µl of PBS (hMSC group, n = 8) or 50 µl of PBS (Control group, n = 8). Transplantation was performed in the right lower abdominal quadrant with careful handling to reduce loss of solution through backflow. The retinas of recipient mice were examined at P17.

### (3) Immunohistochemistry and visualization of vasculature

Retinas of P17 were harvested for imaging of the vasculature, localization, and characterization of the injected or endogenous cells. Quantification of vascular obliteration and preretinal neovascular tufts at P17 was performed. Immunohistological assays to stain blood vessels were conducted as previously described. Briefly, retinas were fixed in 4% paraformaldehyde (PFA) for 1 hour, followed by blocking in 10% normal goat serum (NGS) for 1 hour, followed by overnight incubation with Griffonia simplicifolia lectin (GS-lectin) (Vector Laboratories Inc., Burlingame, CA) conjugated to a fluorescent label.

After washes, retinas were counterstained with DAPI to visualize the cell nuclei. Retinas were laid flat with radial-relaxing incisions to obtain whole-mount preparations. In some cases, for tracking of the injected cells, only blocking was conducted, without the vessel staining, to prevent fluorescence interference. Images were acquired using a microscope fitted with a digital camera system (Nikon, Tokyo, Japan), connected to a Windows PC for quantitative analyses using Adobe Photoshop CS5 software.

### (4) RNA Isolation from retina of animal model and Reverse Transcriptase-PCR

RNA was isolated from the cells and retinal tissue using TRIzol reagent (Invitrogen) according to the manufacturer's instructions. The retinal RNA was extracted from the retina of one eye from each mouse in nine litters. 100 ng of purified total RNA from samples was converted into cDNA by using SuperScript™ III (Invitrogen) and all cDNA samples were stored at -80°C. RNA from human bone marrow MSCs (hBM-MSCs) and human adipose MSCs (hAdipo-MSCs) at passage 5 were kindly donated by Prof. Hyung-Min Chung (CHA University, Korea). Reverse transcriptase-PCR was carried out with 20 µl of a reaction volume of AccuPower PCR PreMix (Bioneer).

### 5) Quantitative real-time PCR

Real-time PCR was performed using a SYBR-Green reaction kit (Roche, Lewes, UK) according to the manufacturer's instructions in a LightCycler (Roche), and the relative expression levels of a housekeeping gene and the genes for VEGF, TGF-β, PEDF, and Ang-1 were determined. Primer sequences are shown in Table 3. Analysis of relative gene expression data was calculated by a comparative threshold cycle method (CT). The values for target gene expression levels were normalized against the expression level of a housekeeping gene. An expression ratio for a control in a data set was assigned as the standard level and relative expression levels for all other samples were calculated.

**[Table 3]**

| Primer sequences used in RT-PCR | | | |
|---|---|---|---|
| Gene name | Accession No. | 5'-Forward primer sequence-3' | 3'-Reverse primer sequence-5' |
| mTGF-β | NM_011577.1 | CAAGGGCTACCATGCCAAC (SEQ ID NO: 7) | AGGGCCAGGACCTTGCTG (SEQ ID NO: 8) |
| mPEDF | NM_011340.3 | GCCCAGATGAAAGGGAAGATT (SEQ ID NO: 23) | TGAGGGCACTGGGCATT (SEQ ID NO: 24) |
| mAng-1 | NM_0096403 | CCTCTGGTGAATATTGGCTTGGGA (SEQ ID NO: 25) | AGCATGTACTGCCTCTGACTGGTT (SEQ ID NO: 26) |
| mGAPD H | NM_008084.2 | GTGAGGGAGATGCTCAGTGTT (SEQ ID NO: 13) | GGCTGGCATTGCTCT (SEQ ID NO: 14) |
| hTGF-β | NM_000660.4 | AAATTGAGGGCTTTCGCCTTAGCG (SEQ ID NO: 15) | TCTTCTCCGTGGAGCTGAAGCAAT (SEQ ID NO: 16) |
| hPEDF | NM_002615.5 | TATCACCTTAACCAGCCTTTCATC (SEQ ID NO: 27) | GGGTCCAGAATCTTGCCAATG (SEQ ID NO: 28) |
| hAng-1 | NM_001146.3 | CCGTGAATCTGGAGCCGTTTG (SEQ ID NO: 29) | AAGCCCGACAGTCAGTGGAG (SEQ ID NO: 30) |
| hGAPDH | NM_002046.3 | AGGGCTGCTTTTAACTCTGGT (SEQ ID NO: 21) | CCCCACTTGATTTTGGAGGGA (SEQ ID NO: 22) |

### (6) ELISA of retinal proteins dissected from animal model

The dissected, snap-frozen retinas were homogenized on ice in RIPA buffer containing a protease inhibitor cocktail (Sigma-Aldrich). Since total retinal proteins from three mouse pup were collected for one sample, a total of three samples were obtained from each of nine litters in each group. The samples were centrifuged for 10 minutes at 8,000 × g at 4°C. The TGF-β1 present in the retina samples and culture supernatants were assayed using Human TGF-β1 Quantikine kits (R&D), according to the manufacturer's instructions. The TGF-β1 concentrations were calculated by comparison with standard samples. The TGF-β1 average for each group represents three experiments.

### (7) Statistics

Statistical analyses were conducted on a CHA University mainframe computer using the Statistical Analysis System software (SAS; SAS Korea, Inc., Seoul, Korea), version Enterprise 4.0. Data are presented as means ± SEM. All statistics were calculated using independent t-tests. Real time PCR data were analyzed with raw data. A value of p < 0.05 was considered significant.

### <Results>

### (1) Cytokine modulation by PDCs under retinopathic conditions in-vivo

To evaluate the possible effects of PDCs in vivo, cytokine expression was assessed in the retinas of mice exposed to OIR after PDC treatment. PDCs were injected into the peritoneal cavity of each mouse at P12, and retinal tissue was assessed at P17. The expression of antineovascular factors in the retina was then evaluated by real-time PCR. Consistent with the in vitro data, expression of TGF-β was significantly increased in the retinas of PDC-treated mice (FIG. 22A). Expression of PEDF in the PDC-treated group was not different from the control group, and no expression of Ang-1 was detected in the retinal tissue. Quantitative analysis of TGF-β expression in retinal tissue lysate by ELISA showed that TGF-β reached levels of 65.23 ± 11.16 pg/ml in the PDC-treated group, while the mean level of TGF-β in the control group was 27.73 ± 3.57 pg/ml (p < 0.01; FIG. 22B). Thus, analysis of TGF-β mRNA and protein levels indicate that TGF-β is secreted by PDCs in response to retinopathic conditions in vivo.

### (2) Effect of PDCs on retinal neovascularization

Next, the potential of PDCs to reduce pathological angiogenesis was examined in-vivo. As shown in FIG. 23A, administration of PDCs dramatically altered the degree of pathological neovascularization induced by OIR compared with a control group. Retinal neovascular tufts, which are abundant in the central and midperipheral retina of mice in the control group, were present only at low levels in the PDC-treated group (FIG. 23A, left and middle panels). Areas of neovascular tufts are shown in gray (FIG. 23A, inset). Quantitative analysis confirmed a reduction in neovascular tuft area of over 4.73% in the retinas of the PDC-treated mice, compared with the control mice.

Consistently, the retinal vasculature in the PDC-treated group exhibited normal morphology and branching in the midperipheral and peripheral regions, with the exception of sparse avascular areas around the central retina (FIG. 23C). Areas of vascular obliteration are shown in gray (FIG. 23C, inset). Retinas of mice treated with PDCs exhibited an approximately 10.93% reduction in avascular area, compared with the retinas of mice treated with PBS (p < 0.001; FIG. 23D). These results indicate that PDCs suppress neovascular tuft formation and increase normal vascularized areas in proliferative retinopathy.

The capacity of TGF-β siRNA to nullify the beneficial effect of PDCs in vivo was also examined. For this experiment, functional analyses were conducted using PDCs treated with TGF-β siRNA (FIGS. 23A, C, right panel). As expected, the TGF-β siRNA-treated PDCs failed to reduce neovascular tuft formation, compared with non-silenced PDCs (p < 0.001; FIG. 23B); however, normal vascularized areas were marginally increased in the TGF-β siRNA-treated group, compared with the control group (p < 0.06; FIG. 23D), although the difference in the quantity of normal vascularized areas between untreated PDCs and TGF-β siRNA-treated PDCs was not significant (p > 0.27; FIG. 23D). These results imply that PDC treatment effectively suppresses retinal neovascularization through the expression of TGF-β, while its effect on vascular obliteration is irrespective of TGF-β.

### (3) Possible PDC-mediated suppression of retinal neovascularization

To investigate the possible mechanism of PDC-mediated suppression of retinal neovascularization, intraperitoneally administered PDCs were labeled with CM-Dil and tracked (FIG. 15A). It is important to note that PDCs were not administered intravenously, to rule out the possibility of circulating PDCs from retinal vascular leakage. The migrated PDCs were detected in the retina at 5 days post-injection (FIG. 24, left and middle panels). The PDCs were located in retinal neovascularized areas, but did not integrate into the vascular network (FIG. 24, right panel), suggesting that PDCs did not differentiate into endothelial cells or pericytes. These results indicated that PDC-mediated regulation of abnormal angiogenesis was conducted through a paracrine mechanism such as the release of cytokine.

### Example 4. Induction of Neuronal Progenitor Cells from Placenta-Derived Cells and Effect Thereof on Improvement of Functional Impairment of Parkinson's Disease-Induced Model

### <Experimental method>

### (1) Isolation of hPDCs from human term placenta and treatment

Normal human term placenta (= 37 weeks of gestation) without symptoms of internal, obstetric, or surgical complications was obtained after cesarean section. The placenta was carefully dissected. The collected tissue fragments were washed several times with PBS, mechanically ground, and digested with Hank's Balanced Salt solution(HBSS, Gibco) containing 2 mg/ml trypsin (Sigma), 20 ug/ml DNase I (Sigma), 1.2 U/ml Dispase (Gibco), and 1 mg/ml collagenase IV (Sigma) for 30 min at 37°C. The recovered cells were cultured in alpha-MEM medium (Gibco, New York, USA) supplemented with 10% fetal bovine serum (FBS, Gibco), penicillin and streptomycin (Gibco), 25 ng/ml FGF4 (R&D system), and 1 µg/ml heparin (Sigma) in a T25 flask (2 X 10⁵ cells/mm³, Nunc). At 3 days, human amniotic stem cells (hPDCs) were trypsinized, and counted by using a hemocytometer.

### (2) Culture of human ESC, human BM-MSC, human AD-MSC, human fetal NPC

Human bone marrow mesenchymal stem cells (hBM-MSCs, Lonza) were cultured in Alpha-MEM medium supplemented with 10% FBS and penicillin/streptomycin and 100 mM L-glutamine at 37°C under 5% CO₂. Human adipose mesenchymal stem cells (hAD-MSCs) were cultured in Alpha-MEM medium supplemented with 10% FBS and penicillin/streptomycin at 37°C under 5% CO₂. When cells reached 80% confluence, cells were trypsinized, and transferred to a new culture dish.

Human neural precursor cells were spread at a density of 30,000 cells/cm² on a culture dish which was pre-coated with 15 µg/ml polyornithine and 4 µg/m fibronectin. Cells were cultured in DMEM/F12 (Gibco) supplemented with 1 ug/ml tocopherol and 1 ug tocopheryl acetate and 20 ng/ml of human epithelial growth factor (EGF), 20 ng/ml human fibroblast growth factor (FGF-2; basic fibroblast growth factor) and 2% B-27 Supplement Minus AO. A culture was placed at 37°C, 5% CO₂ under humid environment of 92% N₂ and 3% O₂.

### (3) Flow cytometry analysis, quantitative PCR, and immunoblotting analysis

Flow cytometry analysis: For phenotyping, cells were stained with the following antibodies of appropriate concentrations at 4°C for 20 minutes in a FACS buffer: FITC-labeled anti-SSEA4, TRA-1-81, CD90, CD34 and PE-labeled anti-TRA-1-60, CD9, CD44, CD13 (BD Pharmingen). After washing the cells, analysis was performed by using Cell Quest software and a FACSCaliber (Becton Dickinson).

Quantitative RT-PCR: Total RNA was isolated from cells using TRIzol (Invitrogen). cDNA was synthesized from 1 µg of total RNA using Superscript II reverse transcriptase, Oligo-d(T) 12-18 primer, DTT, and dNTPs according to the manufacturer's protocol. RT-PCR was performed by using a Pre-Mix kit (Bioneer). Primers used are listed in Table 1. A PCR product was electrophoresed on a 2% agarose gel using RedGel (Biotium, Inc), and visualized by a UV-transilluminator.

Immunoblotting analysis: Cells were washed with cold PBS, and lysed with a lysis buffer containing a protease inhibitor cocktail (Pierce). A total cell lysate was normalized with a Bradford reagent (Bio-Rad), and 30-50 µg of the cell lysate was applied to 8 ∼ 12% SDS-PAGE and transferred onto a PVDF membrane (Milipore). The membrane was blocked with TBS-T (50 mM Tris-HCl, pH 7.6, 150 mM NaCl, 0.05% Tween-20) containing 5% skim milk (Becton Dickinson), and examined with respective antibodies. An immune reaction was performed by using an ECL Western Blotting system (Millipore, Amersham).

### (4) Culture system of hPDCs for NPC

hPDCs were cultured in alpha-MEM supplemented with 10% FBS (Gibco), each 100 µg/mL of penicillin and streptomycin (Gibco), 25 ng/mL FGF4(R&D Systems), and 1 µg/mL heparin (Sigma). For nerve induction, a high cell density culture method was used to increase interactions between cells. Cells were spread at a density of 10,000 cells/cm² in the center of a dish coated with 15 ug/mL polyornithine (Sigma) and 4 µg/mL fibronectin (Sigma) by spotting cells at about 1/3 of the total plate space, followed by incubation of the cells in a complete medium containing 25 ng/mL FGF4 and 1 µg/mL heparin under environment of 37°C, 5 % CO₂ for 6 days.

### (5) Analysis of differentiation potential of human PDCs

Adipogenic differentiation: Cells were seeded on a 6-well culture dish at a density of 1.5 X 10⁴ cells/ cm², and cultured in high glucose DMEM (Gibco) supplemented with 10% FBS (Gibco) until cells reached 100% confluence. Subsequently, cells were subjected to three cycles of induction/maintenance by sequentially using an adipogenic induction medium (high glucose DMEM supplemented with 1 mM dexamethasone (Sigma), 0.5 mM 3-isobutyl-1-methyl-xanthine (Sigma), 10 ng/mL recombinant human insulin (Sigma), 100mM indomethacin (Sigma), and 10% FBS) for 7 days and an adipogenic maintenance medium (high glucose DMEM supplemented with 10 ng/mL recombinant human insulin and 10% FCS) for 7 days. After differentiation, cells were fixed in 10% formalin, and stained with 2%(wt/vol) Oil Red O reagent (Sigma)at room temperature for 5 minutes to examine generation of oil droplets in the cytoplasm.

Osteogenic differentiation: Cells were seeded at a density of 3 × 10³ cells/cm², cultured in high glucose DMEM (Gibco) supplemented with 10% FBS (Gibco) until they reached 70-80% confluence, and then fed twice a week for 2.5 weeks with osteogenic induction medium (IMDM basal medium (GIBCO) supplemented with 100 nM dexamethasone (Sigma), 10 mM β-glycerophosphate (Sigma), 0.2 mM ascorbate (Sigma), and 10% FBS). Osteogenic differentiation was detected by fixing the cells with 10% formalin for 15 minutes at room temperature and then staining them with von Kossa to show deposition of hydroxyapatite matrix.

Chondrogenic differentiation: To induce chondrogenic differentiation, cells were first seeded (3 x 10³ cells/cm²), and transferred into a 15-ml polypropylene tube and centrifuged at 1,000 rpm for 5 min to form a pelleted micromass at the bottom of the tube, and then treated with a chondrogenic medium (high-glucose DMEM supplemented with 0.1 M dexamethasone, 50 g/ml AsA, 100 g/ml sodium pyruvate, 40 g/ml proline, 10 ng/ml TGF-1 (Peprotech), 6.25 g/mL insulin, 6.25 g/mL transferrin, 6.25 ng/ml selenius acid, 1.25 mg/ml BSA, and 5.35 mg/ml linoleic acid (all excluding TGF-1 were purchased from Sigma). Medium changes were carried out twice weekly and chondrogenesis was assessed at weekly intervals. After 2-4 weeks of culture, the cells were washed twice with PBS, fixed in 4% PFA, and stained with Alcian Blue.

### (6) Suppression of FGF4 downstream signaling

To suppress FGF4 downstream signaling, hPDCs were cultured for 1 hour in a 10% FBS-supplemented alpha-MEM medium pretreated with or without 5 µM of ERK-inhibiting U0126 or 10 µM of JNK-inhibiting SP600125(Calbiochem) in dimethyl sulfoxide (DMSO), and then stimulated with 25 ng/mL FGF4 for 3 days. To analyze suppression of Notch signaling, ASCs were in cultured for 1 hour in a 10% FBS-supplemented alpha-MEM medium pretreated with or without 50 µM of Notch-inhibiting DAPT (N-[N-(3,5-difluorophenacetyl)-1-alanyl]-S-phenylglycine t-butyl ester, Sigma), and then stimulated with 25 ng/mL FGF4 for 3 days.

### (7) Immunocytochemistry

Cells were first fixed in 4% PFA at room temperature for 15 minutes, and then permeabilized with 0.1 % Triton X-100 for 20 minutes, followed by blocking with 10% normal goat serum (NGS). The following primary antibodies were used: anti-neuron classl β-tubulin (Tuj1), -Nestin(Covance), -Ki-67(Novocastra), -SOX2, -NANOG, KLF4, Brachyury/Bry (all from Abcam), OCT3/4, GATA4 (all from Santa Cruz). Cells were incubated with Alexa Fluor 488 or 594-conjugated antibody (Molecular Probes) at room temperature for 1 hour, and to examine nuclei, cells were stained with 4', 6-diamino-2-phenylindole (DAPI, 2 mg/mL in PBS). A cover-slip was sealed to a glass slide. Cells were visualized by a fluorescence microscope or a confocal fluorescence microscope (LSM 510 confocal microscope, Zeiss).

### (8) Animal test

### Preparation and treatment of experimental animal model

Female adult Sprague-Dawley rats (220-250 g) were maintained at room temperature on a standard 12 hr light/dark cycle (light on at AM 7), and allowed to freely access feed and water. All experimental procedures were performed according to the Guidelines for the Care and Use of Laboratory Animals of the Animal Welfare Committee.

### 6-Hydroxydopamine lesion and transplantation

Rats were anesthetized with ketamine (4.5 mg/kg, i.p.) containing rumpun (1.5 mg/kg). 9 µg of 6-OHDA and 0.2 mg/mL of L-ascorbic acid were injected into right medial forebrain bundle (MFB) of rats by bilateral stereotaxic injection using a 26 gauge Hamilton syringe: tooth bar set at anterior-posterior (AP) -4.4, medial-lateral (ML) -1.2, dorsal-ventral(DV) -7.8, -2.3, and tooth bar set at AP -4.0, ML -0.8, DV -8.0, 3.4; all represent millimeter adjustments from bregma (Paxinos et al., 1997, Espino et al., 1995, Kim et al., 1998). The inserted needle was withdrawn from each location after 5 minutes. 4 weeks after 6-OHDA injection, an amphetamine-induced rotational asymmetry test of rats was performed. Rotation scores were monitored using an automated Rotameter device (Med Associates Inc., St. Albans, VT, USA) for 90 minutes.

For cell transplantation, rats with 6 turns/min ipsilateral to the lesion were assigned to one of the following four groups: sham control (n=11), hPDCs (n=9), hPDCs-NPC (n=8), and hES-NPC (n=4). On the day of injection, cells were trypsinized, and washed with PBS, and then counted. 3 µl of a cell supernatant (1.5 x 10⁵ cells/µl in PBS) was injected into the ipsilateral striatum using a 22-gauge needle and a KDS310 nano pump (KD Scientific Inc., Holliston, MA, USA) for 5 minutes or longer. Under anesthesia induced with ketamine (4.5 mg/kg) and rumpun (1.5 mg/kg), cells or salts were injected into two sites (AP, ML and DV of (1) 0.07, -0.30, -0.55;(2) -0.10, -0.40, -0.50 relative to bregma and dura). 10 minutes later, the needle was removed from each site. All animals including the sham control were intraperitoneally (i.p.) administered with cyclosporin A (10 mg/kg, Chongkundang Pharmaceutical Corp.) diluted with an immune-suppressor salt from the next day post-transplantation to 2 weeks post-transplantation. An administration dose was reduced by 5 mg/kg until the end of the experiment.

### Treatment, immunohistochemistry, and immunofluorescence of brain tissue of animal model

At 1, 6 or 12 weeks after cell transplantation or salt injection, animals were euthanized by anesthesia with pentobarbital (3 mL/kg), and then transcardially perfused with 4% PFA in 0.05 M phosphate buffer. The brain was removed, post-fixed, and cryoprotected. Immunohistochemistry was performed in a free-floating cryomicrotome-cut section (40 µm in thickness) including the whole brain as described in known methods. After the sections were incubated in 0.05 M PBS at 3% H₂O₂, and incubated in 0.3% Triton X-100 and 3% BSA in 0.1 M PBS, the sections were stained with a primary antibody to recognize dopaminergic neurons (1:200 TH from Abcam) at 4°C overnight. A Vectastain Elite ABC kit (Vector Laboratories., Burlingame, CA, USA) was used as a secondary antibody. Tissues were visualized by a fluorescence microscope or a confocal fluorescence microscope (LSM 510 confocal microscope, Zeiss).

Alternatively, the obtained free-floating 40-µm section was stained with the following primary antibodies at 4°C overnight: SOX2(1:200; Chemicon), Nestin(1:200; Chemicon) and DCX (1:200; Cell-Signaling Technology). The tissue was washed with PBS three times and incubated with secondary fluorescence-conjugated goat anti-mouse or anti-rabbit antibody (DAKO, Denmark, Cy3, FITC, both diluted 1:1,000) for 2 hours, and finally washed and mounted in Vectashield. All tissue samples were further counterstained with DAPI. Tissues were visualized by a fluorescence microscope or a confocal fluorescence microscope (LSM 510 confocal microscope, Zeiss).

### Behavioral Tests

In 6-OHDA Parkinson models, four groups of control (n=11), hES-NPC(n=4), hPDC(n=9), and hPDCs-NPC(n=8) were subjected to the following behavioral tests.

Rotational test: After intraperitoneal injection of 5 mg/kg amphetamine (Sigma), rotational behavior was measured using an automated rotameter system (Med Associates Inc.) for 90 minutes. Ipsilateral rotations were calculated at 7 days before cell transplantation and at 3, 7 and 9 weeks after transplantation.

Cylinder test: Spontaneous movement was measured by placing animals in a transparent cylinder (height, 40 cm; diameter, 20 cm). Spontaneous activity was videotaped for 5 minutes. Movements of a total of 6 patterns (left forelimb touch, right forelimb touch, first left touch of both forelimbs, first right touch of both forelimbs, touch of both forelimbs, and raising body only) were evaluated as spontaneous movements of rats. The number of forelimb steps was measured.

Rotarod test: An accelerating rotarod test was performed with an ACCELER rotarod treadmill for rats. After adaptation of fixed speed (4 rpm) for 3 minutes, rats were placed on a horizontal plastic rod rotating at an initial speed of 4 rpm, and the rotational velocity of the rod was linearly increased from 4 rpm to 50 rpm within 5 minutes. The time when each rat was able to maintain its balance walking on the top of the rod was measured. The rod rotating time was calculated at 21 days, 22 days, 23 days, and 42 days after cell transplantation.

### (9) Western blotting of striatum of animal model

To obtain protein extracts, rats were sacrificed by cervical dislocation at 6 weeks after transplantation or salt injection, and striatum (ST) and subtantia nigra (SN) were rapidly dissected. Samples were sonicated in a 400 µl ice-cold lysis buffer (50 mM Tris-HCl [pH 7.5], 150 mM NaCl, 1% Triton X-100, 0.5% sodium deoxycholate, 0.1% SDS and 0.02% sodium azide). Protein concentrations of lysates were determined by using a Bradford reagent.

For Western blot analysis, samples (10-20 µg protein) were loaded on 12% sodium dodecyl sulfate- polyacrylamide electrophoresis gel. After separation, proteins were blotted on a nitrocellulose membrane and stirred in TBS (Tris-buffered saline) containing 0.1% Tween-20, 5% skim milk, and 0.2% BSA at room temperature for 1 hour. After stirring, the membrane was incubated with a primary antibody (rabbit monoclonal anti-TH; Biotechnologies Santa Cruz, CA. USA; 1:1000) in TBS containing 0.1% Tween-20 at room temperature for 1 hour. A horseradish peroxidase (HRP)-conjugated secondary antibody (anti-rabbit; 1:1000) was used to detect the primary antibody, and the bound secondary antibody was visualized by enhanced chemiluminescence (Amersham Biosciences, Buckinghamshire, UK).

### (10) Safety test by teratoma formation assay and karyotype analysis

Teratoma formation assay: Both left and right testis of 6-week-old 6 BALB/c-nu Sic male mice (Central Lab. Animal Inc, Korea) were transplanted with 5 X 10⁵ cells in 10 µl of a culture medium by using a 21-gauge needle. At 8 weeks, 12 weeks or 48 weeks after transplantation, mice were sacrificed and testis was fixed in 10% formalin. All tissues were embedded in paraffin blocks for analysis, and the blocks were sectioned 10-15 µm, and resulting slides were stained with hematoxylin and eiosin.

Karyotype analysis: Karyotyping was performed by the Cytogenomic Services Facility of Samkwang Medical Laboratories. For karyotyping, cell division was arrested in metaphase by using 0.05 µg/mL colcemid for 1 hour-2 hours. Chromosomes were visualized by G-band staining. 100 or more of cells in metaphase were analyzed and at least 3 were subjected to karyotyping.

### (11) Statistical Analysis

Statistical analyses were conducted on a CHA University mainframe computer using the Statistical Analysis System (SAS; SAS Korea, Inc. Seoul, Korea), version Enterprise 4.0. The number of TH⁺ cells was analyzed using T-test or two-way ANOVA, followed by LSD post hoc tests. For behavioral assay, mean values of the rotational test, and rotarod and cylinder tests were used as dependent variables, and "treatment" (sham control, hPDCs, hPDCs-NPC, and hES-NPC-transplanted groups) were used as independent variables. A mixed model analysis of variance procedure (SAS, PROC MIXED) was used to account for the random effect of rats.

### <Results>

### (1) Characterization of hPDCs of human term placenta

hPDCs isolated from a placenta amniotic membrane were characterized by several in-vitro assays. The karyotype analysis of the isolated cells confirmed human fetal origin (FIG. 25A) and retention of highly flat asymmetrical spindle-shaped morphology strongly suggesting mesenchymal cells (FIG. 25B). A growth curve of hPDCs demonstrated that an amount of cells accumulated in vitro increased rapidly and steadily with increasing cell passage, indicating active proliferation of cells. A doubling time of hPDCs is 31 hours and a population doubling level (PDL) lasts up to 32 days (FIGS. 25C, D). Flow cytometry revealed that hPDCs was positive for MSC-associated surface markers CD44 (94.3 ± 3.7%), CD13 (84.1 ± 7.1%) and CD90 (84.0 ± 7.5%) and negative for CD34 which is a hematopoietic stem cell marker.

Some stem cell surface markers appear in freshly isolated hPDCs (FIG. 25E). The cells expressed other surface markers of embryonic stem cells (ESC), including containing SSEA-4 (97.0 ± 1.4%) and TRA-1-60 (56.9 ± 19.3%), and TRA-1-81, and did not express SSEA-1. Detection of CD9 (99.8 ± 0.1%) confirmed that the cells were not derived from trophoblasts (FIG. 25E). Both human ESC and ASC express mRNAs of characteristic genes of pluripotent stem cells, including OCT-3/4, NANOG, KLF4, and SOX2 (FIG. 25G). However, immunohistochemical profile of hPDCs indicated that the cells were positive only for SOX2, and no OCT4, NANOG, and KLF4 proteins were observed (FIG. 25F).

Further, in immunoblotting, protein levels of OCT4, NANOG, and KLF4 were not detected in freshly isolated hPDCs (FIG. 25H), while expression of mRNAs for four genes of KLF4, OCT-3/4, NANOG and SOX2 was detected in hPDCs (FIGS. 25G, H). H9 cells showed alkaline phosphatase activity, but hPDCs did not (FIG. 25I). In addition, hPDCs showed an ability to differentiate into mesenchymal-lineage cells such as adipocytes, osteoblasts, and chondrocytes (FIG. 26A).

### (2) Nestin-positive cells of hPDCs increased by FGF4 treatment

Some growth factors including bFGF, EGF, and FGF4 were tested to examine the neural induction potential. The expression levels of Nestin and Musashi1 which are NPC markers were much higher when treated with 25 ng/mL FGF4 than the other two conditions (treated with 10 ng/mL EGF or 10 ng/ mL bFGF) (FIG. 26B). Subsequently, it was examined whether FGF4 affects the fate of hPDC proliferation. Thus, it was investigated whether treatment with different doses of FGF4 (10 ng/mL, 25 ng/mL, and 50 ng/mL) for 3 days influences expression of Nestin which is a typical marker for NPC of hPDC.

The highest mRNA expression level of Nestin was obtained at 25 ng/mL of FGF4 (FIG. 27A). Expression of Nestin protein as measured by immunoblotting was similarly increased (FIG. 27B). On the third day of differentiation, the number of Nestin-expressing cells was significantly different depending on whether FGF4 (25 ng/mL) was present in the medium (total cells counted in 7 sets of independent cultures, p <0.0001) (FIGS. 27C, D). Expression of SSEA-4, TRA-1-60, and TRA-1-81 was also lower in FGF4-treated cultures than in control cultures in which FGF4 was not treated (FIG. 28), whereas CD9, CD90, CD13, and CD44 were maintained in FGF4-treated cultures.

### (3) FGF4 downstream signaling of hPDC

Intracellular signaling activities such as mitogen-activated protein kinase (MAPK; including ERK and JNK), which is a downstream target of FGF4 and activates neurogenesis of hPDCs, were investigated. In FGF4-treated or untreated cells, protein levels of signal molecules activated (phosphorylated) for 72 hours were measured. Immediate increases in pERK (phosphorylated ERK) levels were observed at 12 hours and pERK levels gradually increased for 24 hours after FGF4 treatment.

Moreover, a level of pJNK, which is another key downstream signaling material of FGF4, increased sharply for 12 hours of FGF4 treatment. Interestingly, in FGF4-treated cells, pERK1/2 and pJNK were the highest levels at 12 hours, and a significant increase in Nestin expression was detected at 12 hours (FIG. 27E). In order to analyze whether the induction of Nestin expression by FGF4 is due to FGF4/ERK signaling or FGF4/JNK signaling, hPDCs were treated with 5 mM of an ERK inhibitor U0216 or 20 mM of a JNK inhibitor SP600125 at 1 hour before FGF4 administration, ERK phosphorylation and JNK phosphorylation were analyzed at 72 hours. Inhibition of FGF4-MAPK signaling by the specific inhibitors U0126 (ERK signaling) and SP600125 (JNK signaling) led to a decrease in Nestin protein levels (FIGS. 27F, G). There was a significant difference between groups (F(_{3,23})=11.56, P < 0.0001 in ERK inhibition experiment, F(_{3,23}) = 137.22, P < 0.0001 in ERK and JNK inhibition experiment). Total cells of 47.61 ± 3.46 were Nestin⁺ in the presence of 5 mM U0216 and total cells of 48.47 ± 3.07 were Nestin⁺ in the presence of 20 mM SP600125 (P <0.004 and P <0.006, respectively, in DMSO), whereas 68.19 ± 4.2 in DMSO.

In contrast, a proportion of Ki-67⁺ proliferating cells was low in the presence of 5 mM U0216 (% Ki-67⁺ cells in total DAPI⁺ cells: 64.46 ± 2.14 in 5 mM U0216, and 67.3 ± 2.39 in DMSO; P <0.0001, n = 6), whereas the proportion more significantly decreased in the presence of 20 mM SP600125 (% Ki-67⁺ cells in total DAPI⁺ cells: 37.53 ± 1.89 in 20 mM SP600125 (compared with DMSO-treated group, P <0.0001, n = 6) (FIGS. 27G, H). These data suggest that FGF4-induced ERK signaling is specific to signal induction, but does not influence Ki67⁺ proliferative cells whereas FGF4-JNK signaling seems to influence not only Nestin expression but also Ki-67 expression.

### (4) Cell-cell contact induced by high level of NPC due to increased expression of DLL1

To investigate how to increase the yield and purity of neurons, effects of some cell-to-cell contact levels on nerve/neuron induction were examined under different cell density conditions which enhance cell-cell contact. Thus, hPDCs cultured in a medium containing FGF4 were spotted on plates to form morphologically spherical aggregates, named "High-Density (HD) culture systems". In general, morphological changes of cells are accompanied by the formation of specific structures in the development of cell migration, differentiation and multicellular tissue.

FGF4-treated hPDCs in the HD culture system (10 × cell density) or in normal culture conditions (10 × cell density) were visualized using a phase contrast microscope, which can visualize morphological changes in hPDC cell differentiation, by time-lapse digital imaging for 24 hours (FIGS. 29 and 30A). The expression levels of Nestin/Ki-67 and Nestin/TUJ1 were compared between normal and HD cultures for 3 days in a medium containing 25 ng of FGF4. In general, Nestin-immunoreactive cells gradually increase during differentiation (FIGS. 30B, C). As shown in FIG. 30B, at 12 hours, Nestin⁺ cells were observed in hPDCs cultured in HD of 10x density; this was the highest ratio among groups of different conditions.

On the first day, hPDCs of 1 × density cultured in HD showed Nestin⁺ cells at a slightly high ratio than cells in normal culture, and no HD showed at 5 × density. On the other hand, at 10 x cell density, a sharp increase in the number of Nestin⁺ and TUJ1⁺ cells was observed. After 3 days of neural induction, hPDCs cultured by the HD method of 10 x density showed the highest level of Nestin⁺ cells. Moreover, hPDCs cultured by the HD method of 10 x density showed the highest expression of TUJ-1 after 3 days of neuronal induction.

Regarding proliferation, cells cultured at 10 × density demonstrated increased expression of Ki-67, compared with cells cultured under HD or normal culture of 1 × density. A sudden increase in all groups was observed on the third day, compared with the first day. However, the cell growth at 10 X density of the HD method was reduced (FIG. 30B). As shown in FIG. 30C, protein immunoblotting demonstrates a steady increase in the relative expression of Nestin and TUJ-1 as cell density increases, regardless of the culture method used. At the same cell density (1x density), Nestin mRNA, Musashi mRNA, and TUJ1 mRNA levels generally increased under normal and HD differentiation conditions. HD conditions induce a slight increase in Nestin mRNA level on day 3 and a strong increase in Nestin on day 6, compared with expression levels under normal culture conditions while Musashi mRNA and TUJ1 mRNA levels did not show a remarkable increase under HD conditions, compared with those under normal growth conditions (FIG. 30D). Furthermore, Nestin, Muhashi, and TUJ1 protein levels increased significantly under all culture conditions on day 3 in proportion to cell density (FIG. 30E, F(_{1,12}) = 68.84, P <0.0001, n = 3).

Surprisingly, a Nestin protein level was higher at 3 different cell concentrations under HD conditions, as compared with normal conditions (P = 0.0001 at 1x, p = 0.002 at 5x, and P = 0.0004 at 10x, as compared with those under normal conditions), and the Nestin protein reached a maximum level at 10x cell density under HD culture conditions (FIGS. 30F, G). At three different cell concentrations, the same pattern of TUJ1 protein levels was observed (P = 0.0007 at 1x, p = 0.004 at 5x, and P = 0.01 at 10x, compared with those under normal conditions), and TUJ1 protein reached a maximum level at 10x cell density under HD culture conditions (FIGS. 30F, I). A Musashi1 protein level increased in proportion to the cell density (F_{(1,12)}=16.15, P < 0.001, n=3), that is, the Musashi1 protein level increased from 1x to 10x under normal culture conditions (P <0.0001 from 1x to 5x and P<0.0001 from 5x to 10x under normal conditions), and the Musashi1 protein level increased at 5x to 10x under HD culture conditions (P<0.0001 from 5x to 10x under HD culture conditions) (FIGS. 30F, H). In contrast, a PCNA protein level was slightly reduced under HD culture conditions (FIGS. 30F, J).

### (5) Neuronal induction mechanisms of cell-cell interactions

To further investigate the neural induction mechanism of cell-cell interactions, mRNA expression of some Notch receptors and ligands was measured in hPDC and hPDC-NPC by RT-PCR. Initially, mRNA levels were compared between hPDC cultured under normal culture conditions and hPDC-NPC induced under HD culture conditions. In all culture conditions, the same medium containing FGF4 was used for 3 days. RT-PCR analysis revealed that hPDC expressed all human Notch receptors of Notch1, Notch2, Notch3 and Notch4, and Notch ligand expressed DLL1, DLL3, and JAG2, and did not express DLL4 and JAG1 (FIG. 31A). In particular, the expression level of the DLL-1 ligand was much stronger in HD-cultured hPDC-NPC, compared with normally cultured cells, whereas the expression of JAG2 ligand and Notch receptor showed no change between the two cultures, indicating that DLL1 is an important factor in optimal nerve/neuronal induction.

To examine how DLL1 neurotransmission modulates transcription factors, a gene expression pattern of bHLH protein was evaluated, which regulates development of the neural lineage and produces progenitors including HES1, HES5, MASH1, NEUROD, and NGN2. Semi-quantitative RT-PCR showed that the expression levels of MASH1 and NEUROD increased in the HD culture, compared with those under the normal culture. Expression of HES1 was detected in both normal and HD cultures. In the HD culture method, the expression of HES1 decreases with increasing cell density, while the level of DLL1 increases with increasing cell density. Considering that HES1 is a negative bHLH transcription factor inhibiting neuronal development and DLL-1 is essential for neuronal or neuronal development through cell-cell interactions, an improvement in cell-cell interaction is highly likely to increase nerve/neuronal induction (FIG. 31 B).

To further confirm this finding, whether or not binding of DLL1 blocks Notch under nerve/neuron induction conditions (HD containing FGF4) during hPDC culture was examined by using the effect of a Notch inhibitor DAPT(N-[N-(3,5-difluorophenacetyl)-1-alanyl]-S-phenylglycine t-butyl ester) (FIG. 31 C). In order to analyze the effect of DAPT inhibitors on nerve maintenance, gene expression of DLL, Nestin, and TUJ was measured in DAPT-treated cells and DMSO-treated cells.

DLL1, Nestin, and TUJ1 were expressed at significantly low levels in the DAPT-treated cells, compared with the DMSO-treated cells (P=0.009, P=0.003, and P=0.02, respectively, n=3, respectively). The up-regulation of DLL1 signaling was further measured by using DLL1-FLAG and DLL1-Fc. Three days after treatment of DLL1-FLAG and DLL1-Fc with FGF4 in hPDC cultures, the recombinant DLL1 protein up-regulated mRNA levels of DLL1 (P=0.0005 for DLL1-Flag and P=0.0002 for DLL1-Fc, n=3, respectively), Nestin (P=0.01 for DLL1-Fc, n=3, respectively), and TUJ1 (P=0.05 for DLL1-Flag and P=0.03 for DLL1-Fc, n=3, respectively) genes. These results indicate that the level of DLL1 through cell-cell interactions is an important factor in inducing nerve/neuronal differentiation of hPDC (FIG. 31 D).

To test whether FGF4 exposure increases other lineage cells, immunohistochemical assays were performed. Mesodermal markers, Brachyury and Desmin were not expressed, and an endoderm marker GATA4 was expressed regardless of FGF4 exposure (FIG. 32).

### (6) Comparison of neural induction potential of MSCs derived from bone marrow and adipose tissue and placenta-derived cells

In order to further investigate whether the novel neural induction method according to the present invention is applicable to other types of adult MSCs, adult human bone marrow MSCs (BM-MSCs) and adipose tissue-derived MSCs (AD-MSCs) were first incubated under normal conditions or HD conditions in the presence or absence of 25 ng/mL of FGF4 for 3 days. Although normal or HD cultures with FGF4 significantly induces Nestin⁺ cells (Ps < 0.0001 under all conditions, compared with the culture conditions without FGF4, n = 7 in each experiment), a proportion of Nestin⁺ cells was the highest in hPDCs (F(_{3,84})=602.5, P < 0.0001, n=7), as compared with other adult MSCs in FGF4 medium, which was comparable to fetal NPC used as a positive control. The number of Nestin⁺ showed the greatest difference between hPDCs cultured by the normal method and hPDCs cultured by the HD method, indicating that cells derived from hPDCs were highly sensitive to FGF4 neuronal induction (P <0.0001, n = 7). Neuronal induction of fetal NPCs was hardly influenced by FGF4 (FIGS. 31G, H). Semi-quantitative RT-PCR analysis of the tested cells demonstrated that DLL1 was hardly absent or not absent as mRNA in both adult MSCs; However, the level of DLL1 in hPDC increased only under the "HD" culture condition, and fetal NPC showed strong DLL1 expression (FIG. 32I).

### (7) Characterization of transplanted hPDC-NPCs in striatum graft site

Direct immunohistochemical staining with human-specific human nucleus (HN) markers was used to easily recognize the graft site in brain slices. HN-immunoreactive cells were observed around the vertical needle tract one week after transplantation (FIG. 33A). One week after transplantation, almost all HN⁺ cells simultaneously expressed SOX2 (FIG. 33B). At about 1 and 6 weeks post-transplantation, Nestin⁺ cells were co-labeled with HN⁺ cells (FIG. 33C). At 1 week post-transplantation, no other germ layer markers, for example, BRA (mesoderm), Desmin (mesoderm), and c-KIT (endoderm), were detected. At six weeks post-transplantation, cells positive for DCX, which is a migration marker, were distributed in the striatum. Some DCX⁺ cells were co-stained with HN (FIG. 33D). Some TH⁺ cells were also co-expressed with HN, indicating that the transplanted hPDC-NPCs differentiate into neurons, in particular, TH⁺ neurons in striatum areas. Furthermore, some cells were positive for AADC (FIGS. 33E, F).

### (8) Improvement effect of hPDC-NPC transplantation on functional impairment

Four rat groups of hPDC, hPDC-NPC (HD condition group), H9-NPC, and sham control, which showed experimentally induced Parkinsonian symptoms, were used for behavioral assays. Amphetamine-induced rotational behavior was measured to assess the extent of motor impairment or the degree of improvement in the 4 animal groups prior to transplantation and at 3, 6, and 9 weeks after transplantation (FIG. 34A). Significant interactions between groups and times were observed ((F_{9,117} = 1.30, P < 0.04). At 6 and 9 weeks post-transplantation, the hPDC-NPC group was significantly different from the sham control (P<0.03 and P<0.003, respectively) and hPDC and hPDC-NPC groups showed 20% and 30% decrease in ipsilateral rotational behavior, respectively, as compared with the sham control. In the hPDC-NPC-transplanted rats, the amphetamine-induced ipsilateral rotation continued to decrease from 3 weeks to 9 weeks.

In the H9-NPC group, the asymmetric behavior was about 20% lower at 6 weeks, but about 10% at 9 weeks. The unilateral lesion of MFB prefers use of non-injured foot. Cylinder tests, which is another experiment used to identify asymmetric behavior, were performed to evaluate use of injured forelimb in a transparent cylinder (FIG. 34B). A significant effect of the number of injured forelimb touch was observed (F_{3,32} = 2.52, P < 0.05).

A markedly increased use of injured forelimb was demonstrated in hPDC-NPC-transplanted rats, compared with sham control (P <0.05). Finally, exercise memory was analyzed by the rotarod test. The latency of falling from the accelerated load was measured. This experiment was conducted as short-term memory and long-term memory experiments. To measure short-term memory, three trials on the first day were analyzed, and the slope difference between trials was analyzed. The hPDC-NPC-transplanted rats showed a gradual increase in latency and spent a significant amount of time on the rod during three trials (p<0001, FIG. 34C).

In the long-term memory test, animals were subjected to the same experiment for 3 consecutive days following 21 days and 42 days after transplantation. The slope difference within the period represents the time for the animal to remember how the experiment was performed. The latencies of the hPDC-NPC and hESC-NPC groups were longer (Ps <0.001) over the three consecutive days, as compared with the sham control and hPDC group. At 42 days, hPDC-NPC-transplanted group cultured under HD conditions showed better rotarod performance, compared with the other groups, while the time spent on the rods was reduced significantly (Ps<0.0001, FIG. 34D).

In order to quantify DAT-ligand binding to left and right striatums at specific binding sites and DAT-ligand binding to the cerebellum at non-specific binding site in an equilibrium state, PET analysis was performed at 9 weeks after cell transplantation. Sham control was ¹⁸F FP-CIT PET of the dopaminergic impairment model. BP of the dopaminergic impairment model was 0.06 at a 6-OHDA injected site (right striatum) and 2.70 at a normal site (left striatum). ASI was 1.91 (FIG. 34E, left). After hPDC-NPC cell transplantation, increased uptake of radionuclide was detected at the striatum site.

BP was 1.75 at the placenta-derived cell-injected site (right striatum) and 2.75 at the normal site, and ASI was 0.44 (FIG. 34E, middle). In the normal control, BP in the striatum was 2.89 (left striatum) and 2.95 (right striatum) and ASI was 0.02 (FIG. 34E, right). After hPDC transplantation, the visual evaluation showed that the average number of striatum sites was significantly high. Molecular imaging by using ¹⁸F FP-CIT PET showed reduced radioactivity at striatal lesions in 6-OHDA-injected sites. However, after cell transplantation, BP significantly increased and ASI significantly decreased, indicating recovery of FP-CIT in the DAT terminal. Finally, after cell transplantation, TH protein levels were measured to assess in-vivo survival of dopaminergic neurons in striatum and SN. Six weeks after transplantation, TH protein was detected in uninjured striatum and uninjured SN, and not in lesioned striatum or lesioned SN of the hPDC-injected group or the sham control group. Surprisingly, TH protein was observed weekly in the lesioned striatum and SN of the hPDC-NPC-injected group (FIG. 34F).

To test whether hPDC-NPC forms a teratoma, hPDC-NPC was transplanted into SCID mice. No teratoma formation was observed at 6 weeks and 32 weeks (FIGS. 35A and B).

### Example 5. Suppressive effect of human placenta-derived cells on senescence

### 5.1. In-vitro study

An anti-senescence effect of hPDCs was demonstrated through co-culture of human-derived senescent cells and human placenta-derived cells (hPDCs).

### (1) Co-culture of human lung-derived senescent cell line and human placenta-derived cell

The effect of the cell of the present invention on senescence of LL-24 was examined by co-culturing human lung-derived cells which were senescent according to progression of generations (LL-24: 17-23 generations) with placenta-derived cells isolated from human placenta amniotic membrane. Specifically, LL-24 of the 17^{th}, 20^{th}, and 23^{th} generations was co-cultured in a transwell chamber, through which substances secreted from hPDCs (below 5^{th} generation) could pass (hPDCs were isolated), under culture conditions for LL-24 for 4 days. Then, cultured LL-24 was collected and analyzed for molecular biomarkers of cell proliferation and cell senescence.

As a result, it was found that 1) a senescence marker SA-b-galctosidase (SA-b-galctosidase assay) was decreased and 2) the number of cells was increased in fibroblasts (p17, p20, and p23) co-cultured with hPDCs, compared with fibroblasts not co-cultured with hPDCs (FIG. 37).

Further, increased expression of 1) PCNA and Cyclin D1 which are cell proliferation markers and 2) Sirt1 which is an anti-senescence marker, 4) a significantly increased expression level of SOD2 which is an anti-oxidation marker inhibiting generation of reactive oxygen species which is a main cause of senescence, and 5) increased expression of Tet1, which is a regulator of epigenetic expression of several target genes were observed in the co-cultured LL-24, as compared with the control, and 6) an increase of Sirt1 and PCNA and a decrease of a senescence marker p16 protein were observed by Western blotting, when co-cultured (FIG. 38). However, these differences were reduced as the generation progressed, indicating that senescence inhibition by hPDCs varies according to age and generation of target cells.

### (2) Co-culture with human-derived neural progenitor cells (human neural precursor cells: hNPCs)

hNPCs cultured until 18^{th} generation were co-cultured for 4 days and 6 days in a chamber, through which materials secreted from hPDCs could pass, under culture conditions for hNPCs. Then, expression of senescence-related markers, cell proliferation markers, regulators of reactive oxygen facilitating senescence, epigenetic markers involved in maintenance of hNPCs characteristics, and Wnt signaling materials which is a key mechanism was examined.

As a result, in the case of hNPCs, co-culture with hPDCs for 4 days and 6 days did not cause changes in cell proliferation and cell proliferation markers due to a long cell subculture period (∼ 14 days) and a slow growth rate. In addition, there was no difference in the expression of senescence markers such as p16, but the expression of enzymes, catalase, SOD2, and GPx1, which inhibit generation of reactive oxygen which is a strong senescence inducer, was increased during co-culture for 6 days, in particular, SOD2 was greatly increased even upon co-culture for 4 days, compared with other enzymes, DNMT3a, DNMT3b, and TET1, which are epigenetic markers involved in the maintenance and regulation of hNPCs, showed a slight increase upon co-culture, and expression of SFRP1, which is an inhibitory peptide of WNT signal involved in proliferation and differentiation of hNPCs, was increased upon co-culture (FIG. 39).

### 5.2. In vivo study

Natural senescence process and mechanism were studied by using natural senescence-induced mice (> 18 months old), and after at least one administration of human placenta-derived cells, senescence regulation and anti-senescence mechanism of placenta-derived cells of the present invention were investigated through various behavioral tests and molecular biological and biochemical analysis of tissues.

### (1) Analysis of anti-senescence activity of placenta-derived cells by using naturally senescent mice

18-20 month-old C57BL/6 mice were injected with 10,000 human placenta-derived cells (hPDCs) or the same volume of physiological saline as the control group at 6-week intervals via the tail vein, and a survival rate was examined and a behavioral test capable of detecting changes in cognitive function was performed.

As a result, compared with the mice of the same age (18-20 months) administered with physiological saline, the mice administered with human placenta-derived cells at 6 week-intervals showed 1) an increased survival rate from the time of administration to 24-25 months (FIG. 40), and 2)improved cognitive function after three administrations due to improvement in the time spent to find a designated spot, the frequency of finding the designated spot, and the time spent to stay in the area where the designated spot is located, in a Morris Water Maze experiment which confirms cognitive function by the ability to find a designated spot (probe) after training (data not shown). In addition, after three administrations, the mice administered with hPDCs showed 4) improvement in the time when mice move in a limited space, compared with the mice administered with physiological saline, indicating improvement of the motor function by hPDCs.

Further, the hippocampus, which is a brain region that plays an important role in the cognitive function of the brain, was analyzed in senescent mice administered with hPDCs three times, and the senescence progression of the hippocampus by the cells of the present invention was examined. As a result, as in the result of cognitive function improvement in the mice administered with placenta-derived cells, expression of senescence-related markers such as p16, HMGA2, and p57 was suppressed in 23-month-old senescent mice (multi-cell group) administered with the cells three times, compared with a control group of the same age. However, there was no difference in the 18-month-old mice administered with the cells once (single cell group), compared with the control group. The expression of p16 and p57 was increased in all senescent mice, compared with 3-month-old young mice (young group). Astrocytes that control immune function and neuronal activity in the brain increased with senescence progression, and decreased in 18-month-old senescence mice administered with hPDCs once, compared with the control of the same age, but the effect was decreased in 23-month-old senescent mice. The energy metabolism and expression of oxidative stress markers UCP2 and SOD2 in tissues tend to decrease but insignificant (FIG. 40).

The effect of exogenously injected placenta-derived cells on the differentiation of neurons by their own neural stem cells in the dentate gyrus of the hippocampus was investigated. Immunohistochemistry confirmed that the placenta-derived cells cause no significant change in the distribution of neurons at an immature stage (Tuj1) and a mature stage (NeuN) in 23-month-old senescent mice administered with hPDCs three times, compared with a control of the same age.

Next, the effect of hPDCs on differentiation of oligodentrocytes which control senescence and neurotransmission of neurons was investigated. Oligodendrocytes and myelin which is a protein produced by oligodendrocytes surround the axons of neurons to regulate neurotransmitter function, and destruction of oligodendrocytes and myelin have been reported to be closely related to senescence-related neurological diseases. An immune antibody staining method revealed that the distribution of oligodendrocyte precursor cells (NG2) was increased in 23-month-old senescent mice administered with hPDCs three times, compared with a control group of the same age. The difference was greater in mature oligodendrocytes (MBP). In particular, MBP-stained cells showed structural and expression differences in senescent mice administered with placenta-derived cells, suggesting that differences in the distribution and function of oligodendrocytes may cause improvement of cognitive function and increase of nerve activity by placenta-derived cells.

### (2) Analysis of transcript of hippocampus of senescent mice administered with placenta-derived cells (RNA sequence analysis)

The transcripts of hippocampus of 23-month-old senescent mice administered with hPDCs were isolated, and RNA sequence analysis (Rseq analysis) was performed to analyze a difference in gene expression by placenta-derived cells. The hippocampus of 23-month-old senescent mice showed a significant difference of 1.5 times or more in the expression of many genes involved in various immune functions, compared with young mice, which is consistent with changes in immune function according to senescence (Table 4). 23-month-old senescent mice administered with placenta-derived cells three times showed a significant difference of 1.5 times or more (p-value<0.05) in many genes involved in the neuronal proliferation and differentiation, glucose metabolism, biosynthesis, and regulation of immune function, compared with the control of the same age (Table 5). The genes listed in Table 4 and Table 5 are the top ranked genes showing significant differences, confirming that the expression of more genes was affected by the administration of placenta-derived cells.

**[Table 4]**

| Comparison of transcripts of hippocampus between 3-month-old young mice and 23-month-old senescent mice | | | |
|---|---|---|---|
| **Term** | **Coun t** | **PValue** | **Benjamini** |
| GO:0006955∼immune response | 25 | 8.97E-10 | 1.14399E-06 |
| GO:0006952∼defense response | 16 | 0.000215 | 0.128061949 |
| mmu04060:Cytokine-cytokine receptor interaction | 11 | 0.001047 | 0.093787922 |
| GO:0050830∼defense response to Gram-positive bacterium | 4 | 0.002262 | 0.618256899 |
| GO:0050766∼positive regulation of phagocytosis | 4 | 0.002262 | 0.618256899 |
| GO:0050764∼regulation of phagocytosis | 4 | 0.002888 | 0.602504662 |
| GO:0009991∼response to extracellular stimulus | 7 | 0.004447 | 0.679390626 |
| GO:0006954∼inflammatory response | 9 | 0.004627 | 0.627056618 |
| GO:0030888∼regulation of B cell proliferation | 4 | 0.005871 | 0.658148649 |
| 00:0016064∼immunoglobulin mediated immune response | 5 | 0.006023 | 0.61844242 |
| GO:0019724∼B cell mediated immunity | 5 | 0.006725 | 0.615829076 |
| GO:0009595∼detection of biotic stimulus | 3 | 0.006814 | 0.582046828 |
| GO:0045807∼positive regulation of endocytosis | 4 | 0.006965 | 0.55549258 |
| GO:0009611∼response to wounding | 11 | 0.007065 | 0.529456684 |
| GO:0002684∼positive regulation of immune system process | 8 | 0.010021 | 0.627882589 |
| GO:0002449∼lymphocyte mediated immunity | 5 | 0.011562 | 0.65353014 |
| GO:0002252∼immune effector process | 6 | 0.015431 | 0.73363622 |
| GO:0009620∼response to fungus | 3 | 0.016101 | 0.725976354 |
| GO:0002250∼adaptive immune response | 5 | 0.016205 | 0.70662904 |

- The genes in the above list correspond to genes showing a significant expression difference of 1.5 times or more in senescent mice, compared with young mice.

**[Table 5]**

| Comparison of transcripts of hippocampus between 23-month-old senescent mice administered with hPDCs three times and control group of the same age | | | |
|---|---|---|---|
| **Term** | **Cou nt** | **PValue** | **Benjamini** |
| GO:0045927∼positive regulation of growth | 4 | 0.003494621 | 0.944318731 |
| GO:0008284∼positive regulation of cell proliferation | 7 | 0.004460707 | 0.84184154 |
| GO:0035270∼endocrine system development | 4 | 0.007066515 | 0.857752987 |
| GO:0009309∼amine biosynthetic process | 4 | 0.007340905 | 0.781210752 |
| GO:0021532∼neural tube patterning | 3 | 0.007473213 | 0.709952571 |
| GO:0045165∼cell fate commitment | 5 | 0.008424757 | 0.687550171 |
| GO:0015758∼glucose transport | 3 | 0.010092495 | 0.697452119 |
| GO:0021915∼neural tube development | 4 | 0.010433485 | 0.660949255 |
| GO:0042401∼biogenic amine biosynthetic process | 3 | 0.010802181 | 0.630493477 |
| GO:0021983∼pituitary gland development | 3 | 0.010802181 | 0.630493477 |
| GO:0008645∼hexose transport | 3 | 0.011533309 | 0.615967271 |
| GO:0015749∼monosaccharide transport | 3 | 0.012285632 | 0.604312019 |
| mmu04080:Neuroactive ligand-receptor interaction | 6 | 0.015403555 | 0.494916026 |
| GO:0021536∼diencephalon development | 3 | 0.018124928 | 0.715641565 |
| GO:0050863∼regulation of T cell activation | 4 | 0.021056125 | 0.74089527 |
| GO:0042127∼regulation of cell proliferation | 8 | 0.026862326 | 0.799031687 |
| G0:0030917∼midbrain-hindbrain boundary development | 2 | 0.026938855 | 0.777306661 |
| GO:0009792∼embryonic development ending in birth or egg hatching | 7 | 0.028070034 | 0.769628766 |
| GO:0042398∼cellular amino acid derivative biosynthetic process | 3 | 0.028087059 | 0.749063813 |

- The genes in the above list correspond to genes showing a significant expression difference of 1.5 times or more in the cell-administered senescent mice, compared with the control group.

Mechanisms involved in genes showing different expression patterns were analyzed by analysis of the transcripts isolated from the hippocampus. Immune-related mechanisms and disease-related mechanisms such as Alzheimer's disease and carcinoma were activated, but neural synapse and sensory development and cell signal transduction-related mechanisms were suppressed in the hippocampus of 23-month-old senescent mice, compared with 3-month-old young mice. Mechanisms involved in signal transduction by calcium and serotonin and signal transduction between neurons were activated, but mechanisms involved in ligand-receptor interactions were suppressed in senescent mice administered with placenta-derived cells three times, compared with senescent mice of the same age. These results suggest that placenta-derived cells may enhance cognitive function and locomotion through the enhancement of the neuronal signaling system of the hippocampus, suggesting that placenta-derived cells may inhibit the development of neurological diseases.

### (3) Metabolomic analysis of plasma of senescent mice administered with placenta-derived cells

In this study, based on the finding that cognitive function and neuronal signal transduction are activated by administration of placenta-derived cells, interactions between changes in the metabolites and changes in senescence and antisense mechanism according to hPDC injection was investigated. Compared with a control group of the same age, 23-month senescent mice administered with hPDCs three times showed significant differences in metabolites which are reported to be associated with neuronal function and degenerative brain diseases, such as linoleic acid and alpha-linolenic acid metabolism, pantothenate and CoA synthesis, sphingolipid and glycerophospholipid metabolism, arachidonic acid metabolism, etc. In particular, lipid metabolites such as sphingolipids and metabolisms are associated with synthesis of myelin, which is secreted by oligodendrocytes and surrounds the axons of nerve cells to regulate neuronal signaling, and therefore, correlation between differences of the metabolites and differences in cellular structures stained by MBP and MBP expression in the control group and the 23-month senescent mice administered with placenta-derived cells may be partially explained. In addition, the senescent mice administered with placenta-derived cells three times showed a significant difference in metabolites related to beta-alanine and glutathione metabolisms responsible for the removal of reactive oxygen species which is a strong senescence inducer, compared with the control group of the same age. There were also significant differences in metabolites associated with starch and sucrose metabolism, arginine and proline metabolism, energy and basal metabolism such as purine metabolism, protein and genome synthesis, and active nitrogen production regulation.

In order to investigate metabolite changes in the senescent mice administered with hPDCs three times, and the short-term effects by administration of senescent mice with placenta-derived cells at the same time, plasma were separated from 18-month-old senescent mice administered with placenta-derived cells once, and compared with that of a control group of the same age. Similar to 23-month-old senescent mice, there were significant differences in metabolites associated with neuronal functions, such as linoleic acid and alpha-linolenic acid metabolism, pantothenate and CoA synthesis, arachidonic acid metabolism, etc., and anti-oxidant functions, such as beta-alanine and glutathione metabolisms. Unlike 23-month-old senescent mice, there were significant differences in amino acid metabolism-related metabolites and fatty acid metabolism-related metabolites such as butanoate and steroid hormones between the mice administered with hPDCs once and a control group of the same age. In addition, there were also significant differences in propanoate which influences length of telomeres to help maintain genomic stability, seleno-amino acids which are toxic to a living body, and vitamin B6 metabolites which prevent protein denaturation called advanced glycation end products (AGE) causing a reduction in cognitive function and senescence, suggesting a potential short-term effect of the administration of placenta-derived cells on suppression of senescence and prevention of neurological diseases.

In conclusion, the above experiments confirmed that administration of senescent mice with hPDCs once or more leads to enhancement of cognitive function and locomotion of the senescent mice, and influences expression of various genes related to senescence and neurological diseases, metabolisms, and metabolite distribution of the metabolisms, thereby inhibiting or preventing senescence neurological diseases.

## Claims

1. A placenta-derived cell secreting a C3 or C1 complement protein.

2. The placenta-derived cell of claim 1, further secreting one or more proteins selected from the group consisting of EPPK1, PTX3, EEF1A1, LGALS3BP, FLJ53478, PXDN, and TGFβ.

3. The placenta-derived cell of claim 2, wherein the cell is CD9⁺, CD13⁺, CD90⁺ and CD200⁺.

4. The placenta-derived cell of claim 1, wherein the C3 or C1r complement protein is expressed at 0.1 pg/ml/1x10⁶ cells to 1000 pg/ml/1x10⁶ cells.

5. The placenta-derived cell of claim 1, wherein the placenta comprises an amniotic membrane, a chorionic membrane, a decidual membrane, an umbilical cord, or a combination thereof.

6. A pharmaceutical composition for treating or preventing neurological diseases, comprising the placenta-derived cell of any one of claims 1 to 4.

7. The pharmaceutical composition of claim 6, wherein the neurological disease is Alzheimer's disease, Parkinson's disease, HIV dementia, epilepsy, schizophrenia, depression, manic depression, neurogenic disorders, autism, stroke, Lou Gehrig's disease, Huntington's disease, multiple sclerosis, brain damage, cerebral palsy, or spinal cord injury.

8. The pharmaceutical composition of claim 7, wherein the neurological disease is Alzheimer's disease.

9. A pharmaceutical composition for treating or preventing angiogenesis-related diseases, comprising the placenta-derived cell of any one of claims 1 to 4.

10. The pharmaceutical composition of claim 9, wherein the angiogenesis-related disease is cancer, diabetic retinopathy, retinopathy of prematurity, corneal graft rejection, neovascular glaucoma, erythrosis, proliferative retinopathy, psoriasis, hemophilic joints, capillary proliferation in atherosclerotic plaques, keloids, wound granulation, vascular adhesion, rheumatoid arthritis, osteoarthritis, autoimmune disease, Crohn's disease, recurrent stenosis, atherosclerosis, intestinal adhesion, cat scratch disease, ulcer, hepatopathy, glomerulonephritis, diabetic nephropathy, malignant neuropathy, thrombotic microangiopathy, organ graft rejection, nephropathy, or diabetes.

11. The pharmaceutical composition of claim 10, wherein the angiogenesis-related disease is retinopathy of prematurity, diabetic retinopathy, or proliferative retinopathy.

12. A pharmaceutical composition for reducing senescence, comprising the placenta-derived cell of any one of claims 1 to 4.

13. A pharmaceutical composition for treating senescence-related symptoms, comprising the placenta-derived cell of any one of claims 1 to 4.
